(19) Europäisches Patentamt
European Patent Office
Office européen des brevets

(11) Publication number: **0 421 919 A2**

(12) **EUROPEAN PATENT APPLICATION**

(21) Application number: 90810643.8

(22) Date of filing: 24.08.90

(51) Int. Cl.5: C12N 15/55, C12N 1/15, C12N 9/26

The applicant has filed a statement in accordance with Rule 28 (4) EPC (issue of a sample only to an expert). Accession number(s) of the deposit(s): DSM 5505, 5866, 5941-5964.

The title of the invention has been amended (Guidelines for Examination in the EPO, A-III, 7.3).

(30) Priority: 02.09.89 GB 8919884
19.06.90 GB 9013616

(43) Date of publication of application:
10.04.91 Bulletin 91/15

(84) Designated Contracting States:
AT BE CH DE DK ES FR GB GR IT LI LU NL SE

(71) Applicant: **CIBA-GEIGY AG**
**Klybeckstrasse 141**
**CH-4002 Basel(CH)**

(72) Inventor: **Visser, Jacob, Prof.**
**Hinkeloordseweg 4**
**NL-6703 CK Wageningen(NL)**
Inventor: **Bussink, Hendrik Jan Dirk**
**Thorbeckestraat 102**
**NL-6702 BT Wageningen(NL)**
Inventor: **Kester, Hermanus Cornelis Maria**
**Hoogstuk 74**
**NL-6651 DR Druten(NL)**
Inventor: **de Graaff, Leendert Hendrik**
**Dullerstraat 10**
**NL-6828 HK Arnhem(NL)**
Inventor: **Buxton, Frank, Dr.**
**Holderstüdeliweg 32**
**CH-4132 Muttenz(CH)**

(54) **Polygalacturonase encoding fungal expression system.**

(57) The invention concerns a recombinant DNA molecule coding for a polygalacturonase (PG) expression cassette comprising the structural gene of a PG and/or corresponding regulatory sequences, or hybrid vectors comprising a DNA derived from a PG gene, further transformed hosts, especially filamentous fungi, methods for the preparation of said recombinant DNA molecules and transformed hosts and the preparation of polypeptides by means of said DNAs and said hosts, purified single PGs either from natural or transformed hosts, the preparation of mixtures of PGs optionally with other enzymes, and the use of PGs or mixtures thereof in industry.

EP 0 421 919 A2

FIGURE 3

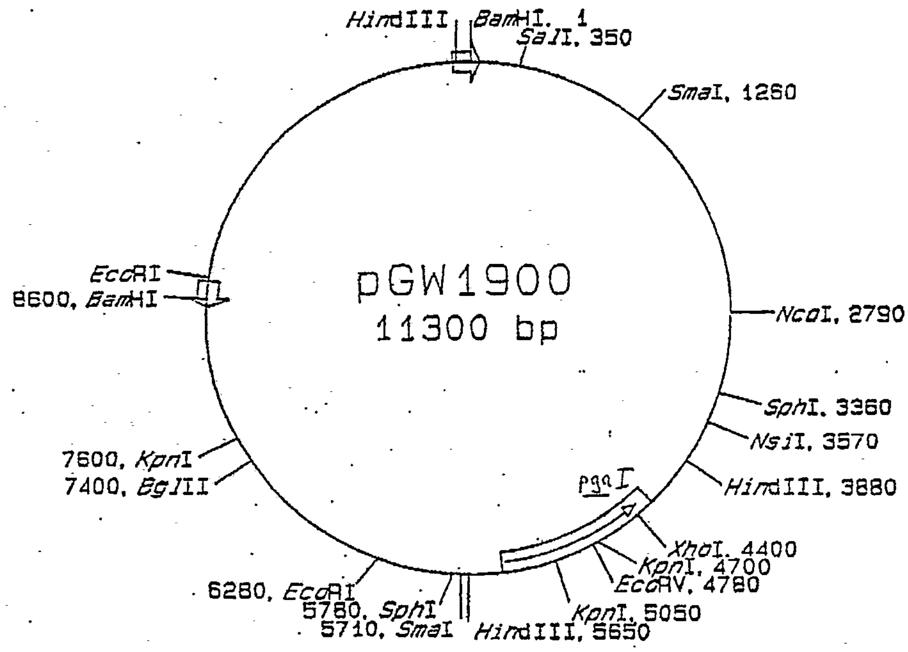
HindIII
BamHI, 1
SalI, 350
SmaI, 1260
NcoI, 2790
SphI, 3360
NsiI, 3570
HindIII, 3880
pgaI
XhoI, 4400
KpnI, 4700
EcoRV, 4780
KpnI, 5050
HindIII, 5650
5710, SmaI
5760, SphI
6280, EcoRI
7400, BglII
7600, KpnI
8600, BamHI
EcoRI
pGW1900
11300 bp

# FUNGAL EXPRESSION SYSTEM

## Field of the invention

The invention relates to the field of genetic engineering and provides novel DNA molecules comprising DNA sequences coding for proteins with polygalacturonase activity and/or the promoter, signal and/or transcriptional terminator sequences naturally linked thereto. The novel DNA molecules are useful for the construction of expression cassettes for the production of polygalacturonases or the expression of foreign genes in filamentous fungi.

## Background of the invention

Although in genetic engineering techniques numerous polypeptide expression systems for prokaryotic and eukaryotic hosts are already known, there is a continuing need for novel systems which have advantages over previous systems.

Very widely used hosts are the prokaryot Escherichia coli and the eukaryotic yeasts, e.g. Saccharomyces cerevisiae , for which a large number of different expression hybrid vectors, mostly plasmids, have been developed. The drawbacks of E. coli hosts are that they cannot glycosylate polypeptides and that intracellular expression of foreign peptides may lead to accumulation within the host cell and prevention of further growth. The yeasts do glycosylate, however, like E. coli , they do not secrete the polypeptides, except small ones, into the nutrient medium, but into the periplasmic space. Higher eukaryotic hosts such as mammalian cancer cells are able to glycosylate and secrete into the nutrient medium, however, cultivation thereof is slow and expensive and the danger exists that oncogenic nucleic acids may be isolated together with the desired peptide.

In the search for other hosts filamentous fungi, such as Neurospora crassa , Aspergillus nidulans and Aspergillus niger , have also been investigated. The application thereof in genetic engineering techniques has been slower, mainly for lack of an appropriate transformation system. In contrast to Saccharomyces cerevisiae , filamentous fungi do not contain plasmids which could be used for the introduction of foreign genes and phenotypic selection. It is, however, possible to transform filamentous fungi with foreign plasmids containing a selectable marker gene. Most vectors described so far for filamentous fungi do not autonomously replicate, as those of yeasts do, but are integrated into the fungal chromosome. This event occurs generally at a lower frequency. However, integrative transformation renders the transformants mitotically very stable, even under non-selective conditions. Stable integration of more than one hundred copies has been reported.

The first vector for filamentous fungi described contained the qa-2 gene of Neurospora crassa as selectable marker [Case, M.E., Schweizer, M., Kushner, S.R. and Giles, N.H. (1979) Proc. Natl. Acad. Sci. USA 76,5259-5263; Case, M.E. (1982) in Genetic Engineering of Microorganisms for Chemicals (Hollander, A., DeMoss, D., Kaplan, S., Konisky, J., Savage, D. and Wolfe, R.S., eds), pp. 87-100, Plenum].

In Aspergillus nidulans , which has a sexual cycle and is therefore amenable to classical genetic manipulations, both negative and positive selection systems have been described (Ballance et al. BBRC 112 ,284,1983; Tilburn et al. Gene 26 , 205,1983; Yelton et al. PNAS 81 , 1470, 1984; Yelton and Timberlake J. Cell. Biochem. Suppl. 9C , 173, 1985; Johnstone et al. EMBO J. 4 , 1307, 1983; Tilburn et al. Gene 26 , 205, 1983; Wernars et al., Curr. Genet. 9 , 361, 1985; Kelly, J.M. et al., EMBO J.4 , 475, 1985).

Compared to N. crassa or A. nidulans , A. niger is by far the more important organism, as it is used widely in the industrial production of enzymes, e.g. for use in the food industry. A. niger is known to secrete a variety of hydrolytic enzymes, e.g. glucoamylase, $\alpha$-amylase, pectinase, cellulase, $\beta$-glucanase, $\beta$-galactosidase, naringinase, pentosanase, acid protease and lignase, the glucoamylase and pectinase complex being the most important ones.

A. niger has no known sexual cycle. Mutations can therefore not be introduced via meiotic recombination. By classical mutation and selection procedures, extensive strain improvements in the secretion of hydrolytic enzymes have however been achieved.

Of the genes of A. niger enzymes only those of glucoamylase (Boel et al. EMBO J. 3 , 1581, 1984) and alcohol and aldehyde dehydrogenase (WO 86/06097) together with their promoter and signal sequences have been characterised and used in transformation experiments with A. nidulans and A. niger .

As selection markers for A. niger the heterologous amds gene (Kelly and Hynes, EMBO J. 4 , 475, 1985), and the argB gene (Buxton et al., Gene 37 ,207,1985; EP 184 438; WO 86/06097), both obtained from A. nidulans , have been used.

A. niger is the most important organism for the industrial production of pectin degrading enzymes, e.g. polygalacturonases, pectin lyases or pectin esterases. Pectins are polygalacturonides of high molecular weight (20000-40000 D) consisting of α-1,4-glycosidic bounded D-galacturonic acid polymers. Some of the uronic acid groups are esterified with methanol which can be split off by pectin esterase. Pectins occur in nature as constituents of higher plant cells, where they are attached to cellulose molecules mainly found in the primary cell wall and the middle lamella. Amongst the richest sources of pectin are lemon and orange rind, which contain about 30 % of this polysaccharide. Pectic enzymes degrade the carbohydrate polymer substrate either by hydrolysis of the α-1,4-glycosidic bond (endo- and exo-polygalacturonases) or by transelemination (pectin lyases) leading to a satured and to an α-4,5 unsaturated poly- or oligogalacturonide. The systematic name of endo-polygalacturonase is (poly-(1,4-α-D-galacturonide))glycanhydrolase (EC 3.2.1.15) and of exo-polygalacturonase is (poly-(1,4-α-D-galacturonide))-galacturonohydrolase (EC 3.2.1.67). Another relevant pectin degrading galacturonase enzyme is rhamnogalacturonase which hydrolyses bonds between α-D-galacturonate and L-rhamnose.

While pectin lyases are specific for highly esterified pectins, polygalacturonases hydrolyse low esterified pectins. The combined action of pectin esterases and polygalacturonases can also depolymerize highly esterified pectins.

The applications of polygalacturonase and enzyme mixtures thereof in fruit and vegetable processing (Table 1) have developed from the original uses of pectic enzymes for treatment of soft fruit to ensure high yields of juice and pigments upon pressing and for the clarification of raw press juices. Technical enzyme preparations in use for these processes contain pectin esterases, polygalacturonases and pectin lyases in varying amounts along with other enzymes such as arabinanases, galactanases, xylanases, β-1,4-glucanases, glycosidases and proteases.

Fungal pectinase preparations containing predominantly endopolygalacturonase and being free of pectin esterase are used successfully as macerating enzymes for the production of pulpy nectars which have a more smooth consistency and have higher contents in soluble solids, pigments and nutrients than products prepared by a mechanical-thermal process.

Table 1 (from ref.32)

| Use of polygalacturonases and mixtures thereof in fruit and vegetable industry. | |
|---|---|
| Enzymes | Use |
| Polygalacturonase | Maceration, Citrus juice stabilization/viscosity reduction |
| Pectinesterase + Polygalacturonase and/or Pectin lyase | Juice clarification, juice/oil extraction, Citrus peel oil, citrus pulp wash |
| Pectinesterase/Polygalacturonase/Pectin lyase + (Hemi-) | Liquefaction, clear/cloudy juices |
| Cellulases | Enhance natural product extraction<br>Valorization biomass/feed |

The presence of pectin esterase can easily transform the macerating activity of a pure polygalacturonase into cell disintegration activity because of the general depolymerizing activity of the pectin esterase/polygalacturonase combination.

Through the use of pectic and cellulolytic enzymes the cell walls of fruit pulps can be degraded to the stage of almost complete liquefaction. The presence of both endo- and exo- β-1,4 glucanases (cellulases) as well as pectic enzymes is essential (ref. 31).

Polygalacturonase and enzyme mixtures thereof are also useful for liquefaction and saccharifaction of biomass, for example, for the production of fermentable polysaccharides from plant cells (ref. 33) or for the modification of pectins (for review see ref. 32). Polygalacturonases in combination with xylanases are also useful in the paper pulping industry for instance (ref. 44).

In A. niger the proteins of the pectic complex are not expressed constitutively. Under inducing conditions using pectin or breakdown products thereof A. niger expresses the above mentioned enzymes, when other carbon sources, such as glucose or sucrose, are growth limiting. In surface cultures the pectic enzymes tend to remain associated with the outer cell wall.

Both Rexová-Benková and Markovic (ref. 29) and Rombouts and Pilnik (ref. 15) disclose a number of polygalacturonases obtained from Aspergillus niger and other fungi, as well as from bacteria and plants, which were described as purified. However, as no structural data were given there is still a need for single polygalacturonases with high specific activity. Advantageously they should be pure enough to allow the determination of their amino acid sequences.

Hereinafter, polygalacturonases are also named PG.

## Object of the invention

Objects of the present invention are purified single PGs either from natural or transformed hosts.

The present invention is based on the purification and partial structure determination of single PGs, e.g. PGI, II, IIIA, IIIB, IV, in particular PGI or PGII, which allows the synthesis of DNA probes coding for relevant parts of the protein.

An object of the present invention is to screen for and isolate by means of the DNA probes, DNA sequences coding for PGII or PGI, eventually together with pre- and post-sequences thereof, from a gene library of A. niger . A further object is to identify further PG genes by hybridization of parts of the PGII gene (named pgaII ) or the PGI gene (named pgaI ) with a gene library of a fungal strain, e.g. of A. niger .

Further objects are recombinant DNA molecules coding for polygalacturonase gene expression cassettes which comprise a structural gene of a polygalacturonase, optionally with intron sequences, and/or regulatory sequences of a PG gene, e.g. promoter, signal and/or transcriptional terminator sequences. Further recombinant DNA molecules of the invention are, for example, hybrid vectors comprising DNA derived from a PG gene of the invention.

Further objects are hosts, especially filamentous fungi, e.g. Aspergillus hosts, transformed with said vectors, methods for the preparation of the recombinant DNA molecules and the transformed hosts and the use of the recombinant DNA molecules for the preparation of the novel expression cassettes or hybrid vectors.

An object of the present invention is also the overproduction of PGs, for example in Aspergillus species, and the production of the single PGs which are uncontaminated with other PGs, or of predetermined artificial mixtures thereof.

Another object concerns the production of any heterologous protein the structural gene of which can be expressed under the control of the present recombinant PG DNAs.

The various subjects of the invention will become more evident from the following detailed description of the invention.

## Detailed description of the invention

### The Recombinant DNA Molecules

The present invention concerns a recombinant DNA molecule comprising a DNA sequence selected from

a) the Aspergillus niger DNA insert of pGW1800 or pGW1900,

b) a DNA sequence which hybridizes to the coding region for the mature PGII or PGI comprised by a DNA insert of a) and which comprises a structural gene for a polypeptide with polygalacturonase activity, and optionally a promoter, a coding region for a signal or leader peptide and/or a transcriptional terminator thereof,

c) a derivative of a DNA sequence defined in a) or b), or

d) a DNA sequence which codes for a mature PG or a signal peptide or a leader peptide thereof and which is degenerate within the meaning of the genetic code with respect to a DNA sequence of a), b) or c).

More particular, the invention concerns a recombinant DNA molecule comprising a DNA sequence selected from

a) the Aspergillus niger DNA insert of pGW1800,

b) a DNA sequence which hybridizes to the coding region for the mature PGII comprised by the DNA insert of a) and which comprises a structural gene for a polypeptide with polygalacturonase activity, and optionally a promoter, a coding region for a signal or leader peptide and/or a transcriptional terminator thereof,

c) a derivative of a DNA sequence defined in a) or b), or

d) a DNA sequence which codes for a mature PG or a signal peptide or a leader peptide thereof and which is degenerate within the meaning of the genetic code with respect to a DNA sequence of a), b) or c), or a recombinant DNA molecule comprising a DNA sequence selected from

a) the Aspergillus niger DNA insert of pGW1900,

b) a DNA sequence which hybridizes to the coding region for the mature PGI comprised by the DNA insert of a) and which comprises a structural gene for a polypeptide with polygalacturonase activity, and optionally a promoter, a coding region for a signal or leader peptide and/or a transcriptional terminator thereof

c) a derivative of a DNA sequence defined in a) or b), or

d) a DNA sequence which codes for a mature PG or a signal peptide or a leader peptide thereof and which is degenerate within the meaning of the genetic code with respect to a DNA sequence of a), b) or c).

The plasmids pGW1800 and pGW1900 are comprised in the E. coli strains JM109/pGW1800 and DH5αF′/pGW1900, respectively, deposited with the Deutsche Sammlung für Mikroorganismen und Zellkulturen (DSM).

Polypeptides with polygalacturonase activity are herein also referred to as polygalacturonases (PGs). This term also includes fragments or mutants of the naturally occurring polygalacturonases which retain enzymatic activity. The terms polygalacturonase or PG used herein also include other galacturonases having as substrate an oligo-or polysaccharide comprising galacturonate, e.g. oligogalacturonase cleaving an oligogalacturonate or rhamnogalacturonase cleaving a copolymer of rhamnose and galac turonate, or fragments or mutants thereof retaining enzymatic activity. A "leader peptide" is intended to be the sequence of a prepro-PG which is cut off during the formation of a mature PG. A "signal peptide" is cut off in a first step by the "signal peptidase" in the endoplasmatic reticulum.

A recombinant DNA molecule of the present invention comprises, for example, the Aspergillus niger DNA insert of pGW1800 or pGW1900.

The 4.1 kb long A. niger N400 insert of pGW1800 (see Fig. 2) extends from the XbaI restriction site (map position 1) to the EcoRI restriction site (map position 4 100). This insert comprises the promoter and the transcriptional terminator region of the PGII gene pgaII as well as the coding region for prepro-PGII, including introns. The DNA sequence of the A. niger insert fragment comprising pga II starting at the XbaI site in the approximate map position 1 and ending at the PvuII site in the approximate map position 3050 shown in Figure 2 was determined and is depicted in the sequence listing under the Sequence Identification Number (SEQ ID NO.) 2.

The promoter region of pga II extends upto position 1357 of this DNA sequence with the Sequence Identification Number (SEQ ID NO.) 2. The coding region for the leader peptide of prepro-PGII extends from position 1357 up to position 1437. The signal peptide is encoded by the nucleotides 1357 up to 1419. The coding region for the mature PGII extends from position 1438 to position 2494. It is followed by the transcriptional terminator region which starts at position 2499.

The 8.6 kbp long A. niger N400 insert of pGW1900 (see Fig. 3) extends from the Bam HI site in map position 1 up to the BamHI site in the approximate map position 8600 indicated in Figure 3. The DNA sequence extending from about map position 6280 up to about 3880 is given in the sequence listing under SEQ ID NO. 1.

The promoter region of the 8.6 kbp insert extends upto position 910 of the DNA sequence with SEQ ID No. 1. The coding region for the 31 amino acid long leader peptide of prepro-PGI extends from position 910 up to position 1002. The signal peptide is encoded by nucleotides 910 to 963. The coding region for mature PGI starts at position 1003 and extends up to 2127. The transcriptional terminator region starts at position 2131.

DNA sequences coding for PGI or PGII derived from the A. niger N400 inserts of pGW1900 or pGW1800 hybridize under "homologous" conditions with the coding region of the PGI or PGII gene, respectively, or of a fragment thereof derived from A. niger N400. DNA sequences which are at least partially homologous to the A. niger N400 PGI or PGII coding region and encode a protein with PG activity are members of the PG gene family. DNA sequences which are only partially homologous hybridize with the A. niger N400 PGI or PGII derived sequences under so called "heterologous" conditions which are less stringent than the "homologous" conditions. A member of the PG gene family which only hybridizes under "heterologous" conditions can be another PG gene of A. niger N400 than the PGI or PGII gene or a PGI, PGII or a different PG gene of a PG producing fungal strain other than A. niger N400. Such fungal strain can be derived, for example, from Aspergillus spec ., e.g. A. japonicus , A. oryzae , A. nidulans , or an A. niger strain other than N400, Trichoderma spec. , Botrytis spec ., Sclerotinia spec ., Fusarium spec . or other phytopathogenic fungi as well as from yeast, for example from PG producing Kluyveromyces spec . such as K. fragilis . A preferred example for such a strain is A. niger NW756. Thus, the PG gene family of the invention comprises the genes encoding PGI, PGII, PGIIIA, PGIIIB, PGIV and other PGs, preferentially derived from Aspergillus spec., more preferentially from A. niger , most preferentially from strain N400 or NW 756, but also from other PG producing fungal strains. Members of the PG gene family are named "PG genes".

The protein products of the members of the PG gene family include enzymes ("galacturonases") which can cleave oligo- or polysaccharides comprising galacturonate, e.g. oligogalacturonase, rhamnogalacturonase or in particular polygalacturonase.

A conventional hybridization procedure is described e.g. by Benton and Davis (ref. 7). Homologous and heterologous hybridization conditions are more precisely defined hereinafter.

The term "derivative" when used in connection with the novel DNA sequences of the invention is intended to include larger derivatives containing flanking sequences, fragments of said DNA sequences and mutants, especially artificial mutants.

Larger derivatives of the novel DNA sequences are those excisable from the A. niger genome and comprising the DNA sequences which hybridize to the coding region for the mature PGII or PGI comprised by the Aspergillus niger DNA insert of pGW1800 or pGW1900, which code for a polypeptide with polygalacturonase activity, and which may contain the same or different promoter, signal and/or transcriptional terminator sequences as the Aspergillus niger DNA insert of pGW1800 or PGW 1900. Such derivatives can be found in a genomic library of A. niger N400 or NW756 obtained by fragmentation of the nucleic acids, treatment of the fragments with a suitable restriction enzyme, e.g. EcoRI, BaMHI or HindIII, ligating into a suitable vector, e.g. the lambda phage or the plasmid pBR322, cloning, e.g. in E. coli, and excising again with a suitable restriction enzyme.

A preferred example for a derivative of a DNA sequences hybridizing with the coding region of the A. niger N400 PGI or PGII gene under "heterologous" conditions is an insert or fragment thereof of a vector selected from the group of vectors consisting of λPG-A9, -A10, -A43, -B4, -B35, -B36, -C1, -C16, -C20, -C37, -D8, -D11, -D12, -E6, -E38, -F5, -G17, -G27, -X31 and -Y33, pGW1756 and pGW1910. The latter one comprises the structural gene pga C encoding A. niger N400 PGC derived from λPG-C20. pGW1756 (Figure 6) comprises the A. niger NW756 derived PGII gene which shows a restriction pattern and a DNA sequence (see SEQ ID NO. 3) distinct from the A. niger N400 PGII gene. The strains NW756 and NW400 are therefore not closely related and might belong to different species of filamentous fungi. Accordingly, the A. niger N400 PGI or PGII structural gene can specifically hybridize under heterologous conditions also with PG genes of other species.

Plasmid pGW1756 is composed of an about 4000 bp long pEMBL18 vector fragment and an about 5500 bp long A. niger NW756 DNA fragment. The PGII encoding gene pga II is located within an about 3300 bp long HincII restriction fragment. pGW1756 is deposited with the DSM.

The promoter region of the A. niger NW756 pga II extends before nucleotide 889 in the DNA sequence with SEQ ID NO. 3 given in the sequence listing. The coding region for the leader peptide of prepro-PGII presumably extends from position 890 up to 970 whereas the signal peptide is encoded by nucleotides 890 to 952. The coding region for the mature protein starts with nucleotide 971, ends with the stop codon at position 2028 and contains presumably one intron. The coding region is followed by the transcriptional terminator region which starts at position 2031.

Plasmid pGW1910 (Figure 7) consists of an approximately 7800 bp long insert derived from λPG-C20 and of the vector pUC9. Within the A. niger N400 insert the polygalacturonase gene pga C is located. The DNA sequence of nearly the entire pga C gene is given in the sequence listing under SEQ ID NO. 4.

The promoter of the A. niger N400 pga C extends before nucleotide 663. The leader peptide of the prepro-PG encoded by pga C extends presumably from nucleotide 663 up to 782 and includes the DNA sequence from 663 up to 710 which codes for the signal peptide. The mature PG is encoded by the sequence extending from nucleotide 783 up to 1995 and including several introns. The transcriptional terminator region starts after the stop codon at position 1999.

Derivatives of hybridizing DNA sequences encompassed by the present invention comprise the same promoter, signal and/or transcriptional terminator sequence as in pGW1800 or as in pGW1900, or another promoter, signal and/or transcriptional terminator sequence which may be derived from another gene of the polygalacturonase gene family. Furthermore, any other promoter, signal and/or transcriptional terminator sequence may be attached to the hybridizing DNA sequences depending on the host in which the desired PG is to be expressed.

A wide variety of promoters signal sequences and transcriptional terminators may be employed. Promoters are commonly available from 5′ noncoding regions of prokaryotic, eukaryotic or viral genes, whereas transcriptional terminators are available from 3′ non-coding regions. Signal sequences can be obtained from the 5′ coding region of preproteins which are introduced into the secretory pathway of the cell. Examples for promoters are given hereinafter.

A signal sequence in context with the present invention is a DNA sequence encoding a signal peptide or a leader peptide of a prepro-PG of the invention.

Flanking sequences within the meaning of the invention are also DNA fragments which are not derived from sequences flanking a PG gene in the genome. Such flanking sequences, for example, have regulatory functions, e.g. promoter function, or encode a polypeptide, e.g. a structural gene, or are linkers which put regulatory sequences and structural gene into the correct reading frame or distance, or sequences derived from a vector, e.g. a phage or plasmid, used in the construction of an expression plasmid. Such flanking sequences may give rise to expression cassettes or fusion genes.

The term "fragment" when used in connection with the novel DNA is intended to include also fragments of the larger derivatives, particularly those retaining promoter, signal, structural or transcriptional terminator functions. They may extend between two restriction sites.

Preferred DNA fragments are those containing a promoter region, encoding a leader or signal peptide of a prepro-PG, or encoding a polypeptide with polygalacturonase activity, or containing a transcriptional terminator region. Accordingly, a DNA sequence of the invention is also such containing any combination of said fragments, e.g. those containing a promoter, and/or a coding region for a leader or signal peptide and/or for a PG and/or a transcriptional terminator, and the like.

A fragment comprising a PG promoter region extends in the DNA sequence before a prepro-PG structural gene up to about 2000, preferably up to about 500 to 1400 nucleotides upstream. A promoter region binds RNA polymerase as well as regulatory proteins.

A fragment comprising a promoter region of the PGI gene pga I extends, for example, from position 1 up to position 909 of the sequence with the SEQ ID NO. 1. A fragment comprising a promoter region of the PGII gene pga II of A. niger N400 extends, for example, from position 1 up to position 1356 of the sequence with the SEQ ID NO. 2. In the A. niger NW756 insert of pGW1756 a fragment comprising a promoter region of pgaII extends, for example, from position 1 up to 889 of the sequence with SEQ ID NO. 3. In the A. niger insert of pGW1910, a fragment comprising a promoter region of pga C extends from position 1 to 662 of the sequence with SEQ ID NO. 4.

A fragment comprising the DNA sequence coding for the leader peptide of a prepro-PG extends for example between the end of the promoter and the beginning of the sequence coding for the mature protein. A coding region for a signal peptide is shorter than for the corresponding leader peptide. It is also starting at the end of the promoter and extends up to a coding region for a signal peptidase cleavage site. In the A. niger N400 prepro-PGII, the leader peptide comprises 27 amino acids. A DNA fragment encoding the leader peptide extends for example from the ATG codon at position 1357 up to the XhoI cleaving site at position 1429 of the DNA sequence with SEQ ID NO. 2. In prepro-PGI the leader peptide has 31 amino acids. A DNA fragment coding for this leader peptide extends for example between positions 910 and 1002 of the DNA sequence with the SEQ ID NO. 1. The leader peptide of the A. niger NW756 prepro-PGII has presumably 27 amino acids and thus the DNA fragment encoding the leader peptide is the 81-bp long DNA fragment extending between position 889 and 971 in the DNA sequence with SEQ ID NO. 3. The DNA fragment encoding the probably 40 amino acid leader peptide of the pga C product of A. niger N400 extends from base position 663 up to 782 of the sequence with SEQ ID NO. 4.

The coding regions for the respective signal peptides are, for example, located on following DNA fragments: Base positions 911 to 963 in SEQ ID NO. 1 1358 to 1419 in SEQ ID NO. 2, 890 to 952 in SEQ ID NO. 3 and 663 to 710 in SEQ ID NO. 4.

A fragment comprising the entire coding region for mature PGI extends, for example, from position 1003 up to position 2127 of the sequence with SEQ ID NO. 1. A fragment comprising the entire coding region for mature A. niger N400 PGII extends, for example, from position 1430 up to 2497 of the sequence with SEQ ID NO. 2. Mature A. niger NW756 PGII is encoded, for example, by the DNA fragment extending from position 953 to 2027 of the sequence with SEQ ID NO. 3 and mature pga C gene product is encoded, for example, by the DNA fragment located between bases No. 782 and 1996 of the sequence with SEQ ID NO. 4. However, also shorter fragments may encode polypeptides retaining PG activity.

The structural genes of PGII or PGI as well as of other PGs may contain introns like many fungal genes. However, included within the scope of the present invention are also intron-free structural genes of PGs, e.g. such which do not contain introns as genomic DNA sequence or also such which can be obtained from cDNA.

A fragment comprising a transcriptional terminator region extends, for example, from a stop codon, e.g. TAA, TAG or TGA, at the end of the coding region for a PG, up to about 300 to 2000, preferentially up to about 500 to 1000 base pairs in the downstream direction.

Such a fragment, for example, extends from base position 2131 up to 2495 of the sequence with SEQ ID NO. 1, from 2498 to 3031 in the sequence with SEQ ID NO. 2, from 2031 to 3047 in the sequence with SEQ ID NO. 3, and from 1999 to 2304 in the sequence with SEQ ID NO.4.

However, the fragments mentioned hereinbefore may be extended by the naturally 5′ flanking sequences or they may be shortened at the 5′ or 3′ end and nevertheless can retain promoter or transcriptional terminator activity, or the polypeptides encoded can retain signal peptide or galacturonase activity. Such fragments are also included within the scope of the invention.

The fragments hereinbefore may contain or may be flanked by linkers which provide for successful linkage to other DNA molecules and/or put DNA fragments having regulatory functions or encoding a polypeptide, e.g. promoter and structural gene, into the correct reading frame or distance.

Suitable linkers to above fragments have a DNA sequence which fits into the restriction site of the DNA to which the fragment is to be linked. They may contain a predetermined restriction site.

Mutants of a DNA sequence of the invention are e.g. naturally occurring mutants. The invention comprises also natural or synthetic mutants of the coding region for the signal or leader peptides with a similar or identical hydrophobicity profile, e.g. wherein the codons for the polar amino acids histidine ($H^{\delta +}$), tyrosine ($^{Y\delta -}$) and arginine ($R^{\delta +}$) are exchanged by codons for other amino acids having similar charges, and the hydrophobic amino acids alanine (A), leucine (L) and threonine (T), are replaced by codons for other hydrophobic amino acids. For example, the codon for the positively charged lysine may be replaced by a codon for arginine and vice versa, the codon for the negatively charged tyrosine by a codon for glutamate or aspartate, and/or the codon for the non-polar, hydrophobic alanine by any one of the codons for threonine, proline, valine, isoleucine, leucine, methionine or phenylalanine, and the like.

A recombinant DNA molecule of the invention also comprises DNA sequences which are degenerated within the meaning of the genetic code in that an unlimited number of nucleotides are replaced by other nucleotides without changing the amino acid sequence for which they code. Such degenerate DNA sequences may be useful because of their different restriction sites or because of preferred codon usage in a particular host.

A recombinant DNA molecule of the present invention preferentially comprises the Aspergillus niger DNA insert of a hybrid vector selected from the group of hybrid vectors consisting of pGW1800, pGW 1900, pGW 1756, pGW 1910, λPG-A9, λPG-A10, λPG-A43, λPG-B4, λPG-B35, λPG-B36, λPG-C1, λPG-C16, λPG-C20, λPG-C37, λPG-D8, λPG-D11, λPG-D12, λPG-E6, λPG-E38, λPG-F5, λPG-G17, λPG-G27, λPG-X31 and λPG-Y33 or a fragment thereof comprising a promoter region of a PG gene, or a coding region for a signal peptide, leader peptide, prepro-PG, PG or a fragment of a

PG with polygalacturonase activity, or a transcriptional terminator region of a PG gene. The inserts themselves are also covered by the present invention.

The invention also concerns recombinant DNA molecules which are expression cassettes comprising a promoter region, a DNA fragment encoding a leader or signal peptide, a structural gene and/or a transcriptional terminator region of the invention, preferentially such derived from a vector selected from the group of vectors mentioned hereinbefore.

The term "expression cassette" means a DNA sequence capable of expressing a polypeptide and comprises a promoter, if desired a signal sequence, further a structural gene, if desired, a transcriptional terminator and optionally a transcriptional enhancer, ribosomal binding site and/or further regulatory sequences.

In an expression cassette of the invention, the PG gene derived functional fragments may be combined with functional fragments derived from other genes.

A wide variety of promoter sequences may be employed, depending on the nature of the host cell. Promoters that are strong and at the same time well regulated are the most useful. Sequences for the initiation of translation are for example Shine-Dalgarno sequences. Sequences necessary for the initiation and termination of transcription and for stabilizing the mRNA are commonly available from the noncoding 5′-regions and 3′-regions, respectively, of viral or eukaryotic cDNAS, e.g. from the expression host.

Examples for promoters are $\lambda P_L$, $\lambda P_R$, E. coli lac, trp, tac, yeast TRP1-, ADHI-, ADHII-, PHO3-, PHO5-, or glycolytic promoters such as the promoter of the enolase, glyceraldehyde-3-phosphate dehydrogenase, 3-phosphoglycerate kinase (PGK), hexokinase, pyruvate decarboxylase, phosphofructokinase, glucose-6-phosphate isomerase, 3-phosphoglycerate mutase, pyruvate kinase, triosephosphate isomerase, phosphoglucose isomerase and glucokinase genes, or promoters derived from eukaryotic viruses, e.g. SV40, Rous sarcoma virus, adenovirus 2, bovine papilloma virus, papovavirus, cytomegalovirus derived promoters or mammalian cell derived promoters, e.g. of the actin, collagen, myosin, or β-globin gene. The eukaryotic promoters may be combined with enhancing sequences such as the yeast upstream activating sequences (UAS) or viral or cellular enhancers such as the cytomegalovirus IE enhancers, SV40 enhancer, immunoglobulin gene enhancer or others.

Enhancers are transcription-stimulating DNA sequences, e.g. derived from viruses such as Simian virus, polyoma virus, bovine papilloma virus or Moloney sarcoma virus, or of genomic origin. An enhancer sequence may also be derived from the extrachromosomal ribosomal DNA of Physarum polycephalum (PCT/EP 8500278), or it may be the upstream activation site from the acid phosphatase PH05 gene (EP Appl. No. 86 111 820.6), or the PH05, trp, PH05-GAPDH hybrid (EP Appl. No. 86 111 820.6), or the like promoter.

Signal sequences may be, for example, a presequence or secretory leader directing the secretion of the polypeptide, or the like. A signal sequence is, for example, a signal or leader peptide of prepro-PGI, prepro-PGII or prepro-PGC. Further signal sequences are known from literature, e.g. those compiled in von Heijne, G., Nucleic Acids Res. 14 ,4683 (1986).

Structural genes in this context are, besides those coding for PGI or PGII, also those coding for other PGs or derivatives, in particular fragments, thereof having PG activity or other Aspergillus genes and structural genes which originate from viruses, procaryotic cells or eucaryotic cells and which may be derived from genomic DNA or from cDNA prepared via the mRNA route or may be synthesized chemically, coding for a wide variety of useful polypeptides, including glycosylated polypeptides, in particular of higher eukaryotic, especially mammalian, such as animal or especially human origin, such as enzymes which can be used, for example, for the production of nutrients and for performing enzymatic reactions in chemistry, or polypeptides, which are useful and valuable for the treatment of human and animal diseases or for the prevention thereof, for example, hormones, polypeptides with immunomodulatory, anti-viral and anti-tumor properties, antibodies, viral antigens, vaccines, clotting factors, foodstuffs and the like.

Examples of such structural genes are e.g. those coding for hormones such as secretin, thymosin, relaxin, calcitonin, luteinizing hormone, parathyroid hormone, adrenocorticotropin, melanoycte-stimulating hormone, β-lipotropin, urogastrone or insulin, growth factors, such as epidermal growth factor, insulin-like growth factor (IGF), e.g. IGF-I and IGF-II, mast cell growth factor, nerve growth factor, glia derived nerve cell growth factor, or transforming growth factor (TGF), such as TGFβ, growth hormones, such as human or bovine growth hormones, interleukin, such as interleukin- 1 or -2, human macrophage migration inhibitory factor (MIF), interferons, such as human α-interferon, for example interferon-αA, αB, αD or αF, β-interferon, λ-interferon or a hybrid interferon, for example an αA-αD- or an αB-αD-hybrid interferon, especially the hybrid interferon BDBB, proteinase inhibitors such as $\alpha_1$-antitrypsin, SLPI and the linke, hepatitis virus antigens, such as hepatitis B virus surface or core antigen or hepatitis A virus antigen, or hepatitis nonA-nonB antigen, plasminogen activators, such as tissue plasminogen activator or urokinase, tumour necrosis factor, somatostatin, renin, β-endorphin, immunoglobulins, such as the light and/or heavy chains of immunoglobulin D, E or G, or human-mouse hybrid immunoglobulins, immunoglobulin binding factors, such as immunoglobulin E binding factor, calcitonin, human calcitonin-related peptide, blood clotting factors, such as factor IX or VIIIc, erythropoietin, eglin, such as eglin C, hirudin, desulfatohirudin, such as desulfatohirudin variant HV1, HV2 or PA, human superoxide dismutase, viral thymidin kinase, β-lactamase, glucose isomerase. Preferred genes are those coding for a human α-interferon or hybrid interferon, particularly hybrid interferon BDBB, human tissue plasminogen activator (t-PA), hepatitis B virus surface antigen (HBVsAg), insulin-like growth factor I and II, eglin C and desulfatohirudin, e.g. variant HV1. In the hybrid vectors of the present invention, the present promoter and/or signal sequence is operably linked to the polypeptide coding region so as to ensure effective expression of the polypeptide.

The invention also concerns recombinant DNA molecules which are recombinant vectors, alternatively called hybrid vectors, which comprise a DNA sequence selected from

a) the Aspergillus niger DNA insert of pGW1800 or pGW1900,

b) a DNA sequence which hybridizes to the coding region for the mature PGII or PGI comprised by a DNA insert of a),

c) a derivative of a DNA sequence defined in a) or b), or

d) a DNA sequence of a), b) or c) which codes for a mature PG or a signal peptide or a leader peptide thereof and which is degenerate within the meaning of the genetic code.

They are usable for cloning and/or expression in hosts, such as bacteria, fungi or animal cells.

Such hybrid vectors may be derived from any vector useful in the art of genetic engineering, such as from viruses, phages, cosmids, plasmids or chromosomal DNA, such as derivatives of SV40, Herpes-viruses, Papilloma viruses, Retroviruses, Baculovirus, phage λ, e.g. NM 989 or EMBL4, or phage M13, e.g. M13mp8 phage DNA (ref. 15) linearized by BamHI digestion, bacterial plasmids, e.g. pBR322, pUC18, or yeast plasmids, e.g. yeast 2μ plasmid, or also chromosomal DNA, derived e.g. from filamentous fungi such as Aspergillus spec ., e.g. A. niger , for example those provided by EP 184 438, or a defective virus, phage or plasmid in the presence of a helper virus, phage or plasmid allowing replication of said defective virus, phage or plasmid, e.g. M13(+)KS vector in presence of e.g. M14K07 helper phage.

The hybrid vectors of the invention provide for replication and optionally expression of a desired DNA in a suitable host, either as an extrachromosomal element or by integration in the host chromosome. Several possible vector systems are available for integration and expression of the cloned DNA of the invention. In principle, all vectors which replicate and/or express a desired polypeptide gene comprised in an expression cassette of the invention in the chosen host are suitable. The vector is selected depending on the host cells envisaged for transformation. In general, such host cells may be prokaryotic or eukaryotic microorganisms such as bacteria, fungi such as yeasts or filamentous fungi, or cells of higher eukaryotic origin such as vertebrate, for example mammalian, cells. Suitable host cells will be discussed in detail hereinbelow. In principle, the hybrid vectors of the invention comprise the DNA as defined hereinbefore, an origin of replication or an autonomously replicating sequence, dominant marker sequences, optionally expression control sequences essential for the transcription and translation of the desired DNA and, optionally, for the secretion of the desired product and, optionally, additional restriction sites.

An origin of replication or an autonomously replicating sequence (a DNA element which confers autonomously replicating capabilities to extrachromosomal elements) is provided either by construction of the vector to include an exogeneous origin such as derived from Simian virus (SV 40) or another viral source, or by the host cell chromosomal mechanisms.

A hybrid vector of the invention molecules of the present invention may contain selective markers depending on the host which is to be transformed, selected and cloned. Any marker gene can be used which facilitates the selection of transformants due to the phenotypic expression of the marker. Suitable markers are particularly those expressing antibiotic resistance, e.g. against tetracycline or ampicillin, or, in the case of auxotrophic fungal mutants, genes which complement host lesions. Corresponding genes confer, for example, resistance to the antibiotic cycloheximide, or provide for prototrophy in an auxotrophic yeast mutant, for example the ura3 , leu2 , his3 or trp1 gene. It is also possible to employ as markers structural genes which are associated with an autonomously replicating segment providing that the host to be transformed is auxotrophic for the product expressed by the marker.

Of particular importance are marker genes which complement A. niger host lesions, such as the arg B gene coding for the ornithine carbamoyl transferase, e.g. derived from A. niger or A. nidulans (EP 184 438), or A. nidulans DNA fragments homologous to the N. crassa pyr4 gene (ref. 27).

Preferred embodiments of the present invention are hybrid vectors comprising an expression cassette of the invention, e.g. such comprising the structural gene and/or the promoter and/or the DNA sequence coding for a leader or signal peptide and/or the transcriptional terminator derived from a PG gene of the invention.

Examples of hybrid vectors comprising an expression cassette of the invention are pGW1800, pGW1900, pGW1910, pGII-IFN AM119, pGIIss-IFN AM119, pGW1756, λPG-A10, λPG-A43, λPG-D8 and λPG-D11.

Subject of the invention is not only a recombinant DNA molecule comprising a PG gene derived insert defined hereinbefore, but also a DNA molecule comprising a PG gene or a functional fragment thereof, e.g. such comprising promoter, coding region for signal peptide, leader peptide or a protein with PG activity or transcriptional terminator region itself which can be isolated from a PG producing fungal strain, for example, from Aspergillus spec ., e.g. A. japonicus , A. oryzae , A. nidulans , A. niger , Trichoderma spec ., Botrytis spec ., Sclerotinia spec ., Fusarium spec. or other phytopathogenic fungi as well as from yeast, for example from PG producing Kluyveromyces spec . such as K. fragilis . Preferred are DNA molecules isolated from A. niger.

The DNA molecules of the invention and derivatives thereof, including fragments, can be used for screening DNA gene libraries or mRNA for further similar DNAs or mRNAS.

## Process for the Preparation of the Recombinant DNA Molecules

Further object of the invention is a process for the preparation of a recombinant DNA molecule defined hereinbefore comprising culturing a host transformed with such a recombinant DNA molecule or preparing it by an in vitro synthesis.

The culturing of the hosts is carried out in a conventional nutrient medium which may be supplemented with or deprived of chemical compounds allowing negative or positive selection of the transformants, i.e.

such hosts containing the desired DNA molecule together with a selection marker, from the non-transformants, i.e. such hosts lacking the desired DNA molecule.

Any transformable hosts useful in the art may be used, e.g. bacteria, such as E. coli , fungi, such as Saccharomyces cerevisiae , Kluyveromyces lactis , or in particular filamentous fungi, such as Aspergillus , e.g. A. nidulans , A. oryzae , A. carbonarius , A. awamori and especially A. niger . A preferred host is A. niger An8 , a mutant lacking the pyr A gene, as described further below, or A. niger N593. Transformation of the hosts is carried out by conventional methods.

A DNA sequence comprised in a recombinant DNA molecule of the invention can be obtained from the genome of a fungus expressing polygalacturonase, or can be prepared for example by culturing a host which is transformed with a recombinant DNA molecule of the invention and, when required, isolating the desired DNA sequence therefrom, or by chemical synthesis through nucleotide condensation.

In particular, such DNAs can be prepared by

a) isolating genomic DNA from suitable fungal cells, and selecting the desired DNA, e.g. using a DNA probe or using a suitable expression system and screening for expression of the desired polypeptide, or

b) isolating mRNA from suitable fungal cells, selecting the desired mRNA, e.g. by hybridization with a DNA probe or by expression in a suitable expression system and screening for expression of the desired polypeptide, preparing single-stranded cDNA complementary to that mRNA, then double-stranded cDNA therefrom, or

c) isolating cDNA from a cDNA library and selecting the desired cDNA, e.g. using a DNA probe or using a suitable expression system and screening for expression of the desired polypeptide, or/and

d) incorporating the double-stranded DNA of step a), b) or c) into an appropriate vector,

e) transforming appropriate host cells with the obtained hybrid vector,

f) selecting transformed host cells which contain the desired DNA from untransformed host cells and multiplicating the transformed host cells, and, when required,

g) isolating the desired DNA and/or converting the DNA into a mutant or fragment thereof.

Genomic DNA may be isolated and screened for the desired DNA (step a). Genomic DNA is isolated from suitable fungal strain expressing proteins with PG activity. A genomic DNA library is prepared therefrom by digestion with suitable restriction endonucleases and incorporation into suitable vectors following established procedures. The genomic DNA library is screened with a DNA probe as described hereinafter, or expressed in a suitable expression system and the obtained polypeptides screened in conventional manner. A detailed description of the isolation of a DNA molecule of the invention from a genomic library is given hereinafter.

Polyadenylated messenger RNA (step b) is isolated from the suitable cells, by known methods. Isolation methods involve, for example, homogenizing in the presence of a detergent and a ribonuclease inhibitor, e.g. heparin, guanidinium isothiocyanate or mercaptoethanol, extracting the mRNA with suitable chloroform-phenol mixtures, optionally in the presence of salt and buffer solutions, detergents and/or cation chelating agents, and precipitating mRNA from the remaining aqueous, salt-containing phase with ethanol, isopropanol or the like. The isolated mRNA may be further purified by centrifuging in a cesium chloride gradient followed by ethanol precipitation and/or by chromatographic methods, e.g. affinity chromatography, for example chromatography on oligo(dT) cellulose or on oligo(U) sepharose. Preferably, such purified total mRNA is fractionated according to size by gradient centrifugation, e.g. in a linear sucrose gradient, or chromatography on suitable size fractionation columns, e.g. on agarose gels.

The desired mRNA is selected by screening the mRNA directly with a DNA probe, or by translation in suitable cells or cell-free systems and screening the obtained polypeptides.

The selection of the desired mRNA is preferably achieved using a DNA hybridization probe, thereby avoiding the additional step of translation. Suitable DNA probes are DNAs of known nucleotide sequence consisting of at least 17 nucleotides, for example synthetic DNAs, cDNAs derived from mRNA coding for the desired polypeptides, or genomic DNA fragments comprising e.g. adjacent DNA sequences which are isolated from a natural source or from a genetically engineered microorganism.

Synthetic DNA probes are synthesized according to known methods as detailed hereinbelow, preferably by stepwise condensation using the solid phase phosphotriester, phosphite triester or phosphoramidite method, e.g. the condensation of dinucleotide coupling units by the phosphotriester method. These methods are adapted to the synthesis of mixtures of the desired oligonucleotides by using mixtures of two, three or four nucleotides dA, dC, dG and/or dT in protected form or the corresponding dinucleotide coupling units in the appropriate condensation step as described by Y. Ike et al. (Nucleic Acids Research 11 , 477,1983).

For hybridization, the DNA probes are labelled, e.g. radioactively labelled by the well known kinase reaction. The hybridization of the size-fractionated mRNA with the DNA probes containing a label is performed according to known procedures, i.e. in buffer and salt solutions containing adjuncts, e.g. calcium chelators, viscosity regulating compounds, proteins, non-homologous DNA and the like, at temperatures favoring selective hybridization, e.g. between 0 °C and 80 °C, for example between 25 °C and 50 °C or around 65 °C, preferably at around 20 ° lower than the hybrid double-stranded DNA melting temperature.

Fractionated mRNA may be translated in cells, e.g. frog oocytes, or in cell-free systems, e.g. in reticulocyte lysates or wheat germ extracts. The obtained polypeptides are screened for enzymatic activity or for reaction with antibodies raised against the native polypeptide, e.g. in an immunoassay, for example radioimmunoassay, enzyme immnoassay or immunoassay with fluorescent markers. Such immunoassays and the preparation of polyclonal and monoclonal antibodies are well known in the art and are applied accordingly.

The preparation of a single-stranded complementary DNA (cDNA) from the selected mRNA template is well known in the art, as is the preparation of a double-stranded DNA from a single-stranded DNA. The mRNA template is incubated with a mixture of deoxynucleoside triphosphates, optionally radioactively labelled deoxynucleoside triphosphates (in order to be able to screen the result of the reaction), a primer sequence such as an oligo-dT residue hybridizing with the poly(A) tail of the mRNA and a suitable enzyme such as a reverse transcriptase e.g. from avian myeloblastosis virus (AMV). After degradation of the template mRNA e.g. by alkaline hydrolysis, the cDNA is incubated with a mixture of deoxynucleoside triphosphates and a suitable enzyme to give a double-stranded DNA. Suitable enzymes are for instance a reverse transcriptase, the Klenow fragment of E. coli DNA polymerase I or T4 DNA polymerase. Usually, a hairpin loop stucture formed spontaneously by the single-stranded cDNA acts as a primer for the synthesis of the second strand. This hairpin structure is removed by digestion with S1 nuclease. Alternatively, the 3′-end of the single-stranded DNA is first extended by homopolymeric deoxynucleotide tails prior to the hydrolysis of the mRNA template and the subsequent synthesis of the second cDNA strand.

In the alternative, double-stranded cDNA is isolated from a cDNA library and screened for the desired cDNA (step c). The cDNA library is constructed by isolating mRNA from suitable cells, and preparing single-stranded and double-stranded cDNA therefrom as described above. This cDNA is digested with suitable resctriction endonucleases and incorporated into λ phage, e.g. λ charon 4A or λ gt11 following established procedures. The cDNA library replicated on nitrocellulose membranes is screened by using a DNA probe as described hereinbefore, or expressed in a suitable expression system and the obtained polypeptides screened for reaction with an antibody specific for the desired compounds.

A variety of methods are known in the art for the incorporation of double-stranded cDNA or genomic DNA into an appropriate vector (step d). For example, complementary homopolymer tracts may be added to the double-stranded DNA and the vector DNA by incubation in the presence of the corresponding deoxynucleoside triphosphates and an enzyme such as terminal deoxynucleotidyl transferase. The vector and double-stranded DNA are then joined by base pairing between the complementary homopolymeric tails and finally ligated by specific joining enzymes such as ligases. Other possiblities are the addition of synthetic linkers to the termini of the double-stranded DNA, or the incorporation of the double-stranded DNA into the vector by blunt- or staggered-end ligation. Appropriate vectors will be discussed in detail hereinbelow.

The transformation of appropriate host cells with the obtained hybrid vector (step e) and the selection and multiplication of transformed host cells (step f) are well known in the art. Examples for such methods are given further below. Hybrid vectors and host cells may be particularly suitable for the production of DNA, or else for the production of the desired polypeptides.

The isolation of the desired DNA, mutants and fragments therof according to the invention is achieved by methods known in the art, e.g. extraction with phenol and/or chloroform. Optionally, the DNA can be further manipulated e.g. by treatment with mutagenic agents to obtain mutants, or by digestion with restriction enzymes to obtain fragments, modify one or both termini to facilitate incorporation into the vector, remove intervening sequences and the like.

The nucleotide sequence of a DNA according to the invention can be determined by methods known per se , for example by the Maxam-Gilbert method using end-labelled DNA or by the dideoxy chain termination method of Sanger.

PG gene sequences of the present invention can also be prepared by an in vitro synthesis according to conventional methods. The in vitro synthesis is especially applicable for the preparation of smaller fragments of a PG expression cassette, e.g. of the DNA sequences of a PG gene coding for a promoter or a signal peptide, for example of the PGI, PGII or the pgaC gene, of a PG gene comprised in a DNA molecule defined hereinbefore, especially in the λ-clones, or a mutant thereof.

Suitable methods for the synthesis of DNA have been presented in summary form by S.A. Narang (Tetrahedron 39 , 3, 1983). The known synthesis techniques allow the preparation of polynucleotides up to 120 bases in length, in good yield, high purity and in a relatively short time. Suitably protected nucleotides are linked with one another by the phosphodiester method (K.L. Agarwal et al., Angew. Chemie 84 , 489, 1972), the more efficient phosphotriester method (C.B. Reese, Tetrahedron 34 , 3143, 1972), the phosphite triester method (R.L. Letsinger et al., J. Am. Chem. Soc 98 3655, 1976) or phosphoramidite method (S.L. Beaucage and M.H. Carruthers, Tetrahedron 22 , 1859, 1981). Simplification of the synthesis of the oligonucleotides and polynucleotides is made possible by the solid phase method, in wich the nucleotide chains are bound to a suitable polymer. H. Rink et al. (Nucl. Acids Research 12 , 6369, 1984) use trinucleotides instead of individual nucleotides and link them by the phosphotriester method in the solid phase synthesis. A polynucleotide can thus be prepared in a short time and with good yields. The actual double-stranded DNA is built up enzymatically from chemically prepared over-lapping oligonucleotides from both DNA strands, which are held together in the correct arrangement by base-pairing and are then chemically linked by the enzyme DNA ligase.

Another possibility comprises incubating overlapping single oligonucleotides from the two DNA strands in the presence of the four required deoxynucleoside triphosphates with a DNA polymerase, for example DNA polymerase I, the Klenow fragment of polymerase I or T4 DNA polymerase, or with AMV (avian myeloblastosis virus) reverse transcriptase. The two oligonucleotides are thereby held together in the correct arrangement by base-pairing and are supplemented with the required nucleotides by the enzyme to give a complete double-stranded DNA (S.A. Narang et al., Anal. Biochem. 121 , 356, 1982).

In the following the preparation of a recombinant DNA molecule of the present invention from a genomic library and homologous and heterologous hybridization conditions for identifying members of the PG gene family are described in more detail.

A genomic library can be prepared e.g. by partial digestion of genomic DNA of an A. niger strain, e.g. NW756 or N400, with e.g. Sau 3AI or MboI, and cloning the high molecular weight DNA fragments in a suitable host vector, e.g. the E. coli plasmid pUN121 or a lambda vector, e.g. EMBL4.

Other fungal strains producing the desired PGs, for example, Aspergillus spec. , e.g. A. japonicus , A. oryzae , A. nidulans , A. niger , Trichoderma spec. , Botrytis spec ., Sclerotinia spec ., Fusarium spec. or other phytopathogenic fungi as well as yeast, for example PG producing Kluyveromyces spec . such as K. fragilis may serve as source for the genomic library and other suitable vectors, e.g. those mentioned hereinbefore, may be used as recipient for the fragments.

In order to successfully screen the genomic library for DNA sequences coding for PGs a hybridizing DNA probe is necessary. This can be a synthetic DNA probe if the amino acid sequence or part thereof of the desired PG is known, or another PG gene or a part thereof, which hybridizes to a desired PG gene. As neither a PG sequence nor a PG gene or part thereof were known prior to the invention, the problems of purification of PGs, structure determination of the N-terminal sequence of a PG and preparation of useful DNA probes were solved first.

For the purification of the present PGs any source containing it may be used. For example, crude sources such as enzyme mixtures obtained from Aspergillus niger , such as Rapidase®, a commercially available mixture of pectinolytic enzymes, may serve as source.

The purification follows conventional purification methods, such as salting out, desalting, reprecipitation in form of a different salt, chromatography, e.g. affinity chromatography such as on a crosslinked alginate, ion exchange chromatography, e.g. on a DEAE-Sephadex or Sepharose column, gel permeation chromatography, e.g. on a Sephacryl column, electrophoresis, e.g. with SDS-polyacrylamide gel, isoelectric focusing, and the like, or any combination thereof.

In the present invention, PGI, PGII, PGIIIA, PGIIIB and PGIV were isolated in a pure form from Rapidase®. Said PGs are endo-polygalacturonases (E.C. 3.2.1.15) with following physico-chemical properties: PGI has a Mr of 55 K, an isoelectric point (IEP) of between 3.2 and 3.5 and a pH-optimum for the enzymatic activity of 4.9. The values for PGII are a Mr of 38 K, an IEP of between 4.6 and 5.9, a pH-optimum of 4.8; PGIIIA has a Mr of 57 K, an IEP of 3.3 and a pH-optimum of 4.3, PGIIIB has also 57 K and an IEP of 3.3, however, the pH-optimum is 4.5. Finally, PGIV has a Mr of 59 K, an IEP of 3.7 and a pH-optimum of 4.8. The Mr values given are determined by SDS-polyacrylamide gel electrophoresis, the IEP values by thin-layer isoelectric focusing.

The N-terminal sequencing of PGI revealed following sequence:

$ala^1$-ser-thr-$X^4$-$thr^5$-phe-thr-ser-$ala^9$,

The N-terminal sequencing of a 21 kDa BrCN-fragment of PGI revealed the sequence ala-ser-thr-$X^4$-thr-phe-thr-ser-ala-ser-glu,

i.e. the N-terminal sequence of PGI, and sequencing of a 5.5 kDa BrCN-fragment revealed the sequence ala-asp-gly-ala-val-ile-asp-gly-asp-gly-ser.

The N-terminal sequencing of PG17 revealed the sequence
asp$^{1}$-ser-$X^{3}$-thr-phe$^{5}$-thr-thr-ala-ala-ala$^{10}$-ala-lys-ala$^{12}$
and of a BrCN-fragment of 17 kDa the sequence
ala-phe-ser-val-gln-ala-asn-asp-ile-thr-phe.

$X^{3}$ and $X^{4}$ in the sequences at the time were not identified.

Based on the amino acid sequence of the 5.5 kDa BrCN-fragment of PGI the following oligonucleotide mixture was synthesized

```
                      met  ala  asp   gly  ala  val
HR 6298 5'(d)  ATG  GCI  GAR1  GGI  GCI  GTI

                      ile   asp   gly  asp   gly
                      ATR3  GAR1  GGI  GAR1  GG      3'
```

Based on the amino acid sequence of the 17 kDa BrCN-fragment of PGII the following two oligonucleotide mixtures were synthesized

```
                    met  ala  phe   ser  val  gln   ala
HR6195  5' (d)  ATG  GCI  TTR1  TCI  GTI  CAR2  GCI
HR6196  5' (d)  ATG  GCI  TTR1  AGI  GTI  CAR2  GCI

        asn   asp   ile
HR6195  AAR1  GAR1  AT   3'
HR6196  AAR1  GAR1  AT   3'
```

In the sequences of the oligonucleotides, I is inosine, $R_1$ is T or C, $R_2$ is A or G and $R_3$ is T, C or A.

The mixtures were radioactively marked and used to screen a genomic library of Aspergillus niger N400 for PGI and PGII genes, respectively.

The identified genes or gene fragments are then sequenced according to conventional methods. The sequences of the PGI and PGII genes of A. niger N400 are represented by the sequences with SEQ ID NO. 1 and 2, respectively, depicted in the sequence listing.

For screening purposes the DNA probes are radioactively labelled by methods known in the art, e.g. using $\gamma^{32}$P-ATP and T4 kinase or $\alpha^{32}$P-dATP and E. coli DNA polymerase I, depending on the type of probe used. Host microorganisms carrying nucleic acids of the present invention as an insert, are identified by hybridization with the labelled DNA probe on filter replicas of the gene library.

Clones showing a hybridization response to one or more DNA probes are isolated and amplified.

The hybridization conditions used are conventional and can be more or less stringent.

Stringent conditions are such under which only DNA sequences with a high degree in homology can hybridize ("homologous" hybridization conditions). Under "heterologous" or less stringent conditions, also related DNA sequences with a lower degree in homology can hybridize. The stringency of the hybridization is influenced, for example, by the hybridization and washing temperature, formamide or salt concentration, G+C content of the DNA, length of the hybridization time and length of the DNA probe. Illustrative but not limiting examples of "homologous" and "heterologous" hybridization conditions are given hereinafter in the Examples.

With the use of one or more DNA probes derived from the PGI or PGII gene, particularly such comprising at least part of the coding regions thereof, hybridizing clones derived from a genomic library, e.g. of A. niger , preferentially of A. niger N400 or A. niger NW 756, can be identified and divided into different classes based on their degree of homology and on the basis of the hybridizing restriction fragments observed. Examples of preferred clones derived from a A. niger N400 library are λPG-A9, -A10, -A43, -B4, -B35, -B36, -C1, -C16, -C20, -C37, -D8, -D11, -D12, -E6, -E38, -F5, -G17, -G27, -X31 and -Y33.

Their preparation is described in detail hereinafter in the Examples. An Example for a preferred clone derived from A. niger NW756 is the plasmid pGW1756.

These clones as well as plasmids pGW1800, pGW1803, pGW1900, pGW1902 and pGW1910 can be used to prepare other recombinant DNA molecules of the invention, in particular such containing fragments of the PG gene sequences comprised therein. Said other recombinant DNA molecules are prepared in conventional manner by applying conventional restriction enzymes, linkers, ligation, amplification and isolation processes. Preferentially, expression vectors are prepared which may be pro- or eukaryotic vectors or shuttle vectors for propagation both in pro- and eukaryotic cells. Examples for the construction of such shuttle vectors are well known in the art. The expression vectors may contain homologous or heterologous genes. Examples of such genes are given hereinafter.

The inserts of the recombinant DNA molecules of the invention or fragments thereof can be obtained in conventional manner, e.g. after cleaving the recombinant DNA with suitable restriction enzymes and isolating the desired DNA fragments after agarose gel electrophoresis.

Mutants of the DNA molecules of the invention, particularly of the PG gene sequences, containing new restriction sites can also be prepared, for example in vitro by site-directed mutagenesis, according to conventional methods [see review article of M.J. Zoller and M. Smith, Methods Enzymol. 100, 468 (1983), D. Botstein and D. Shortle, Science 229 , 1193 (1985) or K. Norris et al., Nucl. Acids Res. 11 ,5103(1983)].

The invention concerns also the use of the recombinant DNA molecules of the invention for the preparation of hybrid vectors for the expression of a structural gene.

## Transformed hosts

Furthermore, the invention concerns transformed host cells for amplifying the recombinant DNA molecules of the invention or particularly for expressing an expression cassette comprised in a recombinant DNA molecule of the invention.

Examples of suitable hosts, particularly for amplifying the recombinant DNA molecules of the invention, are microorganisms which are devoid of or poor in restriction enzymes or modification enzymes, such as bacteria, in particular strains of Escherichia coli , for example E. coli X1776, E. coli Y1090, E. coli W3110, E. coli HB101/LM1035, E. coli JA 221, E. coli DH5α, or preferentially E. coli DH5αF′, JM109, MH1 or HB101, or E. coli K12 strain, Bacillus subtilis , Bacillus stearothermophilus , Pseudomonas , Haemophilus , Streptococcus and others, and yeasts, for example Saccharomyces cerevisiae such as S. cerevisiae GRF 18. Further suitable host cells are cells of higher organisms, in particular established continuous human or animal cell lines, e.g. human embryonic lung fibroblasts L132, human malignant melanoma Bowes cells, HeLa cells, SV40 virus transformed kidney cells of African green monkey COS-7 or Chinese hamster ovary (CHO) cells.

Examples of suitable hosts for expressing an expression cassette of the invention are the cells mentioned hereinbefore and in particular filamentous fungi, for example Penicillium, Cephalosporium or preferentially Aspergillus spec., e.g. A. carbonarius , A. awamori or preferentially A. niger , A. nidulans or A. oryzae .

Preferred transformed hosts are E. coli MH1 transformed with pGW1800 or pGW1803, E. coli JM109 transformed with pGW1800 or pGW1803, E. coli DH5αF′ transformed with pGW1900, 1902, 1756 or 1910, pGII-IFN AM119 or pGIIss-IFN AM119, Aspergillus niger An8 or N593 or Aspergillus nidulans transformed with pGII-IFN AM119 or pGIIss-IFN AM119 and optionally with the selection marker plasmid pCG59D7.

The invention concerns also a method for the preparation of such transformants comprising treatment of a suitable host cell under transforming conditions with a recombinant DNA molecule of the present invention, especially a hybrid vector of the invention, optionally together with a selection marker gene and optionally selecting the transformants.

Transformation of microorganisms is carried out according to conventional methods as described in the literature, for example for S. cerevisiae (A. Hinnen et al., Proc.Natl.Acad.Sci.USA, 75 , 1929,1978), for B. subtilis (Anagnostopoulos et al., J. Bacteriol. 81 , 741, 1961), and for E. coli (M. Mandel et al., J. Mol. Biol. 53 , 159, 1970).

Accordingly, the transformation procedure of E. coli cells includes, for example, $Ca^{2+}$ pretreatment of the cells so as to allow DNA uptake, and incubation with the hybrid vector. The subsequent selection of the transformed cells can be achieved, for example, by transferring the cells to a selective growth medium which allows separation of the transformed cells from the parent cells dependent on the nature of the marker sequence of the vector DNA. Preferably, a growth medium is used which does not allow growth of cells which do not contain the vector. The transformation of yeast comprises, for example, steps of enzymatic removal of the yeast cell wall by means of glucosidases, treatment of the obtained spheroplasts with the vector in the presence of polyethylene glycol and $Ca^{2+}$ ions, and regeneration of the cell wall by embedding the spheroplasts into agar. Preferably, the regeneration agar is prepared in a way to allow regeneration and selection of the transformed cells as described above at the same time.

Transformation of cells of higher eukaryotic origin, such as mammalian cell lines, is preferably achieved by transfection. Transfection is carried out by conventional techniques, such as calcium phosphate precipitation, microinjection, protoplast fusion, electroporation, i.e. introduction of DNA by a short electrical pulse which transiently increases the permeability of the cell membrane, or in the presence of helper compounds such as diethylaminoethyldextran, dimethyl sulfoxide, glycerol or polyethylene glycol, and the like. After the transfection procedure, transfected cells are identified and selected e.g. by cultivation in a selective medium chosen depending on the nature of the selection marker, for example standard culture media such as Dulbecco's modified Eagle medium (DMEM), minimum essential medium, RPMI 1640 medium and the like, containing e.g. the corresponding antibiotic.

The transformed host cells are cultured by methods known in the art in a liquid medium containing assimilable sources of carbon, e.g. carbohydrates such as glucose or lactose, nitrogen, e.g. amino acids, peptides, proteins or their degradation products such as peptones, ammonium salts or the like, and inorganic salts, e.g. sulfates, phosphates and/or carbonates of sodium, potassium, magnesium and calcium. The medium furthermore contains, for example, growth-promoting substances, such as trace elements, for example iron, zinc, manganese and the like.

The medium is preferably so chosen as to exert a selection pressure and prevent the growth of cells which have not been transformed or have lost the hybrid vector. Thus, for example, an antibiotic is added to the medium if the hybrid vector contains an antibiotic resistance gene as marker. If, for instance, a host cell is used which is auxotrophic in an essential amino acid whereas the hybrid vector contains a gene coding for an enzyme which complements the host defect, a minimal medium deficient of said amino acid is used to culture the transformed cells.

Cells of higher eukaryotic origin such as mammalian cells are grown under tissue culture conditions using commercially available media, for example Dulbecco's modified Eagle medium (DMEM), minimum essential medium, RPMI 1640 medium and the like as mentioned above, optionally supplemented with growth-promoting substances and/or mammalian sera. Techniques for cell cultivation under tissue culture condition are well known in the art and include homogeneous suspension culture, e.g. in an airlift reactor or in a continuous stirrer reactor, or immobilized or entrapped cell culture, e.g. in hollow fibres, microcapsules, on agarose microbeads, porous glass beads, ceramic cartridges, or other microcarriers.

Culturing is effected by processes which are known in the art. The culture conditions, such as temperature, pH value of the medium and fermentation time, are chosen so that a maximum titer of the polypeptide or derivative of the invention is obtained. Thus, an E. coli or yeast strain is preferably cultured under aerobic conditions by submerged culture with shaking or stirring at a temperature of about 20°C to 40°C, preferably at about 30°C, and a pH value of 4 to 8, preferably of about pH 7, for about 4 to 30 hours, preferably until maximum yields of the polypeptide or derivative of the invention are reached.

In order to allow selection of the transformed from the nontransformed cells, the DNA molecules of the invention carry a selection marker or, alternatively, the cells are cotransformed with a second vector containing such marker. As in other systems such selection marker is an expressible, structural gene, the expressed polypeptide of which (an enzyme) provides resistance against compounds toxic to the receipt organism or which completes the enzyme system of a mutant lacking such essential polypeptide. Such marker genes suitable for selection of transformed filamentous fungal cells are, for example, the known qa-2, pyrG, pyr4, trpC, amdS or argB genes.

As described in EP 278.355 a marker gene, named pyrA, was isolated from the genomic library of A. niger, which is related to and has similar function as pyr G of A. nidulans and pyr4 of N. crassa, namely producing the enzyme orotidine 5′-phosphate decarboxylase. This enzyme catalyses the decarboxylation of orotidine 5′-phosphate to uridylic acid (uridine 5′-phosphate) and also of fluoro-orotic acid to the toxic fluoro-uridine. An E. coli clone containing the pyrA gene was identified by hybridization with the 1.1 kb Hind III fragment of pDJB2 (ref. 25) containing part of the pyr4 gene. However, DNA of any other pyr gene coding for orotidine-5′-phosphate decarboxylase may be used. From a positive clone named E. coli B75183/pCG59D7, the plasmid pCG59D7, comprising the pyrA gene, was isolated and used for cotransformation of an A. niger pyrA$^-$ mutant. Such pyrA$^-$ mutant is defective in the orotidine 5′-phosphate decarboxylase gene and therefore is unable to produce the corresponding enzyme. Such mutant was prepared by treating conidiospores of A. niger N756 under mutating UV-irradiation and colonies surviving in the presence of fluoro-orotic acid and uridine are selected. Colonies surviving in the presence of fluoro-orotic acid and absence of uridine are eliminated. The remaining uridine-requiring mutants, according to their ability of being transformable, belong to two complementation groups pyr A and pyr B, represented by mutants An8 and An10, respectively. They are treated in the form of protoplasts thereof under transforming condition with the pyr A containing plasmid pCG59D7. Only the An8 colonies were found to be transformed and to contain the pyr A gene as evidenced by the hybridizing ability of digested DNA thereof with DNA of pUN 121.

## Preparation of polypeptides

The invention concerns further a method for the preparation of polypeptides, characterized in that a homologous or heterologous structural gene, for example such the meaning of which is given hereinbefore, inserted in an expression cassette of the invention is expressed in a suitable transformed host according to conventional methods. When required, the polypeptide is isolated in conventional manner. Depending on the construction of the expression cassette, the products are either produced or, if a signal sequence is present, are produced and secreted. Usually, the expression cassette is comprised in a hybrid vector, but may also become integrated into the host genome after transformation.

A suitable host is for example a fungus, e.g. a filamentous fungus, particularly an Aspergillus strain, if a promoter derived from a PG gene of the invention or another promoter derived from filamentous fungi is used for the expression of a homologous or heterologous structural gene. However, if other promoters are used, for example such derived from prokaryotes or higher eukaryotes, other hosts are suitable, for example bacteria such as E. coli, or higher eukaryotic cells, respectively.

The promoters of the PG genes of A. niger are inducible in the A. niger cell, i.e., the expression of the structural gene attached thereto, e.g. the structural gene coding for a PG or any foreign gene, is induced by addition of pectin or pectin degradation products to the medium. In the presence of sufficient glucose, however, the promoter is not inducable, if an A. niger strain, e.g. An8 or N593, is used as host. This means, genes under the control of an A. niger PG promoter are "catabolite repressed" in A. niger . However, if another Aspergillus strain is used, preferentially A. oryzae or most preferentially A. nidulans , a gene under the control of an A. niger PG promoter is expressed constitutively, i.e. also in the absence of pectin and/or in the presence of glucose. It can therefore be advantageous to express genes under the control of an A. niger PG promoter in an Aspergillus host other than A. niger , preferentially A. oryzae or most preferentially A. nidulans , because, for example, glucose instead of pectin can be added to the nutrient medium as energy and carbon source during the expression of a desired gene.

If a promoter not derived from a PG gene of the present invention is used for the con struction of an expression cassette of the invention, e.g. comprising a structural gene encoding a PG, other hosts can be used as expression hosts. The suitable hosts depend on the promoter used and are e.g. bacteria such as E. coli , or yeast, such as S. cerevisiae or Kluyveromyces lactis . Suitable hosts and promoters for the preparation of polypeptides according to the invention are also those suitable for transformation given hereinbefore.

It is now possible to overexpress one or more desired PGs, whereby various methods can be applied. A purified single PG can be prepared by subjecting a pectinolytic enzyme mixture containing a PG to conventional purification methods. Another method for the production of a single PG, preferentially PGI, PGII or the product of the pga C gene, is characterized in that a suitable host which is not capable of expressing any PG or which expresses PGs in low amount or which does not express PG under the induction conditions used for the introduced PG gene, is transformed with a hybrid vector comprising a structural gene coding for a PG, e.g. PGI, PGII or the pgaC gene product, or a fragment of a PG with PG activity, and that said structural gene is expressed. If a host not capable of expressing any PG is used, the respective single PG can be obtained in pure form, that means uncontaminated by any other PG. It is also possible to produce predetermined mixtures of PGs, optionally together with other enzymes, by expressing in a transformed host not only a single but more than one desired PG genes, optionally together with genes encoding other enzymes. Predetermined mixtures can also be obtained by expressing one or more desired genes for PG and/or other enzymes, e.g. for pectin esterases, pectin lyases, cellulases, mixed endo-glucanases, hemicellulases, xylanases, arabinases, galactanases, α-and β-glycodidases, and the like, in a host strain, optionally such with a certain background in enzyme production.

Predetermined mixtures of enzymes can also be obtained by disrupting particular PG genes in the host by "gene disruption", which is a technology well known in the art, using the isolated PG genes of the invention.

A host not capable of expressing any PG is either a microorganism having no corresponding gene e.g. a $PG^-$ Aspergillus strain, another $PG^-$ non-Aspergillus fungus or any other $PG^-$ eukaryotic or prokaryotic cell, or an Aspergillus strain, e.g. A. oryzae or A. nidulans , whose expression of endogeneous PG genes are suppressed in an appropriately conditioned growth medium, e.g. are "catabolite repressed" and/or uninduced, whereas the exogenous PG promoter operatively linked with the desired PG structural gene, e.g. an A. niger derived promoter, is active under these conditions or where the PG gene is fused to another promoter.

Other promoters and strains suitable for the preparation of PGs are the same as given hereinbefore in the description of the expression cassettes.

## The polypeptides and compositions

The single PGs purified from a pectinolytic enzyme mixture, preferentially from an Aspergillus niger derived pectinolytic enzyme mixture, in particular from Rapidase®, and PGs encoded by a DNA sequence according to the present invention and derivatives thereof with PG activity, in particular if produced in a suitable host transformed with an expression hybrid vector coding for such PG or a derivative thereof with PG activity, and physiologically acceptable salts thereof are also subjects of the present invention. In particular preferred are PGI, PGII, PGIIIA, PGIIIB and PGIV of Aspergillus niger , in purified form. The invention concerns said polypeptides whenever produced by a method according to the present invention.

The invention concerns further enzymatic compositions comprising one or more of such single PG and/or a derivative thereof with PG activity and/or biologically acceptable salts thereof optionally in a predetermined combination with one or more suitable enzymes having other than PG activity.

Enzymes having other than PG activity suitable for the preparation of said enzymatic compositions are degrading and modifying plant cell wall polymers. Such enzymes are e.g. pectin esterases, pectin lyases, cellulases, mixed endo-glucanases, hemicellulases, xylanases, arabinases, galactanases, $\alpha$- and $\beta$-glycodidases, and the like.

The present invention concerns further the preparation of said enzymatic compositions in conventional manner.

The use of said single PGs and/or of derivatives thereof with PG activity and/or of said enzymatic compositions in plant material processing are also a subject of the present invention.

Single PGs of the invention and/or derivatives thereof with PG activity or enzymatic compositions comprising such PG or derivative are useful e.g. for clarification of vegetable or fruit juice, for the enhancing of the juice yield in vegetable or fruit juice production and of pressing yield of oil-containing seeds or fruits, for stabilization of vegetable or fruit juice, for reduction of the viscosity of vegetable or fruit juice, for the liquefaction of biomass, for maceration, for the enhancement of natural product extraction like natural pigments, aromas and flavours, for the valorization of biomass, food or feed, to improve the recovery of cellulose fibers in paper pulping, and the like.

The most preferred embodiments of the invention are those described hereinafter in the Examples.

## Short Description of the Figures

Figure 1 : Partial restriction maps of the EcoRI fragments of phages λD and λE obtained from a genomic library of A. niger by hybridization with the combined DNA probes HR6195 and HR6196 coding for part of the PGII gene. The numbers indicate the approximate distances in kbp from the right hand EcoRI sites.

Figure 2 : Partial restriction map of pGW1800. The plasmid contains DNA of the vector pEMBL18 and the 4.1 Kbp XbaI-EcoRI insert obtained from phage λE which comprises the A. niger N400 PGII gene. Ap is the ampicillin resistance gene and the arrows indicate the A. niger derived DNA.

Figure 3 : Partial restriction map of pGW1900. It is composed of the pUC9 vector and a 8.6 kbp BamHI fragment of phage PGI-λ7.

Figure 4 : Cloning strategy for plasmids pGII-IFN AM119 and pGIIss-IFN AM119.

Figure 5 : Stepwise preparation of DNA inserts DNA 5 and DNA 6 for the construction of pGII-IFN AM119 and pGIIss-IFN AM119, respectively, with the polymerase chain reaction (PCR) method.

Figure 6 : Partial restriction map of pGW1756. pGW1756 is the 5.5 kbp XhoI-BglII fragment which carries the A. niger NW756 polygalacturonase II gene inserted in the vector pEMBL18. Restriction sites in the vector are not shown. The former XhoI and BglII sites of the fragment are also shown, but these have been destroyed by the insertion in the SalI and BamHI sites, respectively, of the vector.

Figure 7 : Partial restriction map of pGW1910. pGW1910 is the 7.8 kbp BglII fragment of lambda phage λPG-C20 inserted in the BamHI site of the vector pUC9. The approximate location of the A. niger N400 gene encoding PGC (pga C) gene is indicated.

The following examples serve to illustrate the invention, however, are in no way intended to restrict it.

The abbreviations have the following meanings:

Amp ampicillin
bis Tris bis (2-hydroxyethyl) imino-tris (hydroxymethyl) methane
BSA bovine serum albumin
DTT 1,4-dithiothreitol
EDTA ethylenediamine tetra acetic acid, disodium salt
IPTG isopropyl-β-D-thiogalactopyranoside
kbp kilo base pairs
PEG polyethylene glycol
PTH phenylthiohydantoin
SDS sodium dodecyl sulfate
Tet tetracycline
TFA trifluoro acetic acid
TP tryptic peptide
Tris tris (hydroxymethyl) aminomethane
X-gal 5-bromo-4-chloro-3 indolyl-β-galactoside

Buffers, media, reagents

SM 100 mM NaCl, 8.1 mM $MgSO_4$, 50 mM Tris-HCl pH 7.5,0.01 % gelatin
LB 1 % trypticase peptone (BBL), 0.5 % yeast extract (BBL), 1 % NaCl and 0.5 mM Tris-HCl pH 7.5
LM 1 % trypticase peptone (BBL), 0.5 % yeast extract (BBL), 10 mM NaCl and 10 mM $MgCl_2$
PBS 0.37 g $NaH_2PO_4$, 2.7 g $Na_2HPO_4$, 8.5 g NaCl per litre $H_2O$
SSC 0.15 M NaCl, 0.015 M tri-sodium citrate
PSB 10 mM Tris-HCl, pH 7.6, 100 mM NaCl, 10 mM $MgCl_2$, 0.05% (w/v) gelatine.
TE 10mM Tris-HCl pH 8.0, 0.1 mM EDTA pH 8.0
minimal medium 1 litre contains 1.5 g $KH_2PO_4$, 0.5 g KCl, 0.5 g $MgSO_4 \cdot 7H_2O$, 0.9 mg $ZnSO_4 \cdot 7H_2O$, 0.2 mg $MnCl_2 \cdot 4H_2O$, 0.06 mg $CoCl_2 \cdot 6H_2O$, 0.06 mg $CuSO_4 \cdot 5H_2O$, 0.29 mg $CaCl_2 \cdot 62H_2O$, 0.2 mg $FeSO_4 \cdot 7H_2O$, nitrogen and carbon sources as specified in the text or 6 g $NaNO_3$ and 10 g glucose per litre if these sources are not explicitly mentioned, adjusted to pH 6.0 with NaOH
complete medium minimal medium with 6 g $NaNO_3$ and 10g glucose per litre, plus per litre 2 g trypicase peptone (BBL), 1 g casaminoacids (Difco), 1 g yeast extract (BBL), 0.5 g ribonucleic acid sodium salt from yeast (ICN, Cleveland, USA), 2 ml vitamin solution, adjusted to pH 6.0 with NaOH
vitamin solution per 100 ml 10 mg thiamine, 100 mg riboflavin, 10 mg panthotenic acid, 2 mg biotin, 10 mg p-aminobenzoic acid, 100 mg nicotinamide, 50 mg pyridoxin-HCl
TBE 1 litre contains 4 ml of a 0.5 M EDTA pH 8.0 solution, 10.8 g Tris and 5.5 g $H_3BO_3$
phenol phenol treated as described by Maniatis et al . (p438; ref. 6)
sample buffer 10 % (v/v) glycerol, 100 mM EDTA pH 8.0 and 0.01 % bromophenol blue
RNase A RNase A treated as described by Maniatis et al . (p451; ref. 6) The following strains are used:
A. niger N400 wild type
A. niger N402 csp A
A. niger NW756 high pectinase producing strain
A. niger N756 high pectinase producing strain
A. niger An8 DSM 3917, uridine auxotrophic mutant of the pectinase complex highly producing strain A. niger N756
A.niger N593 csp A, pyr A
E. coli NM539 met B, sup E, hsd $M^+$, hsd $R^-$, sup F, (P2cox3)
E. coli LE392 $F^-$, hsd R514 ($rk^-$, $mk^+$), sup E44, sup F58, lac Y1, or lac 1ZY)6, gal K2, gal T22, metB1, trp R55, $\lambda^-$
E. coli DH5aF′ F′, end A1, hsd R17, ($r_k^-$, $m_k^+$), sup E44, thi -1, rec A1, gyr A, rel A1,)80ølac Z M15, Δ(lac ZYA-arg F)U169, $\lambda^-$
E. coli JM109 end A1, rec A1, gyr A96, thi , hsd R17 ($rk^-$, $mk^+$), rel A1, sup E44, $\lambda^-$, (lac-pro AB), [F′, tra D36, pro AB, lac IqZ M15]
E.coli JM110 rps L, thr , leu , thi , lac Y, gal T, ara , ton A, tsx , dam , dcm ,sup E44, (lac-pro AB), [F′, tra D36, pro AB, lac IqZ M15]

The following vectors are used

pGW613

This plasmid has been described by Goosen et al. (ref. 13).

M13mp phage

The M13mp 18 and M13mp19 vectors (Norrander et al ., ref. 24) are derivatives of the single-stranded DNA bacteriophage M13 and are designated to facilitate DNA sequencing by allowing cloning of DNA fragments at a versatile polylinker site and the cloning of the same restriction fragment in both possible orientations. Sequences cloned into these vectors can readily be used as templates for sequencing reactions or the production of single-stranded probes using standard oligodeoxyribonucleotide primer and the Klenow fragment of the E. coli DNA polymerase I. The vector DNA is carrying the E. coli lacoperon promoter and the genetic information of the first 145 amino acids of β-galactosidase. The polylinker sequences containing multiple restriction sites are inserted into the lacZ sequence. The polylinker retains the lacZ reading frame and the vector gives allelic complementation of a LacZa host strain, yielding blue plaques on plates containing IPTG and X-gal. Recombinant phages containing inserts that destroy the reading frame or otherwise interfere with expression of the lacZa peptide are revealed as colorless plaques.

pGW635

The plasmid is a shorter version of pGW613. It also contains the pyr A gene. It is described by Goosen et al. (ref. 42).

EMBL4

EMBL4 is a lambda replacement vector with a cloning capacity of 9-23 kbp (Frischauf et al ., ref. 9). It contains a multiple cloning region between the lambda arms and the non-essential stuffer region. This allows multiple restriction enzyme digestions to be performed in a manner such that religation of the stuffer to the vector arms is reduced as the foreign DNA of interest is inserted. The vector also makes use of the phenotype Spi to provide a direct selection for recombinants (Zissler et al ., ref. 21).

pEMBL18 and pEMBL19

These plasmids have been described by Dente et al. (refs. 10,22,23).

Example 1: Isolation and characterization of polygalacturonase

Example 1.1: Purification of polygalacturonases I, II, IIIA, IIIb, and IV

The enzyme activity is determined in the enzyme fractions obtained hereinafter as described before (Rozie et al ., ref. 2) using a modified ferricyanide test (Robyt et al ., ref. 3).

Polygalacturonases I, II, IIIA, IIIB, and IV are purified from Rapidase®, a commercially available mixture of pectinolytic enzymes obtained from Aspergillus niger (Gist-brocades, Séclin, France). 50 g of the crude enzyme powder (Lot-No. K2B 078) is dissolved in 190 ml of a 20 mM sodium acetate buffer pH 3.6, centrifuged for 10 min. at 25,000 g to remove solids and desalted on a Sephadex G-50 column (5 x 90 cm) equilibrated in the same buffer.

The first step in the purification procedure is an affinity chromatography step using crosslinked alginate which is prepared as described in Rombouts et al . (ref. 1). The cross-linked alginate which has a bed volume of 5.2 m/g, is also equilibrated with 20 mM sodium acetate buffer pH 3.6 (column dimensions 2.5 x 30 cm). The desalted enzyme is loaded onto this column and then washed by applying a 100 mM sodium acetate buffer pH 4.2 and pH 5.6, respectively (400 ml of each). Whereas PGI and PGII adsorb to the alginate matrix, PGs IIIA, IIIb, and IV do not.

Example 1.1.1: Purification of PGI and PGII

The adsorbed PGI and PGII proteins from the crosslinked alginate column are eluted by a linear sodium chloride gradient (0-1 M) in a 100 mM sodium acetate buffer at pH 5.6 which is a slight modification of the procedure used before (Rombouts et al. , ref. 1). PGI coelutes with PGII at about 0.5 M NaCl.

The enzyme fraction comprising PGI and PGII is dialyzed against 20 mM sodium acetate buffer pH 5.7 and applied to a DEAE-Sephadex A-50 column (2.5 x 30 cm) equilibrated in the same buffer. The enzymes are eluted using a linear NaCl gradient (0-0.6 M). PGI elutes at about 0.3 M NaCl, PGII at about 0.2 M. The enzyme fractions containing PGI and PGII are collected separately, dialyzed against 20 mM bis Tris HCl buffer pH 5.8 and chromatographed on a DEAE-Sepharose Fast Flow colum equilibrated in the same buffer. Upon applying a NaCl gradient, PGII is eluted at approximately 120 mM sodium chloride, PGI at about 250 mM. Active enzyme fractions containing PGI or PGII are concentrated after dialysis against 20 mM sodium acetate buffer pH 5.7 to a final volume of 25 ml on a small DEAE-Sepharose Fast Flow column (1.6 x 10 cm) by eluting with 1 M sodium chloride in this buffer. Final purification of each of the two enzymes PGI and PGII is achieved by gel permeation chromatography on Sephacryl S200 (1.6 x 90 cm) in 0.1 M sodium acetate pH 4.2.

PGII shows a single band upon SDS-polyacrylamide gel electrophoresis and has an apparent molecular mass of 38 kDa. The specific activity is 2760 U/mg of protein determined in 0.075 M sodium acetate buffer pH 4.2. Upon iso-electric focusing the enzyme shows microheterogeneity. Besides a pronounced major component at approximately pH 5.2, a large number of minor bands is observed in the pH range between 4.6 and 5.9

PGI also shows a single band upon SDS-polyacrylamide gel electrophoresis. This enzyme has an apparent molecular mass of 55 kDa. The specific activity is 550 U/mg of protein determined in 0.075 M sodium acetate buffer pH 4.2. Upon iso-electric focusing the enzyme also shows micro-heterogeneity. Several bands are observed in the pH-range of pH3.2-3.5.

Example 1.1.2.: Purification of PGIIIA, PGIIIB and IV

The effluent of the crosslinked alginate column (Example 1.1) containing the unbound PGs is adjusted to pH 5.7 with 1 M sodium hydroxide and is loaded on a DEAE-Sephadex A-50 column (2.5 x 30 cm), equilibrated in 0.02 M sodium acetate buffer pH 5.7. The enzymes are eluted from the DEAE-Sephadex column with a linear sodium chloride gradient (0-1 M). PGIV elutes at 0.38 M sodium chloride, PGIIIA and PGIIIB coelute at 0.5M.

5 ml of each of the two enzyme fractions are desalted on a Sephacryl S-200 column (1.6 x 90 cm) in 0.1 M sodium acetate buffer pH 4.2. The active fractions are pooled, diluted five fold with distilled water and loaded on a MONO Q column (Pharmacia Fine Chemicals, Uppsala, Sweden) equilibrated in 0.02 M bis-Tris/HCl buffer pH 5.8. Upon applying a 20 ml linear sodium chloride gradient (0.1-0.4 M), PGIV elutes at 0.32 M sodium chloride, whereas PGs IIIA and IIIB coelute at 0.4 M sodium chloride.

Both enzyme fractions are separately re-chromatographed on a MONO Q column under the conditions described above. Fractions containing PG activity are pooled, dialyzed against 0.025 M piperazine/HCl buffer pH 6.0 and loaded on a MONO P column (Pharmacia Fine Chemicals, Uppsala, Sweden) equilibrated in the same buffer. The column is eluted with 10 % (v/v) poly buffer 74 (Pharmacia Fine Chemicals, Uppsala, Sweden) pH 3.0 at a flow rate of 0.75 ml/min.

Final purification is achieved by gel permeation chromatography on a TSK G 3000 SW column (0.75 x 30 cm) (LKB, Bromna, Sweden) equilibrated in 0.05 M sodium phosphate buffer pH 6.2 at a flow rate of 0.5 ml/min.

The column is calibrated with the following protein standards: bovine serum albumin (68 kDa); egg albumin (45 kDa) and chymotrypsinogen A (25 kDa).

An apparent molecular mass of 53 kDa is determined for PGIV by gel permeation chromatography. PGIV shows a single band upon SDS-polyacrylamide gel electrophoresis and has an apparent molecular mass of 59 kDa determined by electrophoresis on a 15 % polyacrylamide gel. The specific activity is 780 U/mg of protein determined in 0.075 M sodium acetate buffer pH 4.2. Upon iso-electric focusing the enzyme shows a single sharp band at pH 3.7.

Gel permeation chromatography of the active fractions containing PGIIIA and IIIB on the calibrated TSK G 3000 SW column, under the same conditions as described for PGIV, results in separation of the two enzymes with apparent molecular masses of 32 and 52 kDa respectively. Upon SDS-polyacrylamide gel electrophoresis (15 % polyacrylamide gel), however, both enzymes show a single band with an identical molecular mass of 57 kDa. PGIIIA and IIIB have a specific activity of 150 and 250 U/mg protein, respectively, determined in 0.075 M sodium acetate buffer pH 4.2. Upon iso-electric focusing both enzymes show a single sharp band at a pH-value of 3.3.

Example 1.2.: A comparison of the properties of the polygalacturonases and their immunological relationship

Starting with 50 g Rapidase (Gist-brocades, Séclin, France) five endopolygalacturonases are purified as described in Example 1.1. The properties of these purified enzymes are summarized in Table II.

Table II.

| Physico-chemical properties and pH-optima of five *A. niger* polygalacturonases. | | | |
|---|---|---|---|
| Enzyme | Mr[a] | I.E.P.[b] | pH-optimum (activity) |
| PGI | 55K | 3.2-3.5 | 4.9 |
| PGII | 38K | 4.6-5.9 | 4.8 |
| PGIIIA | 57K | 3.3 | 4.3 |
| PGIIIB | 57K | 3.3 | 4.5 |
| PGIV | 59K | 3.7 | 4.8 |
| Notes: | | | |

[a] Extrapolated value determined by SDS-gelelectrophoresis.
[b] Isoelectric point, determined by thin-layer isoelectric focusing.

All purified enzymes have an apparent molecular mass on a 15 % SDS-polyacrylamide gel in the range of 55-59 kDa, except PGII which appears to be a much smaller protein (38 kDa). The special nature of PGII is emphasized by its relatively high iso-electric point in contrast to the low iso-electric point values determined for the other polygalacturonases. The pH optimum of all polygalacturonases is in the same range (4.3-4.9).

Specific antibodies against purified PGI and PGII are raised in male New Zealand white rabbits according to the immunization scheme described by Uitzetter (ref. 36).

Crossreactivity between the two antisera and the five purified polygalacturonases is monitored as outlined below. 0.4-1.0 µg purified protein is loaded on a 10 % SDS-polyacrylamide gel and after electrophoresis transferred from the gel to a nitrocellulose membrane using the method described by Bowen *et al* . (ref. 37). Incubation of the nitrocellulose blot with the specific antisera followed by staining with peroxidase labelled goat anti-rabbit IgG is done according to Uitzetter (ref. 36). Upon incubation with the PGI specific antiserum a strong signal is found for PGI and the PGs IIIA, IIIB and IV. PGII gives a slightly weaker signal with this antibody. Incubation of the blot with PGII specific antiserum results in a high intensity band for PGII but only weak bands for the other four polygalacturonases.

Example 1.3: Amino acid sequence determination of cyanogen bromide fragments of polygalacturonase II

Approximately 100 µg of PGII is dissolved in 150 µl 70 % formic acid and solid cyanogen bromide is added in a hundred fold molar excess to methionine (of which 4 residues occur per enzyme molecule). The reaction mixture is stirred continuously for 24 h at room temperature in a closed reaction vial. After 24 h water is added and the preparation is dried by flushing with $N_2$. This step is repeated to remove formic acid.

After electrophoresis on 15 % SDS-polyacrylamide gels approximately 10 individual bands can be stained by Coomassie Brilliant Blue. They represent incompletely and completely cleaved fragments. Besides the intact protein which has a molecular mass of 38 kDa, the following bands are observed: 33, 30, 27, 19, 17, 12, 10, 7.5, 6 and 6 kDa. By taking samples at regular intervals during the 24 h reaction period and analyzing these by SDS-polyacrylamide gel electrophoresis, the order in which partial and final reaction products appear as well as their position with respect to each other has been determined. The 17 kDa fragment is an internal fragment whereas the 5 kDa fragment is located either at the C-terminus or N-terminus. The fragments of the 24 h treatment are then blotted onto Immobilon-P, which is a polyvinylidene difluoride membrane (Millipore), according to the procedure described by Matsudaira et al. (ref. 4). Three fragments are used for gas-phase sequencing viz a 17 kDa internal fragment and two smaller fragments (5 and 6 kDa).

Amino acid sequences are determined with an Applied Biosystems model 470 A protein sequencer, connected on-line with an Applied Biosystems 120 A PTH analyzer. Membrane fragments containing 0.5-1 nmole of a particular peptide are washed, loaded onto the gas-phase sequencer and subjected to sequence analysis according to the programme described by Amons (ref. 5).

For the 5 kDa fragment the following sequence is obtained:

```
Position:    1                       5
Amino acid: asp - ser - X - thr - phe - thr - thr - ala - ala -

Position:    10          12             13
Amino acid: ala - ala - lys (ala) - (ala)
```

At position 3 (X) no amino acid is detected. Since the sequencing programme used only detects cysteine residues if the protein is S-pyridylethylated before, it is likely that position 3 may be a cysteine (Amons, ref. 5). In position 12 and 13 traces of ala are found as is indicated by brackets. While the trace in position 12 clearly results from an incomplete reaction in the previous step the low signal of ala in position 13 represents the amino acid ala in position 13 of the peptide.

The sequence for the 17 kDa fragment is

```
position:    1                           5
amino acid: ala - phe - ser - val - gln - ala - asn - asp -

position:          10
amino acid: ile - thr (ile) - phe (thr)
```

The traces of isoleucine and threonine observed in the last two steps as is indicated by the brackets clearly result from incomplete reactions in the previous steps.

Example 1.4.: Amino acid sequence determination of the N-terminal part of polygalacturonase I

100 µg of PGI purified according to Example 1.1.1 is dialyzed three times against 1l Millipore filtered water and lyophilized. The amino acid sequence is determined as described in Example 1.3. The following N-terminal amino acid sequence has been determined for the enzyme

```
Position  :  1                       5                       9
amino acid: ala - ser - thr - X - thr - phe - thr - ser - ala
```

Thy cysteine residues in the protein have not been modified and are thus not detected. Cysteine is likely to occur at position 4 (X) of the sequence. The N-terminal amino acid sequence of PGI shows homology with the amino acid sequence of the 5 kDa cyanogen bromide fragment of PGII which has been isolated from Rapidase (Example 1.3) and with the N-terminal amino acid sequence of PGII isolated from the A. niger transformant N593/pGW 1800-27 (Example 6.3). This homology is outlined in the following scheme, in which it is assumed that cysteine occurs at position 4 and 3 of the PGI and PGII sequences, respectively:

| | | | | | | | | | |
|---|---|---|---|---|---|---|---|---|---|
| PGI | ala - | ser - | thr - | cys - | thr - | phe - | thr - | ser - | ala |
| PGII | asp - | ser - | | cys - | thr - | phe - | thr - | thr - | ala |

The open space between the serine residue at position 2 and the assumed cysteine residue at position 3 in the polygalacturonase II sequence is introduced in order to align both sequences.

The serine residue at position 8 in the PGI sequence is not present at the corresponding position in the PGII sequence (position 7), but in the latter case a similar type amino acid, i.e. a small hydroxyl amino acid (threonine), is present.

The N-terminal amino acid sequences of PGI and PGII thus show homology, but are not identical. The differences are such that these cannot be the result of partial modification of a product of a single gene, such as for instance partial proteolytic cleavage. It is concluded that PGI and PGII are encoded by different genes of the same family.

Example 1.5: Amino acid sequence determination of cyanogen bromide fragments of polygalacturonase I.

Approximately 100 µg of PG I is dissolved in 150 µl 70% formic acid and solid cyanogen bromide is added to prepare fragments of the protein as described in Example 1.3. 4 methionine residues are present per enzyme molecule. After electrophoresis on 15% SDS-polyacrylamide gels and staining with Coomassie Brilliant Blue, major bands are observed having a molecular mass of 39.5, 35, 21, 19.5 and 5.5 kDa.

The 21 and 5.5 kDa fragments are sequenced using the same equipment and procedures as described in Example 1.3.

The sequence for the 21 kDa fragment is

| | | | | | | |
|---|---|---|---|---|---|---|
| Position | 1 | | | | | |
| Amino acid | ala - | ser - | thr - | X - | thr - | phe - |
| Position | | | | 10 | | |
| Amino acid | thr - | ser - | ala - | ser - | glu | |

Cysteine is likely to occur at position 4 (X) of the sequence similar to what has been described in Examples 1.3 and 1.4. The sequence of the 21 kDa fragment is identical with the N-terminal amino acid sequence of the intact PG I protein described in Example 1.4.

The 5.5 kDa fragment has the following sequence

| | | | | | | |
|---|---|---|---|---|---|---|
| Position | 1 | | | | 5 | |
| Amino acid | ala - | asp - | gly - | ala - | val - | ile |
| Position | | | | 10 | | |
| Amino acid | asp - | gly - | asp - | gly - | ser | |

It is concluded from these sequence data that the 5.5 kDa fragment is not corresponding with the N-terminus of the protein.

Example 1.6: Amino acid sequence determination of a tryptic peptide

PGII is partially denatured by dialysis against 0.5 % (v/v) formic acid and subsequently freeze-dried. The enzyme (1 mg) is suspended in 1 ml of a 0.2 M N-ethyl morpholino-acetate buffer pH 8.0 and then incubated at 37° C with trypsin (Sigma) which is added in a molar ratio of 1:50 with respect to PGII. After 24 h the digestion is stopped by the addition of acetic acid (30 % final concentration). The digest is freeze-dried. Samples of the digest are dissolved in 0.1 % trifluoroacetic acid containing 2.5 mM dithiothreitol and kept at 37° C for at least 1 h. Tryptic peptides (100 µl samples containing 100-200 µg of protein) are separated using a Spectra Physics SP 8000 HPLC equipped with a Nucleosil C-18 column (4.6 x 150 mm) (Supelco) and a Vydac guard column (Chrompack). A a column temperature of 25° C, a flow rate of 2 ml/min and a linear gradient is applied which changes in 50 min from 100% solvent A (0.1 % TFA in water) to 50% solvent A + 50 % solvent B (0.1 TFA in acetonitril). Peptides are detected by UV absorption at 214 nm. One of the peptides which is released late during digestion appears well separated from other peaks in the chromatogram at approx. 21 % acetonitril. This peptide is dried by flushing with dry air at 25° C and sequenced.

The amino acid sequence is determined according to the procedure described in Example 1.4. The sequence is:

```
Position:     1                   5
Amino acid: thr - ile - ser - gly - ala - thr - gly - ser -

Position:            10                  14
Amino acid: val - ser - glu - ile - thr - tyr
```

Example 2: Construction of a genomic library of Aspergillus niger

Example 2.1: Isolation of high molecular weight DNA from A. niger N400

Conidiospores of Aspergillus niger strain N400 are inoculated in 200 ml minimal medium in a final spore density of $10^5$ spores/ml and shaken in 1 l Erlenmeyers for 24 h at 28° C at 300 rpm using a New Brunswick rotary shaker. The mycelium is harvested by filtration using a Büchner funnel with Myracloth, washed with cold sterile saline, frozen in liquid nitrogen and either stored at -60° C or used directly. The method used for isolation of DNA to prepare the genomic library is based on the procedure described by Yelton et al. (ref. 18).

For library construction, 10 g mycelium is ground in liquid nitrogen in 1 g portions in a Braun microdismembrator. The ground mycelium is transferred to a 1 l sterile erlenmeyer, containing 200 ml extraction buffer (50 mM EDTA pH 8.5, 0.2 % SDS) and 200 µl diethylpyrocarbonate. The mixture is slowly warmed up to room temperature and then heated for 20 min to 68° C with occasional shaking. The suspension is cooled to room temperature and centrifuged for 15 min at 12,000 x g. 1/16 volume of an 8 M potassium acetate solution pH 4.2 is added to the supernatant and the mixture is left on ice for 1 h. The precipitate is removed by centrifugation (20 min.; 16,000 x g; 4° C). The nucleic acids are precipitated from the supernatant by an incubation with 0.6 volume of isopropanol on ice for 15 min. The precipitated nucleic acid is collected by centrifugation (10 min.; 6,000 x g; 4° C), washed with 70 % ethanol and briefly dried. The pellet is suspended in 10 ml TE containing 20 µg/ml Rnase A, (Boehringer, Mannheim) and incubated for 15 min at 37° C. The DNA is treated with nuclease free pronase (1 mg/ml final concentration) (Kochlight, Coinbrook) for 1 h at 37° C. The pronase stock solution in TE buffer contains 20 mg/ml of enzyme which is pre-incubated for 1 h at 37° C to digest nucleases.

8.5 g CsCl is dissolved in 9 ml of the DNA solution obtained, 0.2 ml 10 mg/ml ethidium-bromide is added and this solution is either centrifuged in a Beckman SW41 rotor for 60 h at 33,000 rpm, or in a Beckman 50 Ti rotor for 40 h at 45,000 rpm. The DNA band is collected and the ethidiumbromide is removed by multiple extraction with isopropanol equilibrated with a satured solution of NaCl in water. 5 volumes of TE are added and the DNA solution is sequentially treated with TE saturated phenol, phenol/chloroform/isoamylalcohol 25:24:1 and chloroform/isoamylalcohol 24:1. The DNA is precipitated by addition of 0.1 volume of 3 M sodium acetate pH 5.2, 2.5 volumes of ethanol and an overnight incubation at -20 °C. The precipitate is collected by centrifugation (1 h, 30,000 x·g; 4 °C), washed with 70 % ethanol, dried and dissolved in 400 µl TE.

Example 2.2: Partial digestion of A. niger N400 DNA with MboI and isolation of fragments

To test for the MboI concentration which gives the largest amount of DNA fragments between 13.6 and 23 kbp, 1 µg portions of A. niger N400 DNA are digested in the appropriate buffer recommended by the supplier with decreasing amounts of MboI (0.5-0.001 U) for 1 h at 37 °C in a volume of 10 µl. The reaction is stopped by addition of 1 µl 0.25 M EDTA, and the samples are loaded on a 0.6 % agarose gel in TBE buffer, containing 1 µg/ml ethidiumbromide. Convenient size markers are a mixture of lambda DNA and lambda DNA digested with BglII, which gives bands of 49, 22.8, 13.6, 9.8, 2.3, kbp and a nonvisible fragment of 0.45 kbp. The MboI concentration required to give a high yield of the desired 13.6-23 kbp fragments is about 0.02 U/µg DNA. Accordingly, 200 µg of DNA in a total volume of 2 ml are digested, and are divided into 20 equal aliquots immediately after addition of the enzyme. After 1 h at 37 °C the digests are placed on ice. After checking a 1 µl sample for proper digestion by running on a gel, EDTA is added to a final concentration of 25 mM, the enzyme is heat-inactivated at 65 °C for 10 min, samples are pooled and the DNA is precipitated, washed, dried and dissolved in 400 µl TE.

The fragmented DNA is separated on a 0.4 % preparative agarose gel (center well 120 x 1.5 mm). Lambda DNA digested with BglII is used as marker to determine the size of the partially digested DNA fragments upon electrophoresis at 4 °C and 40 V (3 V/cm). The gel region containing fragments of the correct size is cut out of the gel and the DNA is electroeluted from the gel in a sterile dialysis tube in 2 ml TBE for 2-3 h at 100 V. The current is reversed for 30 s, and the buffer containing the DNA is collected. The fragments are then concentrated by ethanol precipitation and dissolved in 100 µl TE.

Example 2.3: Preparation of vector DNA and cloning of high molecular weight DNA fragments of A. niger N400 into EMBL4

The genomic library of A. niger strain N400 is constructed in the lambda vector EMBL4. The vector, which has a cloning capacity of 9-23 kbp, is described by Frischauf et al. (ref. 9) and Karn et al. (ref. 19) and has been purchased from Promega Biotech. Inc. To avoid double inserts originating from different parts of the genome, a minimal fragment length of 13.6 kbp is used for cloning.

10 µg lambda EMBL4 DNA is digested to completion with 50 units of BamHI in the buffer recommended by the supplier in a volume of 100 µl for 2 h at 37 °C. The enzyme is inactivated for 10 min at 65 °C. The NaCl concentration is raised to 150 mM and 50 units of SalI are added and incubation at 37 °C continues for another 2 h. After addition of EDTA to 25 mM and inactivation of the enzyme (heating for 10 min at 65 °C) the solution is extracted with equal volumes of phenol (TE saturated), phenol/chloroform/isoamylalcohol 25:24:1, and chloroform/isoamylalcohol (24:1). To eliminate the small BamHI/SalI polylinker fragments, the DNA is precipitated with 0.6 volume of isopropanol after the addition of 0.1 vol. 3 M sodium acetate pH 5.2. After 15 min on ice and 15 min centrifugation at 12,000 x g at 4 °C, the precipitate is thoroughly washed with 70 % ethanol, dried and dissolved in 40 µl TE.

Example 2.4: Ligation and in vitro packaging of genomic A. niger N400 DNA fragments

It is essential that the cos sites of the vector prepared according to example 2.3 are annealed prior to the ligation reaction. The vector in 100 mM Tris-HCl pH 7.5 and 10 mM $MgCl_2$ is heated for 10 min at 65 °C and then annealed for 1 h at 42 °C. From test ligations a ratio of vector to fragments of approximately 1:1 (by weight) is found to give most recombinants. Ligation took place in 50 mM Tris HCl pH 7.5, 10 mM $MgCl_2$, 10 mM DTT and 1 mM ATP, using 9.5 µg of vector and 10 µg of DNA fragments in a total volume of 100 µl. DNA ligase (BRL) is added at a concentration of 0.5 U/µg DNA and the ligation mixture is incubated overnight at 14 °C. To test for ligation a sample of the ligated DNA is run on an agarose gel. Also, as a control 0.5 µg of vector is ligated without the addition of fragments in a 5 µl volume.

The large volume ligation mixture is concentrated by ethanol precipitation and dissolved in 20 µl TE prior to in vitro packaging. In vitro packaging is done with Promega Packagene extracts according to the instructions of the manufacturer using 10 µl portions to package 1 µg of DNA. 1 µg of the high molecular weight control phage lambda cI857 Sam 7, supplied with the extracts, is separately packaged as a control. After packaging, 500 µl of phage solution buffer (PSB) and 5 µl of chloroform are added. The recombinant phage stocks can be stored at 4° C. The library obtained is constructed from two separate ligation experiments.

Example 2.5: Titration and amplification of the A. niger strain N400 genomic library

Cells of E. coli NM539 are grown on LB medium containing 0.2 % maltose, 10 mM $MgSO_4$ and 1 mM $CaCl_2$ to an optical density (600 nm) of 1.0. 0.2 ml aliquots of this culture are added to 0.1 ml of an appropriate phage dilution in PSB. After adsorption of the phages for 20 min at 37° C, 3 ml 0.6 % LB top-agar having a temperature of 45° C is added, the mixture is plated on LB agar plates and these are incubated overnight at 37° C. The number of plaque forming units (pfu) per ml phage suspension are $12x10^5$ and $4.2x10^5$ pfu/ml for the two phage stocks prepared according to example 1.4. After subtracting the background which is calculated from the control ligations without fragments (17 % and 40 % respectively) the absolute number of recombinants is $6x10^5$. The DNA contained in the recombinants is equivalent to more than 200 of the Aspergillus niger genomes.

To amplify the library, 80 µl aliquots of both phage stocks are used to infect E. coli NM539 cells which are plated in LB top-agarose on LB agar plates and then incubated overnight at 37° C. The phages are eluted from the agarose by gently shaking the plates with 5 ml PSB per plate for 1 h at room temperature. The PSB is collected, centrifuged (10 min at 6000 xg) to remove bacteria and chloroform is added (0.5 % final concentration). Both phage stocks, which are amplified approximately to the same extent, are then mixed (40 ml stock), titrated ($8x10^9$ pfu/ml) and stored at 4° C.

Example 3: Screening the genomic library of A. niger for nucleic acids related to polygalacturonases

Example 3.1: Synthesis of oligonucleotide mixtures coding for the 17 kDa cyanogen bromide fragment of polygalacturonase II

The oligonucleotides for screening the genomic library of Aspergillus niger prepared according to Example 2, are synthesized by using the phosphoramidite method (M.H. Caruthers, ref. 8) with an Applied Biosystem (model 380 B) oligonucleotide synthesizer. The 17 kDa cyanogen bromide fragment described in Example 1.2 is an internally located peptide. Therefore the amino acid sequence determined in Example 1.4 can be extended at the N-terminus with a methionine residue. The oligonucleotides synthesized are complex mixtures as the degeneracy of the genetic code is taken into account. For technical reasons it is necessary to reduce the number of nucleotides in the mixture. To exclude those oligonucleotides with the wrong combination TG and AC at position 10 and 11 and to limit the size of the mixture, two separate mixtures of oligonucleotides corresponding to the sequence in the coding strand are synthesized taking into account the degeneracy of the genetic code. The number of oligonucleotides required is further reduced by introducing inosine as the wobble base at four different positions of the synthesized 29-mers and thus each mixture consists of 16 different oligonucleotides (Ohtsuka et al. , ref. 16). The two mixtures which are named HR6195 and HR6196 represent DNA encoding the following amino acid sequence and have the following composition:

| | | | | | | | | |
|---|---|---|---|---|---|---|---|---|
| amino acid sequence: | | met | ala | phe | ser | val | gln | ala |
| HR6195: | 5' (d) | ATG | GCI | $TTR_1$ | TCI | GTI | $CAR_2$ | GCI |
| HR6196: | 5' (d) | ATG | GCI | $TTR_1$ | AGI | GTI | $CAR_2$ | GCI |

| | | | |
|---|---|---|---|
| amino acid sequence: | asn | asp | ile |
| HR6195: | $AAR_1$ | $GAR_1$ | AT 3' |
| HR6196: | $AAR_1$ | $GAR_1$ | AT 3' |

wherein I is inosine, $R_1$ is T or C, $R_2$ is A or G.

Example 3.2: Synthesis of an oligonucleotide mixture coding for the 5.5 kDa cyanogen bromide fragment of polygalacturonase I.

The 5.5 kDa cyanogen bromide fragment described in Example 1.5 is an internally located peptide which is prepared by CNBr cleavage. Therefore the amino acid sequence thereof can be extended at the N-terminus with a methionine residue. The number of oligonucleotides required is reduced by introducing inosine as the wobble base at five different positions of the 32-mers which are synthesized.
This limits the mixture to 24 different oligonucleotides. The mixture, named HR 6298, represents DNA encoding the following amino acid sequence and has the following composition:

| | | | |
|---|---|---|---|
| Amino acid sequence: | met | ala | asp |
| HR6298 | 5' (d)ATG | GCI | $GAR_1$ |

| | | | | | | | |
|---|---|---|---|---|---|---|---|
| gly | ala | val | ile | asp | gly | asp | gly |
| GGI | GCI | GTI | $ATR_2$ | $GAR_1$ | GGI | $GAR_1$ | GG 3' |

wherein I is inosine, $R_1$ is T or C and $R_2$ is T, C or A.

Example 3.3: $^{32}$P-labeling of oligonucleotides

The lyophilized oligonucleotide mixtures HR6195, HR6196 and HR6298 are dissolved in distilled water at concentrations of 29 µM. Samples of these solutions are diluted tenfold to make the reaction mixture. The reaction mixture is composed of either 20 pmoles oligonucleotides mixture HR6195 together with 20 pmoles HR6196 or of 40 pmoles HR6298 alone, 34 pmoles of [$\gamma$-$^{32}$P]-ATP (NEN, 6000 Ci/mmol), and 30 units T4 polynucleotide kinase (BRL) in 50 µl kinase buffer, which is 50 mM Tris-HCl (pH 7.6), 10 mM $MgCl_2$, 5 mM DTT, 0.1 mM EDTA and 0.1 mM spermidine (Maniatis, et al. , ref. 6, page 122). The incubation is carried out at 37° C for 30 min. The reaction is stopped by the addition of 4 µl of 0.5 M EDTA (pH 8.0). The reaction mixtures are used without purification for screening the genomic library (Example 3.4) or probing Southern blots (Example 3.6).

Example 3.4: Screening of the A. niger N400 library

Part of the genomic library of Aspergillus niger strain N400 described above (Example 2) is diluted in SM and 0.1 ml portions each containing about 2000 pfu are plated. Host cells are prepared by inoculating 50 ml of LB-medium supplemented with 0.2 % maltose with 0.5 ml of an overnight culture of E. coli NM539 in LB-medium, shaking for 4 h at 250 rpm on a Gallenkamp orbital shaker at 37° C, followed by the addition of 0.5 ml 1 M $MgSO_4$ and of 0.5 ml 0.5 M $CaCl_2$. 0.2 ml aliquots of these cells are each mixed with a 0.1 ml portion of the phage suspension and these mixtures are incubated at room temperature for half an hour. Then 3 ml of 0.7 % agarose in LM-medium at 47° C are added, briefly vortexed and immediately plated on LM agar plates. The plates are incubated overnight at 37° C and chilled for 2 h at 4° C.

From each plate two replicas are made according to the Benton and Davis plaque hybridization method (ref. 7). The first filter (Schleicher and Schuell BA85) is placed on top of the plate for 1 min, the second replica for 2 min and the position of the replicas is marked using India ink. After removing the filters they are placed in a dish containing 100 ml of a denaturing solution (1 M NaCl, 0.5 M NaOH) for 0.5 min, and then for 1 min in 100 ml neutralizing solution (0.5 M Tris-HCl pH 7.5, 1.5 M NaCl). The filters are transferred to a dish containing 3xSSC, are gently rubbed with a gloved hand to remove bacterial debris and are rinsed with 3xSSC. The filters are blotted, dried for 10 min at room temperature and baked on Whatman 3 MM paper in an oven at 80° C for 2 h.

The baked filters are wetted in 3xSSC, washed in this solution for 1 h at room temperature and then transferred to a dish containing 250 ml prewarmed (65° C) prehybridization mixture which consists of 2xSSC if oligonucleotide mixture HR6195 and HR6196 is used and of 1xSSC if oligonucleotide mixture HR6298 is used, further of 10x Denhardt's (0.2 % BSA, Boehringer fraction V; 0.2 Ficoll 400, Pharmacia; 0.2 % polyvinylpyrrolidone-10, Sigma), 0.1 % SDS and 0.1 mg/ml sheared and freshly denatured herring sperm DNA. Prehybridization is performed for 5 h at 65° C in a shaking water bath. Next the filters are washed once for half an hour in 250 ml prewarmed (65° C) hybridization mixture, which is identical to the prehybridization mixture, except for the omission of herring sperm DNA. Then the filters are placed in a different dish containing 150 ml of prewarmed (65° C) hybridization mixture to which the previously labeled combined oligonucleotide mixtures HR6195 and HR6196 (Example 3.1) or the labelled mixture HR6298 had been freshly added.

In the case if HR6195 and HR6196 are used, the dish is placed in the shaking water bath at 65° C, the thermostate is adjusted to 47° C and the hybridization is allowed to proceed for 14 h. The filters are washed once in 250 ml prewarmed (47° C) hybridization mixture for half an hour at 47° C, followed by washing at room temperature in two changes of 250 ml 2xSSC, each for 45 min. A stringent wash is performed by transferring the filters to a dish containing 250 ml of prewarmed (63° C) 6xSSC, 0.05 sodium pyrophosphate, and next incubating for 30 min in a shaking water bath at 63° C. Then the filters are washed at room temperature in two changes of 2xSSC, for 30 min each. The filters are dried in the air, fixed on a support of Whatman 3MM paper, covered with plastic wrap and exposed to

Kodak XAR5 film for three days at -60° C, using an intensifying screen.

In this way, 6 positive signals are obtained from the six plates screened. Positive plaques are punched out with a sterile Pasteur pipette by carefully positioning the plates on the autoradiogram using the ink markers. The pieces of agar containing the positive plaques are added to 1 ml of SM and 2.5. µl of chloroform is added. The phages are allowed to diffuse from the agar at room temperature for 1 h with occasional vortexing and then incubated overnight at 4° C. The agar and bacterial cell debris are removed by centrifugation for 5 min, 2.5 µl of chloroform is added and the phage stocks are stored at 4° C.

The positive clones are named λC to λG and λI. Since phages are plated at high density, the positive plaques are purified twice by plating them at a low density and repeating the complete procedure of replica plating, hybridization and picking of positive plaques.

In the case if the oligonucleotide mixture HR6298 is used, the dish is placed into the shaking water bath at 65° C, the thermostat is adjusted to 52° C and the hybridization is allowed to proceed for 14 h. Then the filters are washed once in 250 ml prewarmed (52° C) hybridization mixture for half an hour at 52° C followed by washing at room temperature in two changes of 250 ml 2 x SSC each for 45 min. Subsequently the stringent wash is performed by transfering the filters to a dish containing 250 ml of prewarmed (64° C) 4 x SSC, 0,05% (w/v) sodium pyrophosphate and next incubating it for 0.5 h in a shaking water bath at 64° C. Then the filters are washed at room temperature in two changes of 2xSSC, for 30 min each. The filters are dried in the air, fixed on a support of Whatman 3MM paper, covered with plastic wrap and exposed to Kodak XAR5 film for three days at -60° C, using an intensifying screen.

In this way nine positive signals are obtained from the six plates screened. Positive plaques are punched out with a sterile Pasteur pipette by carefully positioning the plates on the autoradiogram using the ink markers. The pieces of agar containing the positive plaques are added to 1 ml of SM and 2.5. µl of chloroform is added. The phages are allowed to diffuse from the agar at room temperature for 1 h with occasional vortexing and then incubated overnight at 4° C. The agar and bacterial cell debris are removed by centrifugation for 5 min, 2.5 µl of chloroform is added and the phage stocks are stored at 4° C.

The positive clones are named PG I-λ 1-9. The positive plaques are further purified by plating them at a low density using the 1.2 kbp Bam HI/Eco Ri fragment of pGW1803 (see Example 3.8) as a heterologous probe for hybridization which is carried out at 60° C, whereas washing is performed in 2 x SSC, also at 60°C.

Example 3.5: Isolation of lambda DNA

To isolate DNA from the recombinant clones, phages are first amplified. For this purpose E. coli LE392 host cells are grown to an optical density (600 nm) of 1.0 in LB-medium supplemented with 10 mM $MgSO_4$ and 0.2 % maltose. Then 50 µl of the stocks of the purified phages are separately plated as described in Example 2.5. After an overnight incubation at 37°C the phages are eluted from the nonconfluent plates by spreading 5 ml of SM over the plates and incubating for two hours with gentle shaking. The eluted phages are harvested and 0.1 ml chloroform is added. The mixture is briefly vortexed and cellular debris is removed by centrifugation. The supernatants are recovered, chloroform is added to 0.3 % and the resulting plate lysate is stored at 4°C.

In order to obtain nearly confluent plates as starting material for the isolation of phage DNA, 10 µl portions of the plate lysates are plated with E. coli LE392 host cells. After overnight incubation at 37°C the agarose top layer is scraped off from three nearly confluent plates. These layers are combined, 20 ml of SM and 0.4 ml of chloroform are added and the resulting mixture is shaken at 37°C for 30 min. Cellular debris and agarose are removed by centrifugation, the supernatant is recovered and its volume adjusted to 18 ml with SM. An equal volume of 2 M NaCl, 20 % PEG6000 (BDH, Poole, GB) in SM is added and the solutions are mixed and placed on ice. After 75 min the phages are pelletted by centrifugation for 20 min at 12000 x g at 4°C. The supernatant is decanted and the remaining fluid is removed with a Kleenex tissue. The pellet is resuspended in 3 ml SM and subsequently extracted with 3 ml of chloroform. The aqueous phase is treated with RNase A (67 µg/ml) and DNase I (33 µg/ml) for 20 min at 37°C. Then this mixture is extracted by adding 2 ml of phenol, vortexing, adding 1 ml of chloroform, vortexing again and separating the two phases by centrifugation. The aqueous phase is extracted twice more, with 3 ml of phenol/chloroform (1:1) and 3 ml of chloroform, respectively. Then the DNA is precipitated from the aqueous phase by the sequential addition of 0.3 ml 3 M sodium acetate buffer (pH 5.2) and 6 ml of ethanol. This mixture is left at 4°C for 16 h and then the DNA is recovered by centrifugation (10 min, 12000 x g, 4°C). The pellet is dissolved in 0.4 ml of TE buffer, RNase A is added to 200 µg/ml, and incubated at 37°C for 1 h. The DNA is precipitated, by the addition of 38 µl 3 M sodium acetate buffer (pH 5.2) and 0.8 ml ethanol at 4°C for 1 h. The DNA is recovered by centrifugation and subsequently dissolved in 100 µl of TE buffer.

Example 3.6: Restriction analysis of the A. niger N400 PGII and PGI lambda clones

From the positive phages containing PGII gene sequences, λD and λE are selected for further analysis. First it is established by restriction analysis that both phages contain inserts which are derived from the same region of the A. niger genome and next a partial restriction map of λE is constructed.

2 µg of phage DNA is digested with 20 units of EcoRI or BamHI or the combination of these enzymes in a volume of 100 µl for 3 h at 37°C in the buffer recommended by the supplier (BRL) and in the presence of 0.8 mg/ml RNase A. Then 10 µl of a 3 M sodium acetate buffer (pH 5.2) is added and the mixture is extracted with 80 µl of chloroform. The DNA is precipitated from the aqueous phase by the addition of 250 µl of ethanol and the mixture is placed on ice for 1 h. The DNA is collected by centrifugation, dissolved in 25 µl of 1 x sample buffer and heated at 65°C for 10 min. The samples are run on a 0.7 % agarose gel using 1 µg of lambda DNA (BRL) digested with HindIII as size markers. The gel is photographed on a UV transilluminator using polaroid 667 film. The DNA is transferred to a Schleicher and Schuell BA85 nitrocellulose membrane according to the method of Southern (1975) as described by Maniatis et al. pp. 382-386; (ref. 6). Then the membrane is baked and hybridized with the combined, labelled oligonucleotide mixtures HR6195 and HR6196 as described in Example 3.3.

Fragment lengths are calculated from the photograph and autoradiograms by comparison with the known marker bands. The sizes of the fragments smaller than 5 kbp obtained after the digestion of DNA isolated from phages λD and λE with EcoRI, BamHI and the combination of these enzymes, respectively, are given in Table III. With the resolving power of the electrophoresis system employed, it is not meaningful to compare fragments with a greater size. As judged from the presence of a few bands with a reduced intensity in the EcoRI digests, which are indicated in Table III, EcoRI has not completely digested the DNA. The fragments that are present in the EcoRI/BamHI double digest, but not in either one of the single digests, have intensities comparable with those of BamHI fragments in the same size range. Therefore they must represent completely digested products. The fragments that hybridize with the combined oligonucleotide mixtures HR6195 and HR6196 are, in the case of λE, a fragment larger than 10 kbp in the BamHI digest, a 7.4 kbp EcoRI fragment and a fragment larger than 10 kbp in the EcoRI digest and a 2.8 kbp fragment as well as a fragment larger than 10 kbp in the BamHI/EcoRI double digest.

In the case of λD the fragments that hybridize with the combined oligonucleotide mixtures HR6195 and HR6196 are: a fragment larger than 10 kbp in the BamHI digest, a 5.8 kbp fragment and a fragment larger than 10 kbp in the EcoRI digest and a 1.2 kbp fragment as well as a fragment larger than 10 kbp in the BamHI/EcoRI double digest. When more fragments are observed, e.g. in the digests with EcoRI, the larger fragment is regarded as a product which arises from partial cleavage. The hybridization of fragments of equal size obtained from λD and λE with the oligonucleotide mixtures is not observed.

From the differences between phages λD and λE enumerated above it is concluded that phages λD and λE are not identical. However, Table III lists two EcoRI fragments, three BamHi fragments and at least four BamHI-EcoRI fragments that have the same size in λD and λE. These fragments must originate from the inserts of these phages, since the vector does not contain EcoRI or BamHI sites, except for the BamHI sites that were used to insert the fragments of Aspergillus DNA in the vector and the EcoRI sites flanking these BamHI sites and thus flanking the insert as well (Frischauf et al. , ref. 9). Therefore, it is concluded that the inserts of phage λD and λE have been derived from the same region of the A. niger genome. In combination with the fact that the relevant hybridizing BamHI-EcoRI fragment from λD is smaller than the corresponding fragment from λE, e.g. 1.2 kbp versus 2.8 kbp, and under the assumption that the observed differences in the restriction pattern are not the result of a cloning artefact, it follows that the EcoRI site of the hybridizing 1.2 kbp BamHI-EcoRI fragment of λD is not derived from A. niger DNA and is one of the EcoRI sites that flank the insert of this phage. In addition, it follows that in one direction the insert of λD extends at the most 1.2 kbp beyond the sequence that hybridizes with the oligonucleotide mixture. Moreover, it is inferred that the sequence of λE that hybridizes with the oligonucleotide mixture is located within 1.2 kbp from the BamHI site at the border of the hybridizing 2.8 kbp BamHI-EcoRI fragment (Fig. 1).

Table III: The approximate size of the fragments (in kbp) smaller than 5 kbp obtained after the digestion of DNA isolated from phages λD and λE with the restriction enzymes EcoRI, BamHI and the combination of these enzymes, respectively. (P) denotes fragments that probably result from partial cleavage.

| EcoRI | | BamHI | | EcoRI + BamHI | |
|---|---|---|---|---|---|
| λD | λE | λD | λE | λD | λE |
| | | | | 4.4 | 4.4 |
| 3.8 | 3.8 | | | | |
| | 3.5 (P) | | | | |
| 3.2 (P) | | | | | 2.8 |
| 2.4 | 2.4 | 2.4 | | 2.4 | |
| | | 2.2 | 2.2 | 2.2 | 2.2 |
| | | 1.6 | 1.6 | 1.6 | 1.6 |
| | | | | 1.5 | 1.5 |
| | | | | 1.2 | |
| | 0.98 | | | | |
| | | | | 0.84 | 0.84 |
| | | | | 0.72 | 0.72 |
| 0.68 | | | | 0.68 | |
| | | 0.62 | 0.62 | 0.62 | 0.62 |

In order to construct a partial restriction map of λE, phage DNA is digested with EcoRI, XbaI, HindIII and all possible combinations of these enzymes. This is carried out in two steps. First 6 µg of DNA is incubated for 105 min at 37°C either in the presence or in the absence of 200 U of EcoRI, in a volume of 200 µl containing the buffer recommended by the supplier (BRL) plus RNase A (2 mg/ml). Then the reaction mixtures are extracted with 100 µl phenol/chloroform (1:1) and then with 100 µl of chloroform. DNA is precipitated from the aqueous phase by the addition of 0.1 volume of a 3M sodium acetate buffer and 2 volumes of ethanol. After standing on ice for 10 min, the precipitates are collected by centrifugation. The pellets are dried in the air and dissolved in 100 µl TE buffer. Then these solutions are used for subsequent incubations, either in the absence of restriction enzyme (EcoRI digested material only) or in the presence of HindIII, XbaI or the combination of these enzymes. Incubations are carried out for 2 h at 37°C in a volume of 20 µl, containing 10 µl of the appropriate DNA solution, RNase A (2 mg/ml), 10 U of the appropriate restriction enzyme and the buffer recommended by the supplier (BRL). The incubation is stopped by the addition of 5 µl of 5 times concentrated sample buffer and subsequently the samples are analyzed as described above.

On the autoradiogram a single hybridizing band, which is larger than 10 kbp, is detected in the digests with HindIII, XbaI or the combination of these two enzymes. A band of about the same size is present in all other digests, although at a lower intensity, which indicates that the enzymes have not completely restricted the DNA. The second and most strongly hybridizing band in the EcoRI digest represents a 7.4 kbp fragment. Again indicating incomplete digestion, this band is present in the digests with EcoRI and a second enzyme, although at a lower intensity. In addition, the EcoRI/HindIII double digest and the EcoRI/HindIII/XbaI triple digest give a hybridizing fragment of 3.6 kbp. A 4.1 kbp hybridizing fragment is detected in the double digest with the enzymes EcoRI and XbaI. This fragment is also present in the triple digest with the enzymes EcoRI, HindIII and XbaI, but at very low intensity. In the latter case, the 4.1 kbp fragment is regarded as a partial cleavage product.

It is concluded that the 7.4 kbp EcoRI fragment that hybridizes with the combined oligonucleotide mixtures HR6195 and HR6196 can be cleaved with either one of the enzymes XbaI, HindIII or BamHI and that the location of the cleavage site of these enzymes must be as shown in Figure 1. This figure only gives a partial restriction map, e.g. the 7.4 kbp EcoRI fragment might still contain more than one cleavage site for either one of the enzymes HindIII, XbaI and BamHI, in which case only the site closest to the specific sequence that hybridizes with the combined oligonucleotide mixtures HR6195 and HR6196 is shown.

With phage λD no XbaI or HindIII digestion is performed, but it is assumed that the XbaI and HindIII restriction sites are located as in phage λE.

From the nine positive clones, which are carrying PGI gene sequences, the four phages PG I -λ4, λ5, λ6 and λ7 are selected for further analysis. First it is established that the phages contain inserts derived from the same region of the A. niger genome and next a partial restriction map of λ5 is made as described above for the PGII gene sequence containing phages D and E. The DNA is digested with restriction enzymes (for enzymes see Table IV) as recommended by the enzyme suppliers. The fragments are separated on an agarose gel and blotted onto nitrocellulose as described above. Then the membrane is baked and hybridized with a previously nick-translated 1.2 Kb Bam HI/Eco RI fragment of the structural gene coding for PG II. This fragment is derived from the plasmid pGW1803 which is described in Example 3.8. The fragment is isolated from slices of an agarose gel and 50 ng of the fragment is nick-translated as described by Maniatis et al. (pp. 109-112; ref. 6). Before adding it to the hybridization mixture the nick-translated DNA is denatured for 10 min in a boiling water bath. The nitrocellulose filters are hybridzed with the radioactive probe as described in Example 3.8 except that the prehybridization and hybridization temperature is 60°C. Then the membranes are washed at 60°C using a series of prewarmed buffers. First the membranes are rinsed in 6 x SSC and washed twice for 0.5 h in hybridization buffer. Then the filters are sequentially washed in 4 x SSC and 2 x SSC for 30 min per buffer. These buffers contain both 0.1% SDS and 0.1% $Na_4P_2O_7x10H_2O$. After the last wash the membranes are shortly rinsed in 0.1xSSC and then they are allowed to dry and are subsequently exposed to X-ray film.

The probe used represents a small part of the PG II promoter region (200 bp) and a large part of the coding sequence. Under the hybridization conditions described this probe gives strong signals with all the phages isolated above (see Example 3.4) containing PGI gene sequences.

Fragment lengths are calculated from the photograph and autoradiograms by comparison with the known marker bands. In Table IV the hybridizing fragments and their approximate sizes are listed.

Table IV: The approximate size of the hybridizing fragments (kbp) obtained after digestion of DNA isolated from PG I λ4-7 with the restriction enzymes EcoRI, BamHI, XhoI and combinations of these enzymes as well as by BamHI/BglII

| Restriction enzyme | Phage λ4 | λ5 | λ6 | λ7 |
|---|---|---|---|---|
| BamHI | 8.6 | 8.6 | ≥ 23 | 8.6 |
| EcoRI | 6.4 | 6.4 | 6.4 | 6.4 |
| EcoRI/BamHI | 6.4 | 6.4 | 6.4 | 6.4 |
| XhoI | ≥ 23 | ≥ 23 | ≥ 23 | ≥ 23 |
| XhoI/EcoRI | 2.0 | 2.0 | 2.0 | 2.0 |
| BamHI/BglII | 7.5 | 7.5 | ≥ 23 | 7.5 |

From Table IV it is clear that all phages contain an Eco RI fragment of 6.4 kbp and a Xho I/Eco RI fragment of 2.0 kbp whereas an Eco RI/Bam HI double digest also results in a 6.4 kbp fragment. The phages λ4, λ5 and λ7 have also identical fragments when digested with Bam HI (8.6 kbp) and Bam HI/Bgl II (7.5 kbp). The vector does not contain Bam HI sites except for the Bam HI sites used to insert the fragments of Aspergillus DNA (Frischauf et al ., ref. 9). The inserts of these three phages are therefore considered to be derived from the same region of the A. niger genome.

In order to construct a partial restriction map λ5 phage DNA is also digested with Kpn I, Sma I, sst I and Sal I and with Bam HI in combination with these enzymes. Moreover, a Bgl II/Xho I digest is analyzed. All these incubations are also carried out for 3 h at 37° C, except for Sma I (30° C), in a volume of 20 µl containing 1 µg phage DNA. The fragments hybridizing with the PG II probe at 60° C are listed in Table V.

Table V: The approximate size of the hybridizing fragments (kbp) obtained after digestion of DNA isolated from PG I-λ5 with the restriction enzymes BglII, KpnI, SmaI, SstI, SalI and with combinations of BamHI with these enzymes or with XhoI. Also a double digest of BglII/XhoI is listed.

| Restriction enzyme | Fragment length (kbp) | Restriction enzyme | Fragment length (kbp) |
|---|---|---|---|
| BamHI | 8.6 | | |
| BglII | 9.7 | BamHI/BglII | 7.5 |
| KpnI | 5.1; 2.7 | BamHI/XhoI | 4.2 |
| EcoRI | 6.4 | BglII/XhoI | 3.0 |
| SmaI | 4.7 | BamHI/KpnI | 4.8; 2.8 |
| SstI | 9.9 | BamHI/SstI | 8.3 |
| SalI | 8.9 | BamHI/SalI | 8.5 |

A restriction map of PG I-λ5 can be designed from the hybridizing fragments from which it can be concluded that the gene coding for polygalacturonase I is located on the 8.6 kbp Bam HI fragment.

Example 3.7: The construction of pGW1800 and pGW1803

The relevant EcoRI-XbaI restriction fragments of phages λD and λE are inserted into pEMBL vectors (Dente and Cortese, ref. 10), resulting in plasmids pGW1803 and pGW1800, respectively.

From Fig. 1 and Example 3.5 it is inferred that phage λD must have a 2.5 kbp EcoRI-XbaI fragment that is identical to part of the 4.1 kbp EcoRI-XbaI fragment of phage λE. 6 µg of λD DNA are digested with 60 U of XbaI for 2 h at 37° C in a volume of 200 µl in the buffer recommended by BRL plus RNase A (1.5 mg/ml). Then the NaCl concentration is adjusted to 140 mM, concentrated reaction buffer is added to compensate for the increase of volume, 60 U of EcoRI are added and the incubation is continued for 2.5 h in a volume of 230 µl. Then the reaction mixture is extracted with 100 µl of chloroform and the DNA is precipitated from the aqueous phase by the addition of 0.1 volume of 3 M sodium acetate buffer (pH 5.2) and 2 volumes of ethanol. After incubation at 4° C for 16 h the DNA is recovered by centrifugation, the pellet is dried in the air and then dissolved in sample buffer. The restriction fragments are separated on a 0.6 % low melting point agarose (BRL) gel in 0.5 x TBE buffer containing ethidium bromide. The DNA fragments are visualized under UV light and a gel slice containing the 2.5 kbp EcoRI-XbaI fragment is isolated.

Phage λE DNA is incubated with the restriction enzymes XbaI and EcoRI, essentially as described above. Following extraction with chloroform, the DNA is precipitated, pelletted by centrifugation, dissolved in sample buffer and subjected to electrophoresis on a 0.6 % agarose gel in 1 x TBE buffer. A gel slice containing the 4.1. kbp XbaI-EcoRI fragment is recovered and it is stored at -20° C. A plug of silanized glass wool is placed at the bottom of a 0.5 ml micro-centrifuge test tube containing 60 µl of high TE buffer (100 mM Tris-HCl, pH 8.0, 10 mM EDTA). The gel slice is thawed, put in the test tube and the tube is frozen in liquid nitrogen. A small hole is punched in the bottom of the small tube and it is placed in a 1.5 ml micro-centrifuge test tube and the buffer and DNA from the gel slice are collected by centrifugation. The remnants of the gel slice are resuspended in 50 µl of high TE buffer, this suspension is frozen again in liquid nitrogen and the buffer is collected by centrifugation. Then the eluates are combined and extracted with 100 µl of chloroform. The DNA is precipitated from the aqueous phase, collected by centrifugation and dissolved in 40 µl of TE buffer. The DNA concentration is estimated by agarose gel electrophoresis followed by visualisation of the band under UV light, using 1 µg of lambda DNA (BRL) digested with HindIII as a reference.

pEMBL18 and pEMBL19 vectors are prepared by sequential digestion with XbaI and EcoRI, under the conditions recommended by the supplier (BRL). The extraction of DNA with phenol, phenol/chloroform (1:1) and chloroform and the precipitation of DNA with ethanol are carried out as described by Maniatis (ref. 6), except for the omission of isoamylalcohol from the chloroform solution and the temperature used for the precipitation of DNA, is 4° C.

DNA fragments are ligated to the appropriate vector in a reaction volume of 25 µl, containing the buffer recommended by BRL plus ATP (1 mM), 1.5 U of T4 DNA ligase (BRL), 100 ng of vector DNA, which has been prepared as described above, and the relevant restriction fragment. XbaI and EcoRI digested pEMBL18 is used for the construction of pGW1800 plus an equimolar amount of the purified 4.1 XbaI-EcoRI fragment of λE.

The reaction mixture is incubated for 16 h at 16° C and then the reaction is stopped by the addition of 125 µl of distilled water and freezing. XbaI and EcoRI digested pEMBL19 used for the construction of pGW1803. In this case, the slice of agarose that contains the 2.5 kpb XbaI-EcoRI fragment of D is molten at 60° C and 4 µl, containing ca. 15 ng of DNA, are added to 11 µl of distilled water, after which the other components of the reaction mixture are added. The ligation reaction is allowed to proceed for 14 h at 20° C, then 1 additional unit of T4 DNA ligase is added. The reaction is continued for another 7 hrs and then the reaction is stopped as described above.

50 µl of the resulting ligation reaction mixtures are used to transform E. coli as described in the BRL M13 cloning/dideoxy sequencing manual (pp. 30-33; ref. 11) except that E. coli DH5αF′ and E. coli JM 109 are grown to an $OD_{550}$ value of 0.7 and 0.9, respectively, before placing the flasks on ice. The former strain is transformed using the ligation reaction mixture with the fragment of λD, the latter strain is transformed using the ligation reaction mixture with the fragment of λE. After the heat shock the cells are incubated on ice for two min and then 1 ml of LB medium is added and the cells are incubated at 37° C for 1 h. The cells are pelletted by low speed centrifugation, 1 ml of the supematant is removed and the cells are gently resuspended. Then the cells are plated on LB agar plates containing 100 µg/ml ampicillin and on which an IPTG/X-gal solution (= 200 µl $H_2O$, 30 µl dimethylformamide containing 2 % X-gal and 20 µl of a 24 mg/ml IPTG solution in water) had been spread. The plates are incubated overnight at 37° C.

Several single white colonies are used to prepare overnight cultures in LB medium supplemented with 0.1 % glucose and 75 µg/ml ampicillin. These cultures are used to isolate plasmid, using the miniprep method of Holmes and Quigley (ref. 12). The plasmids are digested with several restriction enzymes, according to the recommendations of the supplier (BRL) and in the presence of RNase A (0.5 mg/ml), and the products are analyzed on an agarose gel. Plasmids that give rise to XbaI-EcoRI, BamHI-EcoRI and HindIII fragments of the expected size are selected and the E. coli cells harbouring them are kept on glycerol at -20° C.

Two new plasmids are constructed. pGW1800 is the 4.1 kbp XbaI-EcoRI fragment of phage λE inserted into the vector pEMBL18. pGW1803 is the 2.5 kbp XbaI-EcoRI fragment of phage λD inserted into the vector pEMBL19.

Example 3.8: Restriction analysis of pGW1800 and pGW1803

The relationship between pGW1800 and pGW1803 is confirmed by extended restriction analysis. The specific sequence that hybridizes with the combined oligonucleotides HR6195 and HR6196 is designated to a specific region of pGWI803. The absence of cloning artifacts, such as deletions or ligation of A. niger derived DNA fragments, is established by comparison of restriction fragments obtained from plasmid pGW1800 and from A. niger N400 DNA, respectively.

pGW1800 and pGW1803 are propagated in E. coli strain JM109 and DH5αF′, respectively, and plasmid DNA is recovered from 250 ml overnight cultures in LB medium supplemented with 0.1 % glucose and 100 µg/ml ampicillin as described by Maniatis et al. (pp 90-91; ref. 6) and finally resuspended in TE buffer. Since pGW1800 is also used to transform A. niger , it is purified by banding in a cesium chloride gradient. To 2.5 ml DNA solution in TE, 3.3 g CsCl and 1 ml ethidium bromide (10 mg/ml in water) are added. Centrifugation takes place in a Beckman® VTi 65.2 rotor at 20° C for 16 h at 45,000 rpm. Of the two fluorescent bands visible under UV light, the lower one containing covalently closed circular plasmid DNA is recovered by side puncturing of the tube. The DNA solution (approx. 1 ml) is extracted 5 times with water saturated butanol to remove the ethidium bromide. Then the volume is adjusted to 15 ml with water and the DNA is precipitated by the addition of 30 ml of ethanol. The DNA is collected by centrifugation and next the pellet is washed once with 75 % ethanol and subsequently it is dissolved in TE buffer and stored at -20° C. pGW1803 is not isolated from a CsCl gradient, but it is subjected to a faster procedure. RNase A is added to 4 ml DNA solution to a concentration of 100 µg/ml and this mixture is incubated at 37° C for 1 h and subsequently extracted with an equal volume of phenol, phenol/chloroform (1:1) and chloroform. Then the DNA is precipitated from the aqueous phase by the addition of 0.4 ml 3M sodium acetate buffer pH 5.2 and 8 ml ethanol. The DNA is collected by centrifugation, the resulting pellet is washed with 75 % ethanol and dried in the air and subsequently dissolved in TE buffer.

A. niger DNA is isolated by a slightly modified procedure used to isolate plant RNA (Slater, ref. 21). Mycelium is washed with saline, frozen in liquid nitrogen and 2.5 g is disrupted using a microdismembrator (Braun). The mycelial powder is extracted with freshly prepared extraction buffer. The extraction buffer is prepared as follows: 5 ml tri-isopropylnaphthalene sulphonic acid (TNS, 20 mg/ml) is mixed with 5 ml p-aminosalicylic acid (PAS) (120 mg/ml) and 2.5 ml 5 x RNB buffer (5 x RNB contains 121.1 g Tris, 73.04 g NaCl and 95.1 EGTA in 1 l, pH 8.5). After the addition of 7.5 ml phenol, the extraction buffer is equilibrated for 10 min at 55° C. The warm buffer is then added to the mycelial powder and the suspension is mixed for 2 min. Then 5 ml chloroform is added and mixed for 2 min. The phases are separated by centrifugation (10 min, 10.000 x g) and the aqueous phase is extracted once more with 10 ml of phenol/chloroform (1:1) and then twice with chloroform.

The aqueous phase contains both RNA and DNA. The DNA is precipitated with 2 vol ethanol at room temperature and collected by centrifugation (10 min, 10.000 x g), washed twice by redissolving in distilled sterile water and precipitating it again with ethanol. RNA is removed by adding RNase A (20 µg/ml) to the final solution.

In order to construct physical maps of pGW1800 and pGW1803, several reaction mixtures are prepared that contain approximately 1 µg of plasmid DNA, RNase A in concentration of 1 mg/ml (only in the case of pGW1803), the relevant buffer as recommended by BRL and 10 units of a restriction enzyme, whereas different enzymes are chosen for the separate reaction mixtures. In several cases a combination of two different enzymes is chosen, or plasmid DNA is restricted with a certain enzyme or combination of enzymes, followed by precipitation of the DNA in the presence of sodium acetate and ethanol and subsequent recollection of the DNA by centrifugation, which material is then used as substrate for a second or third restriction enzyme. The reaction mixtures are incubated at 37° C for 1 h, then the reaction is stopped by the addition of 5 times concentrated sample buffer and next the reaction products are analyzed on an 1 % agarose gel in TBE buffer. From the calculated size of the specific fragments the position of the individual restriction enzyme sites relative to a reference point, which is arbitrarily chosen at the unique XbaI site, is inferred. The restriction map of pGW1800 is given in figure 2. The restriction map of the A. niger derived DNA of pGW1803 (not shown) is identical with the map of pGW1800 from the XbaI site at position 1 to the HincII site at position 2000. However, pGW1803 does not contain a BglII site, which is present at position 2400 in pGW1800. The 0.4 kbp HincII-BglII fragment of pGW1800 showed the same electrophoretic mobility as the HincII-EcoRI fragment of pGW1803 which is located at the end of the A. niger derived insert DNA. It is concluded that the A. niger derived DNA of pGW1803 is identical with the A. niger derived DNA of pGW1800 between the XbaI site at position 1 and a location very close to, or at, but not including, the BglII site at position 2400. Thus, the inferred size of the EcoRI-XbaI fragment of phage λD, 2.5 kbp (Example 3.6), presents a slight over-estimation.

In order to locate the specific sequence that hybridizes with the combined oligonucleotide mixtures HR6195 and HR6196, pGW1803 DNA is restricted and the resulting fragments are separated as described above. Then the DNA is transferred to a nitrocellulose membrane and is allowed to hybridize with the combined and labelled oligonucleotide mixtures HR6195 and HR6196, as described hereinbefore. The fragments that hybridize with the combined oligonucleotide mixtures HR6195 and HR6196 are a 0.64 kbp HincII fragment in the HincII digest and a 0.62 kbp NcoI-EcoRI fragment in the NcoI/EcoRI double digest. It is concluded that the specific sequence that hybridizes with the combined oligonucleotide mixtures HR6195 and HR6196 is located between the NcoI site at position 1750 and the HincII site at position 2000 of the restriction map of pGW1803, which coordinates correspond with the same sequence on the restriction map of pGW1800.

The absence of cloning artifacts is established by comparing restriction fragments from pGW1800 with restriction fragments obtained from A. niger N400 DNA. Digests of pGW1800 are prepared as described above. Digests of chromosomal DNA of strain N400 are made at 37° C in a reaction volume of 200 µl consisting of 2 µg DNA, 0.5 mg/ml RNase A, a reaction buffer supplied by BRL (REact Buffer 2 for XhoI, XbaI, EcoRV and BglII, REact Buffer 4 for KpnI and REact Buffer 3 for EcoRI and SalI) and 80 units for each restriction enzyme used. The reaction mixture is incubated for 2 h and then extracted with 100 µl of chloroform. Subsequently the DNA is precipitated from the aqueous phase by the addition of 20 µl of a 3 M sodium acetate buffer pH 5.2 and 0.5 ml ethanol followed by standing on ice for 45 min. Then the DNA is recollected by centrifugation and dried on the air. The DNA is dissolved in sample buffer and these samples and the digests of pGW1800 and lambda DNA size markers are loaded on an 0.7 % agarose gel in TBE buffer. After electrophoresis the resulting pattern is documented and the DNA in the lanes with the digests of A. niger chromosomal DNA is transferred to a nitrocellulose membrane, both as described hereinbefore. The membrane is baked and cut in two parts, which are hybridized with different probes.

The probes used for hybridization are the nick-translated 1.45 kbp and 2.05 kbp EcoRV-BglII fragments of pGW1800. The fragments are previously isolated from slices of an agarose gel as described hereinbefore and 100 ng of the fragments are nick-translated in separate reactions as described by Maniatis et al. (pp 109-112; ref. 6). Before adding it to the hybridization mixture the nick-translated DNA is denatured for 10 min in a boiling water-bath.

The baked nitrocellulose filters are wetted in 3xSSC and are then transferred to separate dishes containing 100 ml prewarmed (68° C) hybridization buffer which consists of 6xSSC, 10 mM EDTA, 0.1 mg/ml freshly added sheared and denatured herring sperm DNA, 0.1 % $Na_4P_2O_7x10H_2O$, 0.5 % SDS and 5x Denhardt's (0.1 % BSA, Boehringer fraction V, 0.1 % Ficoll 400, Pharmacia; 0.1 % polyvinylpyrrolidone-10, Sigma). Prehybridization is for 2 h at 68° C in a shaking water bath and then the hybridization buffer is replaced with fresh buffer (75 ml) to which a nick-translated probe is subsequently added. The hybridization is allowed to proceed at 68° C for at least 16 h Then the membranes are washed at 68° C, using a series of prewarmed buffers that all contain 0.1 % SDS and 0.1 % $Na_4P_2O_7x10H_2O$, but with a decreasing quantity of SSC. First, the membranes are rinsed in 4xSSC and then they are washed in 4xSSC twice for 10 min. Then the filters are sequentially washed in 4xSSC, 2xSSC, 0.5xSSC, 0.2xSSC and 0.1xSSC for 30 min. per buffer. After the last wash the membranes are shortly rinsed in 0.1xSSC and then they are allowed to dry and they are subsequently exposed to X-ray film.

The membrane probed with the 1.45 kbp EcoRV-BglII fragment contains the following hybridizing fragments: a 2.4 kbp fragment in the XhoI digest, a 2.8 kbp and a 1.2 kbp fragment in the KpnI digest and a 1.3 kbp fragment in the BglII/EcoRV double digest. The membrane probed with the 2.05 kbp EcoRV-BglII

fragment contains the following fragments: a large fragment of approximately 11 kbp in the EcoRI/SalI double digest, 2.3 kbp and 2.1 kbp fragments in the XhoI digest, 2.3 kbp and 1.4 kbp fragments in the Xho/XbaI double digest, 1.2 kbp fragments in the KpnI digest and a 2.0 kbp fragment in the EcoRV/BglII double digest.

For four of the hybridizing fragments only the existence and a minimum size can be inferred from the restriction map of pGW1800, i.e. fragments which have homology with a probe used but of which one end is outside the sequence which is present in pGW1800. These fragments are, in the case of the 2.05 kbp EcoRV-BglII probe, the fragment in the EcoRI/SalI digest, the 2.1 kbp fragment in the XhoI digest and the 3.2 kbp fragment in the KpnI digest, and in the case of the 1.45 kbp EcoRI-BglII probe, the 2.8 kbp fragment in the KpnI digest. These fragments are all larger than the minimum size which is inferred from the restriction map of pGW1800. The different hybridizing fragments of genomic A. niger DNA can be correlated with fragments present in the digests of pGW1800 and all have a calculated size which is very close to the size which is inferred from the restriction map of pGW1800. It is concluded that the A. niger derived DNA of pGW1800 is a faithful representation of part of the A. niger N400 genome.

Example 3.9: The construction of pGW1900 and pGW1902 and their restriction analysis.

The 8.6 kbp Bam HI restriction fragment of phage PGI-λ7 which hybridizes with PGII coding sequences (see Table IV, Example 3.6) is inserted into the pUC9 vector (Vieira, J. and Messing, J., 1982, Gene 19, 259-268) resulting in plasmids pGW1900 (see Figure 3) and pGW1901 which have the insert in opposite orientation. To 1.5 μg PGI-λ7 DNA in 5 μl TE buffer, 1 μl spermidine of a 10 mM stock solution, 20 U Bam HI, the reaction buffer recommended by BRL and sterile distilled water are added to a final volume of 30 μl. To the digested samples one quarter (v/v) of loading buffer is added (loading buffer = 0.25% bromophenol blue; 0.25% xylene cyanol and 15% Ficoll 400 in $H_2O$). The reaction fragments are separated on a 0.6% agarose gel in 1 x TAE buffer containing ethidium bromide (1 μg/ml) (50 x TAE buffer = 242 g Tris, 57.1 ml acetic acid and 100 ml 0.5 M EDTA pH 8.0 per liter).

The DNA fragments are visualized under UV light and a gel slice containing the 8.6 kbp Bam HI fragment is isolated. DNA fragments are electrophoretically isolated according to conventional methods Elution is performed at 100 V during 1 h. The DNA is collected and precipitated with 2 vol of ethanol. After centrifugation in an Eppendorf centrifuge for 30 min the precipitate is collected, dried in a Speedvac and dissolved in 10 μl TE buffer. Vector pUC9 (1 μg) is linearized using 10 U Bam HI for 37° C during 1.5 h using the recommended BRL buffer in a total reaction volume of 20 μl. Subsequently 1 μl CIP solution (approx. 2U) is added and the mixture is incubated for 30 min at 37° C. The linearized vector is also prepared by electrophoresis. It is dissolved in 20 μl TE which corresponds with a DNA concentration of approx. 50 ng/μl.

In the ligation reaction one μl of the linearized and CIP treated pUC9 vector (50 ng) and 4 μl of the Bam HI fragment (approx. 100 ng) are ligated with 1.2 U $T_4$ DNA ligase in appropriate ligase buffer. The final reaction volume is 10 ml. The reaction is allowed to proceed for 14 h at 14° C and then the mixture is diluted with TE to 50 μl.

50 μl of the resulting ligation reaction mixtures are used to transform E. coli as described in the BRL M13 cloning/dideoxy sequencing manual (pp. 30-33; ref. 11) except that E. coli DH5aF′ is grown to an $OD_{550}$ value of 0.5 to 0.7 before placing the flasks on ice. After the heat shock the cells are incubated on ice for two min and then 1 ml of LB medium is added and the cells are incubated at 37° C for 1 h. The cells are pelletted by low speed centrifugation, 1 ml of the supernatant is removed and the cells are gently resuspended. Then the cells are plated on LB agar plates containing 100 μg/ml ampicillin and on which an IPTG/X-gal solution (= 200 μl $H_2O$, 30 μl dimethylformamide containing 2 % X-gal and 20 μl of a 24 mg/ml IPTG solution in water) had been spread. The plates are incubated overnight at 37° C. Several single white colonies are used to prepare overnight cultures in LB medium supplemented with 0.1 % glucose and 75 μg/ml ampicillin. These cultures are used to isolate plasmid, using the miniprep method of Holmes and Quigley (ref. 12). The plasmids are digested with several restriction enzymes, according to the recommendations of the supplier (BRL) and in the presence of RNase A (0.5 mg/ml), and the products are analyzed on an agarose gel. Plasmids that give rise to BamHI fragments of the expected size are selected and the E. coli cells harbouring them are kept on glycerol at -20° C. The new plasmids pGW1900 (shown in Figure 3) and pGW1901 carrying the insert in the opposite direction are used for further experiments.

pGW1900 is propagated in E. coli strain DH5aF′ and plasmid DNA is recovered from 250 ml overnight cultures in LB medium supplemented with 0.1 % glucose and 100 μg/ml ampicillin as described by Maniatis et al. (pp 90-91; ref. 6) and finally resuspended in TE buffer. Since pGW1900 is also used to transform A. niger , it is purified by banding in a cesium chloride gradient. To 2.5 ml DNA solution in TE, 3.3 g CsCl and 1 ml ethidium bromide (10 mg/ml in water) are added. Centrifugation is performed in a Beckman® VTi 65.2 rotor at 20° C for 16 h at 45,000 rpm. Of the two fluorescent bands visible under UV light, the lower one containing covalently closed circular plasmid DNA is recovered by side puncturing of the tube. The DNA solution (approx. 1 ml) is extracted 5 times with water saturated butanol to remove the ethidium bromide. Then the volume is adjusted to 15 ml with water and the DNA is precipitated by the addition of 30 ml of ethanol. The DNA is collected by centrifugation and next the pellet is washed once with 75 % ethanol and subsequently it is dissolved in TE buffer and stored at -20° C.

In addition, from a pGW1900 Kpn I digest a 2.7 kbp Kpn 1 DNA fragment is electrophoretically isolated from a slice of agarose gel and is inserted into pEMBL18 (Dente & Cortese, ref. 10) resulting in plasmids pGW1902 (see Figure 4) and pGW1903, respectively.

In order to construct a physical map of pGW1900 and pGW1902 several reaction mixtures are prepared that contain approx. 1 µg of plasmid DNA, the relevant buffer as recommended by BRL and 10 units of a restriction enzyme or a combination of two different restriction enzymes. The reaction products are analyzed on 1% agarose gels in TAE buffer. From the calculated size of the specific fragments the position of the individual restriction enzyme sites in Figure 3 and 4, respectively, is established.

Example 3.10: Molecular cloning of the polygalacturonase II gene (pgaII) of A.niger NW756

The genomic DNA of A. niger NW756 is analyzed by Southern blot analysis using the pgaII gene of A. niger N400 (i.e. the 1.2 kbp BamHI-BglII fragment of pGW1800) as a probe. With respect to what has been described in Example 7.1, two modifications are made. In this case restriction enzymes are chosen which cut within the structural moiety of the A. niger N400 pgaII gene and the hybridization conditions are more stringent, i.e. the "homologous" conditions described in Example 7.2. In the BamHI digest now a single hybridizing fragment of 3.3 kbp is detected. Under these hybridization conditions a single sequence is detected, which sequence is therefore defined as the pgaII gene of A. niger NW756. A single hybridizing HincII fragment of 3.3 kbp is observed, which indicates the absence of a HincII site in the structural gene. The hybridizing XhoI-BglII fragment is 5.5 kbp. These results are in disagreement with the data given in Example 3.7 and the DNA sequence of pgaII of A. niger N400 depicted in the sequence listing under SEQ ID No. 2. Therefore, it is concluded that the pgaII gene of A. niger NW756 is not identical to the pgaII gene of A. niger N400.

A genomic library of A. niger NW 756 is constructed as described for strain N400 in Example 2 with minor modifications. Sau3AI is used to restrict the Aspergillus DNA, instead of its isoschizomer MboI. The library of strain NW756 is constructed in the lambda vector EMBL3, which is a close relative of EMBL4 (ref. 9). EMBL3 DNA, already digested with BamHI and EcoRI and phosphatase treated, has been purchased from Promega. Part of the resulting library is plated and plaque lifts are prepared as described in Example 3.3. The filters are then hybridized with the 1.2 kbp BamHI-EcoRI fragment of pGW1803, using the homologous conditions as described in Example 7.2. Positive phages are purified in a rescreening step and subsequently the DNA of these phages is purified as described in Example 3.4. The DNA is subjected to restriction analysis, using the enzymes BglII and XhoI. Next, the 5.5. kbp XhoI-BglII fragment of one of the positive phages that contain this fragment is isolated as described in Example 3.6. This fragment is then ligated into BamHI and SalI digested pEMBL18 and the resulting ligation mixture is used to transform E. coli JM109 (Example 3.6). The transformants are then analyzed by colony hybridization under "heterologous" conditions as described in Example 7.3. The plasmid DNA of a positive clone is purified and it is subsequently used to construct a physical map (Example 3.7).

The restriction map of pGW1756 is shown in Fig. 6. pGW1756 is the 5.5 kbp XhoI-BglII fragment which contains the A. niger NW756 pgaII gene inserted into the vector pEMBL18.

Example 4: The determination of the nucleotide sequence of the polygalacturonase genes

Example 4.1: The polygalacturonase II gene (pgaII) of A. niger N400

Suitable restriction fragments of pGW1800 and pGW1803 are isolated after agarose gel electrophoresis and ligated into M13mp18RF and M13mp19RF vectors, using a 2-10 fold excess of fragment with respect to the vector. Transformation of E. coli JM109 is carried out as described hereinbefore whereas after the heat shock the cells are immediately plated as described in the BRL M13 cloning/dideoxy sequencing manual (p 34; ref. 11). Isolation of single-stranded DNA templates from recombinant phages is performed as described by Ausubel et al . (section 7.3.9; ref. 40) whereas the cells are harvested in addition to the supernatants and these cells are subsequently stored as a glycerol culture at -20° C.

One of the clones thus obtained, which is the 2.4 kbp XbaI-EcoRI fragment of pGW1803 inserted into the polylinker of M13mp18, is subsequently manipulated in order to be able to obtain sequencing data from the HindIII, PstI, BamHI and NcoI sites respectively. Separate overnight cultures in YT medium are prepared from E. coli JM109 and from the relevant clone, in the latter case using part of the glycerol culture as an inoculum. Then 200 ml of 2xYT medium is inoculated with 0.4 ml of the JM109 culture and this culture is subsequently incubated for 2.5 h in an orbital shaker at 37° C. Then 20 ml of the overnight culture of the relevant clone is added and the incubation in the orbital shaker is continued for 5 h. Replicative form DNA is then isolated from these cells as described for pGW1803 hereinbefore. This DNA is then digested with HindIII, PstI, BamHI and NcoI, respectively. The BamHI and NcoI digested DNA is then digested with SalI, subsequently extracted with phenol/chloroform (1:1) and chloroform and is then ethanol precipitated. The non- compatible sticky ends are then filled in using 5 units of T4 DNA polymerase (BRL) in the buffer as described by Maniatis et al . (p 117; ref. 6) and in the presence of 0.1 mM each of dCTP, dATP, dGTP and dTTP. The reaction mixtures with a volume of 25 µl are incubated for 5 min at 37° C and then 5 µl of 0.5 M EDTA pH 8.0 is added and the mixtures are subsequently phenol extracted. The small DNA fragments present in the four DNA preparations thus obtained, are removed by electrophoresis in a 0.7% low melting point agarose gel (BRL) and gel slices that contain the large fragments are isolated. These DNA fragments are subsequently circularized using T4 DNA ligase. The resulting ligation reaction mixtures are subsequently used to transform E. coli JM109 as described above.

Since BclI is sensitive to methylation of the substrate it can not cut the plasmid DNA isolated from E.coli JM109 or E. coli DH5αF'. Therefore, the previously isolated pGW1800 and pGW1803 are used to transform E. coli JM110 (Yanisch-Perron et al., ref. 29) and the plasmid DNA is subsequently isolated as described in Example 3.8, except for the addition of 0.1% casamino acids to all media used to grow E. coli JM110. The plasmid DNA isolated from E. coli JM110 is then used to obtain subclones which allow sequence analysis from the BclI site.

The resulting clones are sequenced with the T7sequencing™ Kit from Pharmacia, using the conditions recommended by the supplier. In some cases the sequencing data thus obtained are used to program the synthesis of specific oligonucleotides according to the method of Caruthers (ref. 8). These oligonucleotides are HR6425, which is
5'(d)CAAGAACGTCACCATCGAAC3', HR6439, which is
5'(d)GAATTGCTCACGGTGGAGTG3' and HR6440, which is
5'(d)ACTTGGGCTTCTTCTTTCCG3'. These oligonucleotides are subsequently used as specific sequencing primers for the relevant templates. The primer HR6439 gives two superimposed sequencing ladders when used on M13 templates and therefore this primer is used for double strand sequencing of alkali denatured pGW1800, following the instructions of Pharmacia.

The sequence of the gene encoding PGII (pga II) located on pGW1800 is obtained from both strands of DNA. Sequencing in the downstream direction (from the XbaI site at position 1 in the direction of the PvuII site at about position 3028) is performed from the XbaI(1), EcoRV(about 334), HindIII(about 557), PstI (about 827), BclI(about 1056), BamHI(about 1158), HincII (about 1344 and about 1974), KpnI (about 1565 and about 2706), NcoI(about 1749) and the BglII(about 2379) sites, respectively, whereas the primer HR6425 is used to sequence the region of the BglII site. Sequencing in the opposite direction is performed from the PvuII (about 3028 and about 1442), KpnI (about 2706 and about 1565), BglII(about 2379), HincII(about 1974), BamHI(about 1158), BclI(about 1056), PstI(about 827), HindIII(about 557) and EcoRV(about 334) sites, respectively, whereas the primers HR6439 and HR6440 are used to sequence over the HincII(about 1974) and KpnI (about 1565) sites, respectively.

The sequence of pga II is given in the sequence listing under SEQ ID NO. 2. The 3031 bp sequence starts with the first nucleotide of the XbaI site at position 1 in pGW1800 and ends at the last nucleotide of the PvuII site indicated at the approximate map position 3050 in the restriction map of pGW1800. The pga II gene comprises 1356 nucleotides of the promoter region, 1138 nucleotides of the structural part (including a putative intron of 52 nucleotides) and 537 nucleotides of the transcriptional terminator region.

The sequence immediately downstream from the signal sequence and the XhoI cleavage site encodes an amino acid sequence which is in complete agreement with the sequence obtained for the 5 kDa fragment of polygalacturonase II under the prediction that cysteine will be present at position 3 (Example 1.3).

The DNA sequence encodes a leader peptide of 27 amino acids, arginine being the last amino acid before the mature protein sequence. This leader peptide must be removed by proteolytic cleavage. Since signal peptidase cleavage sites are not found immediately after arginine residues and not immediately after charged amino acids in general (von Heijne; ref. 39), the leader peptide is removed in at least two proteolytic steps, i.e. the leader peptide represents a prepro-sequence. In this case, signal peptidase cleaves the pre-sequence, or signal-peptide, whereas the remaining pro-sequence is cleaved by yet another protease.

The polygalacturonase II structural gene contains one intron, most probably comprising the nucleotide sequence from position 1987 to 2038. This sequence contains termination codons in all three possible reading frames. The presence of this intron changes the reading frame, in a manner which is consistent with the protein sequencing data obtained (see Example 1). The reading frame before the intron is confirmed by the amino acid sequences of the cyanogen bromide fragments (Example 1.3), whereas the reading frame after the intron is confirmed by the amino acid sequence of the tryptic peptide TP4 (Example 1.5), which sequence can also be deduced from the nucleotide sequence from position 2183 to 2225. The 5′ splice site of the intron, GTAAGC, resembles the fungal 5′ splice consensus GTPuNGT, whereas the 3′ slice site TAG is in complete agreement with the fungal consensus 3′ splice site PyAG (ref. 41).

Example 4.2: The polygalacturonase I (pgaI) gene of A niger N400

Suitable restriction fragments are isolated from pGW1900 and pGW1902 after agarose gel electrophoresis as described in Example 3.9. These are ligated into M1 3mp 18RF and M13mp19RF vectors according to the BRL M13 cloning/dideoxy sequencing manual (ref. 11). Competent cells are made as described in the Pharmacia Manual for the M13 cloning/sequencing system. Transformation of E. coli JM101 is carried out as described by Messing et al., ref. 30. Isolation of single-stranded DNA templates from recombinant phages follows conventional methods (e.g. ref. 11, pp. 29-34). The resulting clones are sequenced using the T7 sequencing™ kit from Pharmacia (Uppsala, Sweden) using the conditions recommended by the supplier.

pGW1900 is sequenced from the Eco RI site at its approximate map position 5750 until the Cla I site at its approximate map position 3900 which is located close to the Hind III site at about position 3790. Several oligonucleotides (304-307) are synthesized using the method of Caruthers (ref. 8). These oligonucleotides have the following sequences:

Number 304 5′ (d) T T C A G C C C A A G C G T C A A T C C 3′
Number 305 5′ (d) A C C T G A A C G A C T T C A C C A T C 3′
Number 306 5′ (d) T C T G T A G G A C G T C T G G T T G 3′
Number 307 5′ (d) T G G C A G T A A A A C C A C C T A A C 3′

They are used as specific sequencing primers for the relevant templates.

The oligonucleotide number 307 is used for double strand sequencing of alkali denatured pGW1900 following the supplier's instructions for the T7 sequencing™ kit. The entire sequence of the pga I gene is given in the sequence listing under the SEQ ID NO. 1. The sequence is based on the sequencing data obtained with both strands.

The 2495 bp pga I sequence starts with the first nucleotide of the Eco RI site at the approximate map position 6280 in pGW1900 and ends 12 nucleotides beyond the last nucleotide of the Cla I site close to the Hind III site indicated at 3880. The pga I sequence comprises 909 nucleotides of the promoter region, 1218 nucleotides of the structural part, including two putative introns of 52 bp (intron A) and 62 bp (intron B), and 366 nucleotides of the transcriptional terminator region.

The amino acid sequences obtained for the mature polygalacturonase I (see Example 1.4) and for the 21 kDa cyanogen bromide fragment (Example 1.5) are in complete agreement with an amino acid sequence which can be derived from the nucleotide sequence and which starts at position 1003. The DNA sequence thus encodes a leader peptide of 31 amino acids, lysine being the last amino acid before the mature protein starts. For similar reasons as given in the case of PGII where the leader peptide ends with an arginine residue, this PGI leader peptide represents a prepro-sequence which is also removed in at least two proteolytic steps. The sequence which hybridizes with the oligonucleotide mixture used as probe and starts at position 1277 of the nucleotide sequence. There is complete agreement between the N-terminal amino acid sequence determined for the 5.5 kDa cyanogen bromide peptide fragment as described in Example 1 and the putative amino acid sequence based on the nucleotide sequence.

The polygalacturonase I structural gene contains two introns. Intron A comprises the nucleotide sequence from position 1138 to 1189. The 5′ splice site G T A T G T is in agreement with the fungal 5'splice site consensus sequence GT Pu NGT (ref. 41) whereas the lariat sequence GC TAAC and the 3′

splice site TAG also agree with the known consensus sequences Pu CT Pu AC and Py AG. The presence of this intron changes the reading frame in a manner that is consistent with the protein sequence data obtained with the more distally located 5.5 kDa cyanogen bromide fragment (see Example 1). The second intron (B), comprises the nucleotide sequence 1610 up to 1671. Both the lariat sequence and the 3′ splice site agree with the known consensus sequences. The 5, splice site G C A C G A does not agree with the consensus sequence GT Pu NGT, but GC at the 5′ splice site as well as an A at position +6 with respect to the 5′ splice site have been found in some other genes (refs 41 and 43).

The presence of intron B is based on the following arguments:

a) It changes the reading frame whereas in the other two reading frames premature stops occur.

b) The intron in pga II occurs at the same position and the amino acid sequence preceding this intron Asn - Ser - Gly - Glu is identical in both proteins. A region of strong homology is also found between the amino acid sequences deduced from the nucleotide sequences immediately following the putative intron:

Amino acid residue

PG II Asn - Ile - Trp - Phe - Thr Gly - Gly - Thr - Cys
PG I Ser - Ile - Ser - Phe - Thr Gly - Gly - Thr - Cys
PG II Ile - Gly - Gly - His - Gly - Leu - Ser - Ile -Gly
PG I Ser - Gly - Gly - His - Gly - Leu - Ser -Ile - Gly

## Example 4.3: Homology between A. niger N400 polygalacturonases

The polygalacturonases PGI and PGII isolated from A. niger catalyze the same reaction and are immunologically related to each other, as described in Example 1.2.

The genes coding for these enzymes are also closely related since with the pga II gene other polygalacturonase genes are isolated from a genomic A.niger N400 library as described in Example 7.2. A sequence comparison of the putative PG I and PG II amino acid sequences of the mature enzymes which differ 2 residues in length reveals a large degree of homology. These data clearly indicate that genes coding for PGs are closely related and represent members of a polygalacturonase gene family.

## Example 5: Cotransformation of A. niger using the A. niger pyr A gene as selection marker and a plasmid carrying the polygalacturonase I or II gene as cotransforming plasmid

## Example 5.1: Propagation and purification of plasmids used to transform A. niger

Plasmid pGW635 which is a shorter version of pGW613 (Goosen et al. ref. 13) and which also contains the pyr A gene, and plasmid pGW1800, which contains the polygalacturonase II gene, are propagated in E. coli MH1 and JM109, respectively. Plasmid pGW1900 is propagated in E. coli DH5αF′. Plasmid DNA is recovered from 250 ml overnight cultures as described in Maniatis et al. (pp. 90-91; ref. 6) and in Example 3.7.

## Example 5.2: Preparation of protoplasts and transformation of the uridine auxotrophic mutant A. niger strain N593

The A. niger strain N593 (csp A, pyr A) which is a derivative of the parental strain N400 has been obtained by positive selection against the toxic analogue 5-fluoro-orotic acid like in yeast (Boeke et al. , ref. 14) in the presence of uridine as described by Goosen et al. (ref.13).

Liquid minimal medium supplemented with 0.5 % yeast extract, 0.2% casamino-acids, 10 mM uridine (Janssen Chemie) and 50 mM glucose is inoculated with $10^6$ conidiospores of A. niger N593 per ml and incubated for 20 h at 30 °C in a New Brunswick orbital shaker. Mycelium is harvested by filtration, through Miracloth, washed with iso-osmotic minimal medium (STC) of the following composition: 1.33 M sorbitol, 10 mM Tris-HCl pH 7.5, 50 mM $CaCl_2$. An amount of 1 g of mycelium is resuspended in 20 ml STC. Protoplasts are released from the mycelium within 2 h by adding 150 mg of filter-sterilized Novozym 234 (Novo Industries, Denmark) and incubating the mixture at 30 °C in a shaker at 95 rpm. The protoplasts are separated from residual mycelium by filtration using a funnel with a plug of glasswool. Then cold STC is added to a volume of 40 ml and the mixture is placed on ice for 10 min. The protoplasts are recovered by centrifugation (10 min, 2500 rpm) and the pellet is resuspended in 5 ml of STC. The protoplasts are pelleted once more and finally resuspended in 1 ml of cold STC.

For transformation 5 x $10^6$ protoplasts are taken in 200 µl which are then incubated together with 1 µg pGW635 and 20 µg of pGW1800 or pGW1900. After the addition of plasmid DNA to the protoplasts 50 µl PCT (10 mM Tris-HCl pH 7.5, 50 mM $CaCl_2$, 25 % PEG6000) is added and the incubation mixture is kept on ice for 20 min. Then another 2 ml PCT is added and the mixture is incubated for a further 5 min at room temperature. Finally, 4 ml STC are added and mixed. Aliquots of 1 ml of this final transformation solution are mixed with 4 ml liquified iso-osmotic MM top-agar stabilized by 0.95 M sucrose. The protoplast mixture is immediately plated on agar plates containing the same iso-osmotic minimal medium and these are incubated at 30 °C. Appropriate control experiments include protoplasts treated similarly without plasmid DNA and on non-stabilized minimal medium with 2.5 mM uridine.

After 3 days of growth at 30 °C well growing transformants appear which sporulate (150 transformants/µg pGW635). Besides, a large number of presumably abortive transformants appear. Approx. 300 transformants are obtained with pGW1800 and approx. 100 with pGW1900. Of each of the transformants with pGW1800 and pGW 1900 twenty colonies are picked at random, spores of individual transformants are taken and plated out separately on 50 mM glucose minimal medium to obtain single colonies which are purified once more and subsequently used to propagate spores for further transformant analysis.

Example 5.3: Selection of polyg alacturonase overproducing co-transformants of A. niger strain N593 by halo-formation on pectin containing solid media

Approximately 200 colonies transformed with pGW1800 and approximately 100 transformed with pGW1900 obtained as described in Example 5.2 have been screened for an increase in polygalacturonase activity by a colony screening procedure without preceding purification. The medium used for screening is composed out of 2 % (w/v) glucose, 0.5 % apple pectin (34.8 % degree of esterification, Obipektin, Bischoffszell) minimal medium salts and spore elements; 6 g/l $NaNO_3$; 0.2 % yeast extract; 0.2 % peptone, 0.004 % Triton X-100 and 1.2 % agar. The Petri dish is inoculated in the centre and incubated for 2 days at 30 °C. The colonies are stored overnight in the cold. Then the surface of the plate is stained by adding an overlay of 5 ml of a 0.05 % ruthenium red solution, which is incubated for 5 min while shaking. The unbound dye is then removed by washing with distilled water for another 5 min. The production of polygalacturonase under these conditions is indicated by the size of the halo formed. Approximately 50 % of the transformants thus analyzed has a polygalacturonase activity which is higher than the wild type.

Twenty colonies of pGW1800 transformants and 7 of pGW1900 transformants are picked on the basis of the size of the halo formed on pectin containing solid media and after a second screening with these positive colonies after 45 h of growth, 6 of each of the transformants are finally picked and purified as described in Example 5.2.

Example 5.4: Genomic analysis of polygalacturonase II transformed A. niger strains

Transformants obtained according to Example 5.2 or 5.3 as well as the parental wild type strain N402 are cultured on liquid minimal medium supplemented with 0.5 % yeast extract, 0.2 % casamino acids, 50 mM glucose and $NaNO_3$ (6 g/l). After 18 h of growth at 30 °C the DNA is extracted and analyzed for the presence of co-transforming plasmid. A. niger DNA is isolated as already described hereinbefore.

Digests of the chromosomal DNA (2 µg) are made at 37 °C in a reaction volume of 200 µl consisting of 50 mM Tris-HCl pH 8.0, 10 mM $MgCl_2$, 100 mM NaCl and 4 mM spermidine which has been adjusted to pH 7.5 with Tris base before its addition to the reaction mixture. 20 U of both EcoRI and SalI are added and the reaction mixture is incubated for 2 h at 37 °C. Then another 20 U of both enzymes is added and the incubation is continued for another 2 h. The reaction mixture (200 µl) is subsequently extracted with 100 µl chloroform. The DNA is subsequently precipitated from the aqueous phase by the addition of 1/10 (v/v) of a 3M sodium acetate buffer pH 5.2 and 2.5 vol of ethanol. After incubation at 4 °C for 1 h and after centrifugation, the DNA pellet is air-dried and dissolved in 20 µl sample buffer. DNA of the transformants and of A. niger N402, digested by EcoRI/SalI, is analyzed by hybridization of Southern blots as described hereinbefore with the 1.2 kbp BamHI/BglII fragment of pGW1800 as PGII probe.

The cotransformation frequency is found to be over 75 %. A number of 9 transformants has been analyzed by Southern blotting. The probe hybridizes in N402, with a large genomic fragment. In the genomic blots of the transformants which contain pGW1800 sequences, a 4.1 kbp EcoRI-Sal insert is found. Depending on the copy number the intensity of this band varies. In the cases analyzed the genomic fragment which represent the wild type gene, is always found, indicating heterologous integration. The analysis of the polygalacturonase II production in A. niger transformant named hereinafter N593/pGW1800-27 is given as a detailed example (Example 6). From a DNA dilution series, the copy number is estimated to be at least in the order of 20. Besides the wild type hybridizing fragment and the 4.1 kbp fragment a few other minor hybridizing fragments are found in this strain which represent border fragments of type II integration events or rearrangements.

Exampel 5.5: Polygalacturonase II production by transformed A. niger strains and by A. niger N402

Twenty of the pGW1800 transformants described in Example 5.2 and six of the pGW1800 transformants described in Example 5.3 are grown on a medium which consists of a minimal salt medium to which urea (4.2 g/l), 1 % (w/v) apple pectin (d.e. 61.2 %) and 1 % (w/v) wheat bran is added. The cultures are grown for 43 h at 30 °C using a Gallenkamp orbital shaker at 250 rpm and culture filtrates are obtained. The mycelium is removed by filtration over Miracloth using a Büchner funnel.

The PGII content of these samples is examined by Western blotting, which is performed using the alkaline phosphatase detection method according to the Biorad instruction manual. On the basis of the signals on the Western blots, amongst the 20 transformants randomly obtained in Example 5.2, 70 % produces significantly more polygalacturonase II than A. niger N402. The transformants selected on the basis of the halo-size using the plate-screening method (see Example 5.3) all produce significantly more enzyme than A. niger N402.

The A. niger strain N402, and three of the transformants, named N593/pGW1800-27, N593/pGW1800-30 and N593/pGW1800-37, are selected for measuring polygalacturonase activity. The latter three strains are from the set of pGW1800 transformants obtained in Example 5.2.

N593/pGW1800-30 and N593/pGW1800-37 both contain multiple copies of pGW1800, but less copies than N593/pGW1800-27. The strains are grown on a 1 % pectin medium (d.e. 61.2 %) to which 1 % (w/v) dried and milled sugar beet pulp is added, using the conditions as described above. In order to remove reducing sugars and inhibitors of PG activity, PGII is partially purified after the fermentation using cross-linked alginate. 1 ml of culture filtrate is diluted with 1 ml 20 mM sodium acetate buffer pH 4.2 and is then loaded on a small column containing a bedvolume of 2 ml cross-linked alginate (5.2 ml/g) equilibrated in this buffer. The column is subsequently washed with 4 ml of the sodium acetate buffer and next the PGII activity is pulsed from the column, using 4 ml of 20 mM sodium acetate buffer pH 4.2 to which 1 M NaCl has been added. The PG activity in the eluate is then determined as described (ref. 2) and from the value obtained the PG activity in the culture filtrate is calculated.

The following PG activities have been calculated for the culture filtrates obtained 20 h after inoculation: 2.2, 5.6, 5.8 and 10.8 units/ml for the strains N402, N593/pGW1800-30, N593/pGW1800-37 and N593/pGW1800-27, respectively. For the culture filtrates obtained after 40 h these values are, in the same order, 2.8, 13.7, 16.4 and 30.9 units/ml, respectively.

It thus appears that pGW1800 can be successfully used to transform A. niger to produce a much higher PG activity.

Example 5.6: Polygalacturonase I production by transformed A. niger strains and by A. niger N402 and N593.

Seventeen of the pGW1900 transformants described in Example 5.2. and six of the pGW1900 transformants described in Example 5.3. as well as A. niger N402 and a $pyr^+$ transformant of N593 are grown on a medium which consists of a minimal salt medium to which urea (4.2 g/l.), 1% (w/v) apple pectin (d.e. 61.2%) and 1% sugar beet pulp is added. The cultures are grown for 64 h at 30 °C using a Gallenkamp orbital shaker at 250 rpm and in between samples of the culture filtrate are taken after 20 h and 39 h. The PG I content of these samples is examined as the PGII content in Example 5.5 by Western blotting.

On the basis of the signals on Western blots amongst the 23 transformants tested, 65% produces significantly more PG I than A. niger N402. The transformants selected on the basis of the halo-size all produce more PGI than A. niger N402 and four out of six transformants are high producers. Activity measurements indicate that in this medium the activity after 20 h is higher than after 39 h. Taking twelve different PGI transformed A. niger strains the activity varies between 2.8 and 7 U/ml whereas the untransformed control strain N402 and a A. niger N593/pGW635 transformant produce 0.5 and 0.8 U/ml respectively. This activity is at least the result of wild type levels of both PGI and PGII whereas the increase in the transformants is the result of more PGI expression.

Example 6: Isolation, purification and characterization of polygalacturonase II from the PGII overproducing A. niger transformant N593/pGW1800-27

Example 6.1: Culture conditions to prepare polygalacturonase II

The A. niger PGII transformant N593/pGW1800-27 described in Example 5.4. is grown on complete medium in Petri-dishes for 3-4 days at 30 °C to produce conidia. The conidia are harvested in 5 ml sterile saline containing 0.005 % Tween 80 per plate. The spore suspension is agitated on a Griffin shaker for 20 min., and after counting the spores, $10^6$ spores/ml are added to 1 l siliconized Erlenmeyer flasks containing 300 ml sterile growth medium. This medium is a minimal medium containing 70 mM ammonium chloride as the nitrogen source and 1 % (w/v) apple pectin (degree of esterification 61.2 %) and 1 % (w/v) sugar beet pulp as carbon sources. The mycelium is grown for 43 h at 30 °C using a Gallenkamp orbital shaker at 200 rpm. After growth the mycelium is removed by filtration through Miracloth using a Büchner funnel. The culture filtrate is adjusted to pH 4.2 using 1 N NaOH. 0.02 % sodium azide is added in all further steps to prevent microbial growth.

Example 6.2: Purification of polygalacturonase II obtained from the A. niger transformant N593/pGW1800-27

The culture filtrate (approx. 2.5 l) is applied to a crosslinked alginate column (2.5 x 25 cm) equilibrated with 20 mM sodium acetate buffer pH 4.2. After loading the column is eluted consecutively with one bed volume of the equilibration buffer, one bed volume of 20 mM sodium acetate buffer pH 5.6, a 1200 ml linear salt gradient (0-0.5 M NaCl) in the previous buffer, and finally 275 ml of a 1M NaCl solution in the same buffer. Fractions of 6 ml each are collected and tested for their enzymatic activity as described in Example 1.1. The central part of the active fractions is pooled and dialyzed thrice against a 20 mM bis Tris HCl buffer pH 6.0. The final enzyme solution (475 ml) is then applied to a DEAE-Sepharose Fast Flow column (Pharmacia) equilibrated with the same buffer. After washing, a NaCl gradient is applied in the same buffer and polygalacturonase activity becomes eluted at approx. 100 mM sodium chloride. The active fractions are analyzed by SDS-polyacrylamide gel electrophoresis. These fractions which have a high PGII content are pooled (54 ml), dialyzed against 20 mM bis Tris HCl buffer pH 6.0 and further purified on a Mono Q column (Pharmacia) equilibrated in the same buffer. After loading, the enzyme is eluted by applying a salt gradient (0-1 M NaCl).

The enzyme is arbitrarily collected in two different pools A and B corresponding with the 0.2-0.26 M part of the sodium chloride gradient and with the 0.26-0.34 M part of gradient. Both fractions contain a single protein band upon SDS-polyacrylamide gel electrophoresis at the same position as PGII.

Example 6.3: Amino acid sequence determination of the N-terminal part of polygalacturonase II

200 µg of PGII from the combined pools A and B purified according to Example 6.2 is dialyzed three times against 1 l Millipore filtered distilled water and lyophilized. The amino acid sequence is determined as described in Example 1.3, using an Applied Biosystems model 470A gas phase protein sequencer. The following N-terminal amino acid sequence is determined for the enzyme:

```
Position:     1                    5
Amino acid: asp - ser - X - thr - phe - thr - thr - ala -
Position:          10                        15
Amino acid: ala - ala - ala - lys - ala - gly - lys - ala -
Position:                 20
Amino acid: lys - X - ser - thr - ile
```

The cysteine residues in the protein have not been modified and are thus not detected. They are likely to occur at position 3(X) and at position 18(X) of the sequence (see Example 1.3). The last three amino acid residues are only detected at low levels. The sequence obtained for this pure enzyme corresponds exactly with the amino acid sequence of the N-terminus of PGII deduced from the nucleotide sequence of the PGII gene (see Example 4 and Figure 4, formula II). The corresponding nucleotide sequence also shows cysteine residues at the position expected, namely at residues 3 and 18. This sequence also corresponds with the sequence determined for the 5 kDa cyanogen bromide fragment (see Example 1.3) which therefore corresponds with the N-terminus of the protein.

Example 6.4: Properties of polygalacturonase II purified from transformant N593/pGW1800-27

The enzyme purified according to Example 6.2 has been compared with the enzyme purified from Rapidase as described in Example 1.1. Both enzymes have an identical apparent molecular mass of 38 kDa on SDS-polyacrylamide gels and they react identically with polyclonal antibodies raised against the purified PGII. A monoclonal antibody which reacts only with a continuous epitope of PGII, and not with PGI, IIIA, IIIB or IV, also reacts with the enzyme purified according to Example 5.2. Upon isoelectric focusing PGII produced and purified according to Examples 6.1 and 6.2 shows a sharp band at a pI value identical to that of PGII (pI = 5.2). Due to the microheterogeneity of PCII, a number of other bands have been observed with lower pI values when using a pH gradient from pH 3-7 or from pH 3-10. Similar patterns are obtained when using PGII produced according t Example 6.1.

Example 7: Detection and isolation of sequences related to the polygalacturonase II gene

Example 7.1: Detection of sequences related to the polygalacturonase II gene

DNA is isolated from A. niger NW756 using the procedure which is described above. The DNA is restricted with the enzymes BamHI, EcoRI and the combination of these enzymes as described in Example 5.4. The DNA fragments are then separated on an 0.6 % agarose gel and they are subsequently transferred to a nitrocellulose membrane as described in Example 3.7. The baked membrane is prehybridized for 2 h at 60°C in prewarmed hybridization buffer, which consists of 1 % BSA (Boehringer), 1 mM EDTA, 0.5 M sodium phosphate pH 7.2 (added from a 1 M stock which is composed of 89.0 g of $Na_2HPO_4 \cdot 2H_2O$ and 4 ml of 85 % $H_3PO_4$ per litre) and 7 % SDS, as described by Church and Gilbert (ref. 38), and to which sheared and denatured herring sperm DNA is freshly added to a concentration of 0.1 mg/ml. Then the nick-translated 1.2 kbp BamHI-BglII fragment of pGW1800, which is prepared as described in Example 3.7, is added and the hybridization is allowed to proceed for 44 h at 60°C while gently shaking. The membrane is then washed two times in prewarmed (60°C) hybridization buffer (without herring sperm DNA) for 10 min. Subsequently the membrane is washed two times for 20 min at 60°C in a prewarmed buffer which consists of 5xSSC, 0.1 % SDS and 0.1 % $Na_4P_2O_7 \cdot 10H_2O$. The membrane is then dried and exposed to Kodak XAR5 film for 3 days at-60°C.

Under these conditions unspecific hybridization is not observed, which follows from the absence of a significant smear of hybridizing DNA fragments and, in addition, from the absence of hybridization of the probe with the lambda size markers. In each lane several hybridizing bands are observed, whereas in each lane one band is much more intense than other bands. These intense bands corrrespond with a 3.3 kbp fragment in the BamHI digest, a fragment larger than 20 kbp in the EcoRI digest and a 3.3 kbp fragment in the BamHI/EcoRI double digest. The bands with a lower intensity correspond with fragments of 17, 9.5, 6.4 and 2.0 kbp in the BamHI digest, fragments of 15, 12, 6.5 and 4.8 kbp in the EcoRI digest and fragments of 7.6, 6.4, 4.0, 3.7 and 2.0 kbp in the BamHI/EcoRI double digest.

From the fact that specific hybridization signals are observed, it follows that the PGII gene of A. niger N400 can be used to detect specific sequences in the DNA obtained from A. niger NW756. The fragments which give strong hybridization signals are assumed to carry the PGII gene. In principle, a weakly hybridizing fragment can arise if a restriction enzyme cleaves the DNA within and close to the end of the region homologous to the probe used. However, this cannot fully explain the number of hybridizing large fragments which is observed in the present example. It follows that specific DNA sequences have been detected, which share homology with the PGII gene, but are not identical to it. These DNA sequences are assumed to be different PG genes, which is in agreement with the fact that PGs have homology at the protein level (see Example 1.4).

Example 7.2: Isolation of genes related to the polygalacturonase II gene

Approximately 1 x $10^4$ phages from the A. niger N400 library are plated on 5 plates using E. coli LE392 and three nitrocellulose replicas are made from each plate, as described in Example 3.4, whereas the third filter is incubated on top of a plate for 5 min. The first and the second filter of each plate are treated using conditions, which are referred to as "heterologous". The third filter is subjected to stringent conditions, which are referred to as "homologous". Prehybridization, hybridization and washing of the filters are performed at 60 °C under heterologous conditions and at 68 °C under homologous conditions. After baking, the filters are wetted in 3xSSC and then prehybridized for two h in prewarmed hybridization buffer, which consists of 10x Denhardt's (see Example 3.4), 50 mM Tris-HCl pH 7.5, 20 mM EDTA pH 8.0, 1 M NaCl, 0.5% SDS and 0.1 % sodium pyrophosphate, to which 0.1 mg/ml sheared and denatured herring sperm DNA is freshly added. The filters are then transferred, one at a time, to a flask containing 50 ml of hybridization buffer, including herring sperm DNA, to which the nick-translated 1.2 kbp BamHI-BglII fragment of pGW1800 has previously been added. The hybridization is allowed to proceed for 40 h and then the filters are washed, either using homologous or heterologous conditions. The homologous washing conditions are as follows: the filters are washed twice for 0.5 h in 2xSSC, 0.1 % SDS and 0.1 % sodium pyrophosphate and subsequently twice for 0.5 h in 0.2xSSC, 0.1 % SDS and 0.1 % sodium pyrophosphate. The heterologous conditions are as follows: the filters are washed twice for 0.5 h in hybridization buffer and then for 0.5 h in 4xSSC, 0.1 % SDS and 0.1 % SDS and 0.1 % sodium pyrophosphate and finally in 2xSSC, 0.1 % SDS and 0.1 % sodium pyrophosphate. The filters are dried on the air and exposed to Kodak-XAR5 film for 3 days at -60 °C, using intensifying screens. The phages that give positive signals are recovered as described in Example 3.4.

On the filters subjected to the homologous conditions 7 positive signals have been obtained, which signals are also present at the corresponding position on the filters subjected to the heterologous conditions. These signals are regarded to result from recombinant lambda phages which carry the PGII gene. Except for these signals, 30 positive signals have been obtained on the filters subjected to the heterologous conditions and these signals are present at the corresponding position on both filters. The signal strength amongst these 30 signals varies. The majority of these signals is regarded to result from phages which contain a PG gene different from the PGII gene.

The genomic A. niger N400 library is screened for the second time. Plaque lifts are prepared as described above. Except for the temperature used, the hybridization is carried out as described above. Hybridization and washing is at 62 °C for one of the two filters obtained from a plate, whereas the other filter is hybridized and washed at 68 °C. Following the hybridization, the filters are washed using high SSC (HSSC) conditions, either at 62 °C or at 68 °C. The HSSC conditions are as follows: the filters are washed two times for 5 minutes in 4 x SSC, then the filters are washed twice for 0.5 h in 4 x SSC and subsequently twice for 0.5 h in 2 x SSC, whereas the SSC solutions used also contain 0.1% SDS and 0.1% sodium pyrophosphate.

On the filters incubated at 68 °C ("homologous" hybridization condition) five strong positive signals have been obtained which signals are also present at the corresponding position on the filters incubated at 62 °C. Restriction analysis of one of these phages according to Example 3.5 indicates that this phage contains an insert on which the gene encoding PGII (pgaII) is located. These five phages are therefore considered to all contain pga II. In addition, on the filters incubated at 62 °C ("heterologous" hybridization condition) 17 positive signals are present, which are not or only weakly hybridizing on the corresponding filters incubated at 68 °C . The signal strength amongst these 17 signals varies.

16 phages from the first screening and 10 phages from the second screening, which phages do not contain pga II, are selected for further characterization. These phages are purified using the conditions used for their initial detection. The phages characterized are the phages listed in Table VII and eight additional phages (numbers 2,3,7,31,33,40,41,42). The phages numbered 1 to 30 have been obtained in the first screening of the library, whereas the phages with higher numbers have been obtained in the second screening.

In order to discriminate between those phages which contain the polygalacturonase 1 gene and those which contain other polygalacturonase II related sequences, plaque lifts of the selected phages are hybridized with the previously nick-translated 1.8 kbp Hind III fragment of pGW1900 (Example 3.9.). Hybridization is at 62° C, whereas washing is also at 62° C while using a series of SSC buffers which buffers all contain 0.1% SDS and 0.1% sodium pyrophosphate (4 x SSC, 2 x SSC, 1 x SSC, 0.5 x SSC, 0.2 x SSC and 0.1 x SSC; incubation is for half an hour per buffer). Using these conditions, phages 2, 3, 7, 40, 41 and 42 give strong hybridization signals, comparable to the signal obtained with phage PG 1-λ7, which latter phage carries the pga I gene (Example 3.6.). Restriction analysis of DNA obtained from one of these phages also indicates that this phage contains an insert on which the pga I gene is located. These six phages are therefore considered to all contain the pga I gene.The other phages analyzed (1, 8, 31, 33, 35, 36, 37, 38, 43) give only a weak hybridization signal, if any, and these phages, therefore, do not contain the pga I gene.

In order to find out which phages contain identical fragments, DNA is isolated from the phages indicated in Table VII as described in Example 3.5, and digested as described in Example 3.6 and the resulting fragments are subsequently characterized by Southern blot analysis. For the analysis of Hinf I and Hinc II digests, the agarose content of the gels is increased to 1% (w/v). Southern blots are hybridized at 60° C with the previously nick-translated 1.2 kbp Bam HI/Eco RI fragment from pGW1803, whereas washing is also at 60° C, using the HSSC conditions described above. The restriction enzymes which are found to be useful to make an initial classification for these phages are a combination of Bam HI and Bgl II, and Hinf I and Hinc II, which latter enzymes are used separately. Hinc II and Hinf I usually generate small restriction fragments, which, in the present case, minimizes the chance that the resulting hybridizing fragments contain DNA derived from the EMBL4 vector, in addition to the pga II related sequence.

Table VII: Classification of PG II related λ phages based on the size of the hybridizing λ DNA fragments (kbp) obtained after digestion of DNA isolated from these phages with HincII, HinfI and BglII/BamHI and sometimes with SalI using the 1.2 kbp BamHI/EcoRI fragment of pGW1803 as a probe.

| Restriction enzyme | Classes of λ-phages[a] | | | | | | |
|---|---|---|---|---|---|---|---|
| | A | B | C[a] | D | E | F | G[b] |
| | λ Phages belonging to the respective classes: | | | | | | |
| | 9,10,43 | 4,35,36 | 1,16,20,37 | 8,11,12 | 6,38 | 5 | 17,27 |
| | Fragment length (kbp) | | | | | | |
| HincII | 1.25 | 2.4 | 1.75 | 1.3; 0.4 | 1.3;0.78 | 2.3 | |
| HinfI | 0.7; 0.3 | 1.1 | 1.28 | 1.8 | 0.65 | 0.6 | |
| BglII/BamHI | 7.2; 0.56 (λ-10) | 3.1 (λ-4) | 8.7 (λ-20) | 1.7 | 4.1 (λ-6) | ≥23 | 3.0 (λ-17) 4.9 (λ-27) |
| SalI | 6.9 | n.d. | 6.8 (λ-16; λ-20) | 1.3; 0.5 | n.d. | | |

[a] Phages with number 16 and 20 also share a 1.64 KpnI fragment and a 1.38 kbp HincII/KpnI fragment.

[b] Class G phages only weakly hybridize with PG II under non-stringent conditions.
n.d. digestion not done.

From the data presented in Table VII it is clear that for most phage classes several representative phages have been isolated independently. Given the fact that only a selection of all the hybridizing signals has been taken in the initial screening, the number of λ-phages found for each class (class A: 3; B: 3; C: 4; D: 3; E: 2) indicates that different genes are found at a similar frequency. In the case of the PG I gene this number is higher (6 out of 26 phages analyzed) whereas PG II containing phages are found at the same frequency (12 out of 59 phages analyzed). The class G is more weakly hybridizing with both probes derived either from the PG I gene or from the PG II gene. Besides a hybridizing fragment of 3.0 and 4.9 kb in the phages with number 17 and 27, respectively, several non-hybridizing fragments which are identical in both phages and which are derived from the inserts are detected in the Bgl II/Bam HI digest viz . a 5.6 kbp, a 5.1 kbp, a 1.45 kbp and a 0.57 kbp fragment. Also Hinc II and Hin fI digests of both phages are almost the same.

The phages isolated in this Example are renamed as follows:

| Class: | Number: | Name: |
|---|---|---|
| A | 9 | λPG-A9 |
| | 10 | λPG-A10 |
| | 43 | λPG-A43 |
| B | 4 | λPG-B4 |
| | 35 | λPG-B35 |
| | 36 | λPG-B36 |
| C | 1 | λPG-C1 |
| | 16 | λPG-C16 |
| | 20 | λPG-C20 |
| | 37 | λPG-C37 |
| D | 8 | λPG-D8 |
| | 11 | λPG-D11 |
| | 12 | λPG-D12 |
| E | 6 | λPG-E6 |
| | 38 | λPG-E38 |
| F | 5 | λPG-F5 |
| G | 17 | λPG-G17 |
| | 27 | λPG-G27 |
| not determined | 31 | λPG-X31 |
| not determined | 33 | λPG-Y33 |

Example 7.3: Subcloning of the PGC gene (pgaC): the construction of pGW1910

Phage λPG-C20 (Example 7.2; phage 20, class C) is digested with BglII, and the resulting fragments are separated on an agarose gel. The gel slices that contain the hybridizing 7.8 kbp BglII are recovered and the fragment is then isolated as described (Example 3.6). The BglII fragment is ligated into the BamHI digested and CIP (calf intestine phosphatase) treated pUC9. The ligation reaction mixture is used to transform E. coli JM109 (Example 3.6). Several of the resulting white colonies are streaked on YT agar supplemented with ampicillin. After growing overnight the colonies are adsorbed onto a nitrocellulose filter. The colonies on the filter are then lysed and the liberated DNA is fixed by baking as described by Maniatis et al. (ref. 6; p. 314). The baked filters are wetted in 2xSSC and they are gently rubbed with a gloved hand to remove the bacterial cell debris. The filters are then hybridized with the 1.2 kbp BamHI-EcoRI fragment of pGW1803, using the heterologous conditions described in Example 7.2 (60 °C, washes with 2xSSC). A positive clone is chosen and plasmid DNA is purified as described hereinbefore. The plasmid DNA is then used for restriction analysis to construct a physical map.

In this way a new plasmid has been constructed. pGW1910 (Fig. 7) is the 7.8 kbp BglII fragment of phage λPG-C20 inserted in the BamHI site of the vector pUC9. This subclone comprises hybridizing fragments of phage λPG-C20, e.g. the 1.1. kbp SmaI and 1.6 kbp KpnI fragments. From the location of these fragments on the physical map of pGW1910 it is inferred that this plasmid contains the complete PGC gene (pga C).

Example 8: Expression of human hybrid interferon BDBB under the control of the PGII promoter

Example 8.1: Construction of plasmid pGII-IFN AM119 precursor (Figure 4)

Plasmid pGW1800 is digested with EcoRI and treated with $T_4$ polymerase as below. Religation of this DNA and transformation of E. coli DH5αF′ allows the isolation of a plasmid pGW1800-E, which is the same as pGW1800, except that the EcoRI cleavage site is deleted.

Plasmid pGW1800-E is digested with BglII and treated with bacterial alkaline phosphatase (BRL) in the presence of 50 mM Tris-HCl pH 8.0 and 50 mM NaCl for 1 h at 65 °C. The alkaline phosphatase is inactivated by digestion with proteinase K (Boehringer Mannheim) and phenol extracting. The DNA is subsequently ethanol precipitated, dried and redissolved in water. Then the sticky ends are filled in with $T_4$ DNA polymerase as below.

Plasmid pJDB207-IFN AM119 (EP 205 404) is digested with HindIII and ClaI. The sticky ends of these linear fragments are filled in with $T_4$ DNA polymerase (Boehringer Mannheim) in the presence of 0.1 mM each of dCTP, dGTP, dATP and dTTP plus 67 mM Tris-HCl pH 7.5, 6.7 mM $MgCl_2$, 16.7 mM $(NH_4)_2SO_4$ and 5 mM DTT for 30 min at 37° C. The reaction is stopped by heating at 65° C for 5 min. The fragments are separated in a 0.8 % low gelling temperature agarose (BioRad) gel and the 1 kbp fragment, which comprises the IFN AM119 coding region, was excised and the DNA purified on an Elutip D (Schleicher & Schüll) column, and ethanol precipitated (Schmitt & Lohen, ref. 34).

100 ng of IFN AM119 fragment and the prepared pGW1800-E vector are ligated together in 5 µl of 20 mM Tris-HCl pH7.5, 10 mM $MgCl_2$, 10mM DTT, 1 mM ATP and 1 unit of $T_4$ DNA ligase (Boehringer Mannheim) for 2 h at room temperature. This mixture is transformed into competent E. coli DH5αF′ cells. Ampicillin resistant transformants are screened by restriction digestion of their plasmid DNA to identify those that carry the plasmid pGII-IFN AM119 precursor.

Example 8.2: The generation of precise pGII-IFN AM119 and pGIIss-IFN AM119 fusions using PCR (Figures 4 and 5)

The PCR method followed is as described by R.M. Horton et al. (ref. 35) and is outlined in figure 5.

pGW1800 is linearised, by XbaI digestion, and precipitated with ethanol. After resuspension in water, 100 ng of this DNA is subjected to amplification by polymerase chain reaction (PCR) using oligonucleotides A and B (Figure 5) in an automated thermal cycler for 25 cycles (each consisting of 1 min at 94° C, 2 min at 40° C, and 3 min at 72° C) followed by 10 min at 72° C. 100pM of each oligonucleotide and 0.5 µl of Taq polymerase (Perkin Elmer Cetus) are used for each reaction in a volume of 100 µl using the reaction buffer recommended by the supplier. This gives DNA 1 (Figure 5).

Similarly treated pGW1800 with oligonucleotide A and C gives DNA 2.

Similarly pJDB207-IFN AM119 linearised with BamHI and subjected to PCR with either oligonucleotides D and F or oligonucleotides E and F gives DNA 3 or DNA 4 respectively.

These reaction mixtures are precipitated with ethanol, redissolved in water and an aliquot is checked on a gel to determine the concentration of the DNA fragments in the mixtures.

Using the same conditions as above DNA 1 and DNA 4 are subjected to PCR with oligonucleotides A and F to give DNA 5 and DNA 2 and DNA 3 with the same oligonucleotides to give DNA 6 (Figure 5).

The DNA 5 starts with a BamHI site and ends with an EcoRI site and includes a perfect in frame fusion of the original starting methionine codon linked to a PGII promoter fragment with the coding region for mature hybrid interferon BDBB.

The BamHI-EcoRI fragment of DNA 5 is ligated into BamHI-EcoRI cut plasmid pGII-IFN AM119 precursor constructed in example 8.1 to create plasmid pGII-IFN AM119.

Likewise the BamHI-EcoRI fragment of DNA 6, which contains a perfect in frame fusion of the PGII signal sequence linked to a PGII promoter fragment with the coding region for mature hybrid interferon BDBB, was inserted in pGII-IFN AM119 precursor to generate plasmid pGIIss-IFN AM119.

Example 8.3: Cotransformation of Aspergillus niger mutant An8 with pCG59D7 and pGIIss-IFN or pGII-IFN

The uridine anxotrophic mutant An8 (≙ DSM 3917, disclosed in EP 278 355) is cotransformed with plasmid pCG59D7 and pGIIss-IFN or pGII-IFN AM119 to yield uridine prototrophs.

Conidial spores of A. niger An8 are grown for 4 days at 28° C in complete medium until fully sporulated. 2x$10^8$ conidiospores are used to inoculate 200 ml minimal medium supplemented 1 g/l arginine and uridine.

After 20 hours growth at 28° C and 180 rpm. the mycelium is harvested by filtration through Miracloth, washed twice with 10 ml 0.8 M KCl, 50 mM $CaCl_2$ and resuspended in 20 ml 0.8 M KCl, 50 mM $CaCl_2$, 0.5 mg/ml Novozym 234 (Novo Industries). The mixture is incubated in a shaking waterbath (30° C, 50 rpm.) until sufficient protoplasts are released (detected microscopically after 90-120 min). The protoplast suspension is filtered through a glass wool plug in a funnel to remove mycelial debris. The protoplasts are pelleted by mild centrifugation (10 min, 2000 rpm) at room temperature and washed twice with 10 ml 0.8 M KCl, 50 mM $CaCl_2$. The protoplasts are finally resuspended in 200-500 µl 0.8 M KCl, 50 mM $CaCl_2$ to give a concentration of 1x $10^8$/ml.

For transformation a 200 µl aliquot of the protoplast suspension is incubated with 5 µg of pCG59D7 and 50 µg pGIIss-IFN AM119 or pGII-IFN AM119 DNA, 50 µl PCT (10 mM Tris-HCl pH 7.5, 50 mM $CaCl_2$, 25 % PEG 6000). The incubation mixture is kept on ice for 20 min, another 2 ml of PCT are added and the mixture incubated for further 5 min at room temperature. 4 ml 0.8 M KCl, 50 mM $CaCl_2$ are added and 1 ml aliquots of the final transformation solution are mixed with liquified minimal agar medium (Minimal medium + 1 g/l arginine + 10 g/l Bacto-Agar (Difco)), stabilised with 0.8 M KCl. The mixtures are immediately poured on agar plates of the same medium and incubated at 30 ° C.

After 2-3 days of growth at 28 ° C, stable transformants appear as vigorously growing and sporulating colonies on a background growth of many hundred small, presumably abortive, transformants.

Example 8.4: Expression of the Hybrid-Interferon BDBB gene under the control of the PGII promoter

Transformants of the cotransformation experiment (Example 8.3.) are picked and analysed for interferon expression. Interferon activity is determined according to the procedure of Armstrong (J.A. Armstrong, Appl. Microbiol. 21 , 732(1971)) using human CCL-23 cells and vesicular stomatitis virus (VSV) as the challenge virus.

Conidial spores from transformants are individually precultured into 50 ml of a preculture medium (Pectin Slow Set L (Unipectin, SA, Redon, France) 3 g/l, $NH_4Cl$ 2 g/l, $KH_2PO_4$ 0.5 g/l, NaCl 0.5 g/l, $Mg_2SO_4 \cdot 7H_2O$ 0.5 g/l, $Ca_2SO_4 \cdot 2H_2O$ 0.5 g/l, pH 7.0, 1 % arginine). The preculture is incubated for 72 hours at 250 rpm and 28 ° C. 10 % of the preculture is used to inoculate 50 ml of main culture medium (Soybean fluor 20 g/l, pectin Slow Set 5 g/l, 1 % arginine). The culture is grown up for 72-96 hours at 250 rpm and 28 ° C.

At various times (every 20 hours) samples are taken, the cells are pelleted by centrifugation and broken by freezedrying and dry grinding. Supernatant and cell extracts are both tested for inteferon activity as described (supra). The bulk of the interferon activity is found secreted into the medium in transformants carrying pGIIss-IFN AM119 while in transformants carrying pGII-IFN AM119 it is mainly in the cell extract.

Example 9: Polygalacturonase I and polygalacturonase II production by transformed A. nidulans strains

Example 9.1: Propagation and purification of plasmids used to transform A. nidulans G191 E.coli MH1

pGW635 is propagated in E. coli MH1, pGW1800 in JM109 and PGW1800 in DH5αF'.

Example 9.2: Preparation of protoplasts and transformation of the uridine auxotrophic A. nidulans mutant

The A. nidulans mutant strain G191 (pyrG, pabaA1, fwA1, uaY9) has been described by Ballance and Turner, 1985 (ref. 27).

Mycelium is grown at 37 ° C and further as described for A. niger (see Example 5.2.) except that 2 mg p-aminobenzoate is added per liter medium. Protoplasts are prepared following the procedure described for A. niger . For transformation 5 x $10^6$ protoplasts are taken in 200 µl STC, which are then incubated together with 1 µg pGW635 and 20 µg pGW1800 or 25 µg pGW1800. Plates are incubated for 3 days at 37 ° C.

Approx. 50 transformants/µg pGW635 are obtained in the cotransformation experiment, and in each experiment 20 transformants are further purified on 50 mM glucose minimal medium. These strains are subsequently used to propagate spores for further analysis.

Example 9.3: Western blot analysis of polygalacturonase I and II production by transformed A. nidulans strains

The cotransformants obtained according to Example 5.2. using pGW1800 as the cotransforming plasmid are grown submerged in 75 ml minimal medium using 2 mg/l p-aminobenzoate, 7.5 g/l $NH_4NO_3$ as nitrogen source and 1% (w/v) apple pectin (d.e. 61.2 %) plus 1% (w/v) sugar beet pulp as carbon source starting with $10^5$ conidiospores per ml. The Gallenkamp orbital shaker is used at 250 rpm and the growth temperature is maintained at 30 ° C.

Samples are taken after 43 and 68 h of growth, centrifuged and the supernatant is dialyzed overnight at 4°C against 5 mM sodium phosphate buffer pH 6.5 containing 0.02 % (w/v) sodium azide. Analysis of the PG II content of these samples is examined by Western blotting (see page 69) using polyclonal or monoclonal antibodies. The A. nidulans samples used as control do not contain PG II cross-reactive material as tested with monoclonal antibodies.

The twenty transformants analyzed all produce polygalacturonase II although the amount varies amongst the different transformants. Besides a major band of the expected molecular weight (38 kDa) several minor bands of lower molecular weight, most likely representing degradation products, are observed. The transformant which produces the most enzyme is A. nidulans G191/pGW1800-13. The polygalacturonase activity of this transformant using different growth media was compared with the activity of the recipient strain as shown in Table VIII.

Table VIII: Polygalacturonase activities of culture filtrates of the A. nidulans transformant G191/pGW1800-13 and of A.Anidulans G191. The strains are grown by inoculating $10^6$ spores/ml at 30°C in minimal medium using N- and C-sources as defined and high phosphate (15 g $KH_2PO_4$/liter) plus 0.1% yeast extract.

| Strain | C-Source | N-Source | Fermentation time | Polygalacturonase activity (U/ml) |
|---|---|---|---|---|
| G191 | 3 % glucose | 1 % $NH_4Cl$ | 40 | n.d. |
| G191/pGW635-1 | 3 % glucose | 1 % $NH_4Cl$ | 40 | n.d. |
| G191/pGW1800-13 | 3 % D-glucose | 1 % $NH_4Cl$ | 40 | 120 |
| G191 | 1 % pectin + 1 % sugar beet pulp | 1 % $NH_4Cl$ | 40 | n.d. |
| G191/pGW1800-13 | 1 % pectin + 1 % sugar beet pulp | 1 % $NH_4Cl$ | 40 | 130 |

n.d. = not detectable

Since transformant G191/pGW1800-13 synthesizes high levels of PG II in the absence of pectic substances, the PGII production of the other A. nidulans G191/pGW1800 transformants obtained is also further analyzed. To this end 19 transformants as well as G191/pGW635-1 are grown in a medium which consists of minimal medium salts, using 0.4% $NH_4Cl$ as the nitrogen source and 5% glucose as the carbon source, with phosphate added at a high concentration (15 g/l $KH_2PO_4$). Culture filtrates are obtained after 46 and 69 hrs and they are subsequently analyzed by Western blotting and probing with the monoclonal antibody, without prior concentration and dialysis of the culture filtrates. At least 17 out of the 19 A. nidulans G191/pGW1800 transformants synthesize PG II on the glucose medium, whereas no signal is obtained with A. nidulans G191/pGW635-1. The fact that clear signals are obtained implies a high expression level in comparison with A. niger N402 grown under polygalacturonase inducing conditions such as described in Example 5.5, since only weak PG II signals are obtained on Western blots of culture filtrates of the nontransformed strain, when using nonconcentrated filtrates and the monoclonal antibody. The PG II production A. niger G191/pGW1800-13 and six other G191/pGW1800 transformants using the medium as described above, but with 1% beet pulp and 1% pectin as the carbon sources instead of 5% glucose, is also analyzed as described above. In contrast to A. nidulans G191/pGW1800-13 these six transformants produce much more PG II on the medium with pectin and beet pulp, whereas again no signal is found with A. nidulans G191/pGW635-1.

The cotransformants obtained according to Example 9.2. using pGW1900 as the cotransforming plasmid are grown on two different media. The first medium consists of a high phosphate minimal salt medium to which $NH_4Cl$ (4.0 g/l), 1% (w/v) apple pectin (d.e. 61.2%) and 1% (w/v) sugar beet pulp are added, whereas some transformants are also grown in this medium with urea (4.2 g/l) as the nitrogen source instead of $NH_4Cl$. The difference with the previous low phosphate minimal medium (page 28) is that

15 g $KH_2PO_4$ per liter is used instead of 1.5 g. The second medium also consists of a high phosphate minimal salt medium to which 0.1% yeast extract, $NH_4Cl$ (10 g/l) and 3% (w/v) glucose are added as nitrogen and carbon source. In both media 2 mg/l p-aminobenzoate is added. The cultures are grown for 42 h at 30° C using a Gallenkamp orbital shaker at 250 rpm and samples of the culture filtrate are taken after 20 h and 42 h. In Western blots of A. nidulans G191, grown under similar conditions as the transformants, no or very little protein is detected which cross-reacts with polyclonal antibody raised against purified polygalacturonase I. In the culture filtrate of 75% of the transformants PG I is detected at significant levels.

Using $NH_4Cl$ instead of urea and high phosphate also in complex media, considerably reduces the proteolytic degradation of the polygalacturonase I which is produced. On the basis of Western blotting results and activity measurements transformant G191 pGW1900-6 is estimated to produce over 1 g of enzyme per liter as indicated in Table VI since the specific activity of PG I is 550 U/mg (Kester and Visser, 1990).

Table VI: Polygalacturonase activities of culture filtrates of the A. nidulans transformant G191/pGW1900-6 and of the control strain G191/pGW635-1. The strains are grown by inoculating $10^6$ spores/ml at 30°C minimal medium using N- and C-sources as indicated and high phosphate (15 g $KH_2PO_4$/l.)

| Strain | C-Source | N-Source | Fermentation time | Polygalacturonase activity (U/ml) |
|---|---|---|---|---|
| G191/pGW635-1 | 3 % glucose | 0.4 % $NH_4Cl$ | 64 | n.d. |
| G191/pGW635-1 | 1 % pectin + 1 % sugar beet pulp | 0.4 % $NH_4Cl$ | 49 | n.d. |
| G191/pGW635-1 | 1 % pectin + 1 % sugar beet pulp | 0.4 % urea | 26 | n.d. |
| G691/pGW1900-6 | 3 % glucose | 0.4 % $NH_4Cl$ | 64 | 5 |
| G191/pGW1900-6 | 1 % pectin + 1 % sugar beet pulp | 0.4 % $NH_4Cl$ | 22 | 17 |
| | 1 % pectin + 1 % sugar beet pulp | 0.4 % $NH_4Cl$ | 26 | 125 |
| | 1 % pectin + 1 % sugar beet pulp | 0.4 % $NH_4Cl$ | 49 | 570 |
| G191/pGW1900-6 | 1 % pectin + 1 % sugar beet pulp | 0.4 % urea | 22 | 125 |
| | 1 % pectin + 1 % sugar beet pulp | 0.4 % urea | 26 | 225 |
| | 1 % pectin + 1 % sugar beet pulp | 0.4 % urea | 49 | 140 |

n.d. = not detectable

The pga I and pga II gene coding for polygalacturonase I and II respectively are not expressed in A.niger on glucose containing media, even in multicopy transformants with a high gene dose. The expression of these A. niger genes in A. nidulans transformants like in strains G191/pGW1900-6 and G191/pGW1900-10 and like in strains G191/pGW1800-13 and G191/pGW1800-20, respectively, indicates however that in A. nidulans catabolite repression of these genes is circumvented. This implies different specificities in the catabolite repression system amongst these different Aspergillus species. This can be exploited to express a particular A. niger polygalacturonase gene in a different host like A. nidulans under conditions which avoid expression of polygalacturonases of the host itself. The polygalacturonase enzymes thus obtained are practically pure.

Example 10: Isolation, purification and characterization of polygalacturonase II from the A.nidulans transformant G191/pGW1800-13

Example 10.1: Production of PG II using transformant G191/pGW1800-13.

The A. nidulans transformant G191/pGW1800-13 is grown in 100 ml cultures on a 1% sugar beet pulp and 1% pectin medium composed as described in Example 5.3 except that 15 g/l $KH_2PO_4$ is used instead of 1.5 g/l. Similarly, this transformant is also grown both at low and high phosphate levels on minimal medium using 5% or 1% glucose as carbon source. These media do not lead to any detectable polygalacturonase II in A. niger N402 whereas the strain A. nidulans G191 itself is unable to make PG II as described in Example 9.3.

In samples of culture filtrates of the A. nidulans transformant G 19 1/pGW1800-13 taken after 72 h using 5% glucose and high phosphate, approx. 860 U/ml. PG activity are found whereas using low phosphate this amount is 550 U/ml. The reduced levels observed when using 1% glucose and high phosphate are mainly due to an increase in protein turnover when the carbon source becomes exhausted after 48 h. In the case of 1% glucose and low phosphate, the production level remains low throughout the whole culture period. The amount of protein obtained per liter culture filtrate using 5% glucose and high phosphate approximates 1 g per liter. SDS polyacrylamide gel electrophoresis indicates that the culture filtrate of the transformant contains one protein band with a molecular mass corresponding with that of A. niger PG II.

Example 10.2: Isolation and purification of PG II from culture filtrate of A. nidulans transformant G191/pGW1800-13.

The culture filtrate (approx. 2 l) which has a pH of approx. 4.9 is diluted 5-fold with distilled $H_2O$ and then taken to pH 6.0 using 2 N NaOH. To concentrate the enzyme, the solution is loaded on 600 ml Sephadex DEAE-A50 pre-equilibrated in 20 mM potassium phosphate buffer pH 6.0 which is contained in a Büchner funnel to allow high flow rates. All the enzyme activity is adsorbed to the ionic exchange material and collected in 300 ml by elution with 1 M NaCl in the same buffer (recovery: 65%). The enzyme solution is then dialyzed against 10 mM Bis Tris buffer pH 6.0 and loaded on a Sepharose DEAE Fast Flow column (60 ml bed volume) equilibrated in the same buffer.
The enzyme activity is eluted by applying a 0-0.5 M NaCl gradient in buffer at approx. 0.15M NaCl (total recovery: 57%).

Example 10.3: Properties of PG II purified from the culture filtrate of the A. nidulans transformant A. nidulans G191/pGW1800-13.

PG II purified according to Example 10.2 has been compared with the enzyme purified from Rapidase as described in Example 1.1 (PG II-case). Both enzymes have an identical apparent molecular mass of 38 kDa on SDS-polyacrylamide gels and they react identically with polyclonal antibodies and with the monoclonal antibody (see Example 6.4). Upon isoelectric focusing PG II produced by the A. nidulans transformant and purified according to Example 10.2 has much less microheterogeneity than the enzyme produced by A. niger since besides the major band (pI 5.2) only one minor band is found.

The kinetic properties of both enzymes have been compared by measuring at 25 °C in 0.075 M sodium acetate pH 4.2 polygalacturonase activity varying the polygalacturonate concentration between 0.2 and 1 mg/ml using a modified ferricyanide test. For the A. niger PG II from Rapidase the $V_{max}$ is 2760 U/mg of protein with a $K_m$ of 0.8 mg/ml polygalacturonate (USB). For the PG II derived from the transformant the $V_{max}$ value is slightly higher viz . 3480 U/mg of protein whereas the $K_m$ is identical. Finally PG II purified from the A. nidulans transformant has been treated with cyanogen bromide according to Example 1.3 (PG II-case). This leads to an identical pattern of fragments as observed for PG II purified from Rapidase.

Example 11: The expression of the pgaII gene of A. niger NW756 in transformed A. niger N593 strains

pGW1756 (Example 3.10) is used to transform A. niger N593, using pGW635 as the selective vector see (Example 5.2). Eight transformants picked at random are purified and are used for further analysis. They are grown on a liquid minimal salt medium to which ammonium chloride (4.0 g/l), 1 % pectin (d.e.

61.2) and 1 % dried and milled sugar beet pulp is added, using the conditions as described in Example 5.5. PGII overproduction into the culture filtrates of the transformants is shown by Western blotting, using PGII specific antibodies. Two of the PGII over-producing transformants (N593/pGW1756-6 and N593/pGW1756-7) are selected and these strains and the control strain N402 (see Example 5.5) are grown again using the conditions described above. Culture filtrates are obtained at 44 h after inoculation and the polygalacturonase activity in the crude filtrates is determined as described (ref. 2), whereas the enzyme reaction mixture is buffered at pH 4.8 using 50 mM sodium acetate.

The results also show that the pga II gene on pGW1756 is functional and that this plasmid can be used to transform A. niger to overexpress polygalacturonase activity.

Example 12: Polygalacturonasec Production by transformed A. nidulans strains

Plasmid pGW1910 carrying the pga C gene of A. niger N400 (Example 7.3) is used as the cotransforming plasmid in an experiment designed to transform A. nidulans G191 to uridine prototrophy, as described in Example 9.2. Ten of the resulting transformants are purified and they are subsequently used to propagate spores for further analysis. These strains and G191/pGW635-1 are grown in a liquid minimal salts medium supplemented with 2 mg/ml p-aminobenzoate, 4.0 g/l $NH_4Cl$ as the nitrogen source, 1 % sugar beet pulp and 1 % pectin as the carbon sources, with phosphate added at a high concentration (15 g/l $KH_2PO_4$). Inoculation is at $10^6$ spores/ml and incubation is at 30 ° C in a Gallenkamp orbital shaker at 250 rpm. Culture filtrates are obtained at 65 h after inoculation and the filtrates are subsequently analyzed, without prior purification or concentration. Western blots are incubated with a mixture of the antibodies raised against PGI and PGII (Example 1.2). Activity measurements (ref. 2) are performed in a 50 mM sodium acetate buffer pH 4.8 containing 0.25 % polygalacturonic acid (USB).

On the basis of the Western blotting most of the transformants obtained produce high amounts of polygalacturonaseC (PGC). This result is confirmed by the measuring of PG activity in the supernatant of the transformants and of a control strain (A. nidulans G191 transformed with pGW635).

Example 13: The expression of the A. niger pgaA and pgaD genes in transformed A. nidulans strains

In addition to the pga I, pga II and pga C genes other members of the polygalacturonase gene family of A. niger (Example 7.2) are used to transform A. nidulans . In the present Example clones in phage λ are used, instead of subclones thereof in a plasmid vector.

Phage λ DNA is prepared from plate lysates as described in Example 3.5 and is then extracted once more with phenol and chloroform. For a cotransformation experiment approx. 20 µg of λ DNA is used, and the DNA is in about equal amounts obtained from 2 phages of the same class (e.g. λPG-A10 in combination with λPG-A43 or λPG-D8 in combination with λPG-D11). The cotransformation of A. nidulans G191 is performed as described in Example 9.2, whereas the controls now include cotransformations with pGW1900 and λPGI-7, separatedly. Using λ phage DNA prepared as described as above, the transformation frequencies are lower as compared with plasmid DNA purified by banding in cesium chloride gradients.

The relevant transformants are purified and analyzed as described for the pGW1910 transformants in Example 12. In this case the transformants are also grown on a medium containing 3 % glucose as the carbon source (high phosphate, 0.1 % yeast extract, 1 % ammonium chloride).

Transformants A. nidulans G191/lambdaA-1 and G191/lambdaD-1 have been obtained using the class PG-A and PG-D phages, respectively, mentioned above. The polygalacturonase activities in the culture filtrates of the beet pulp/pectin medium at 65 h after inoculation were significantly higher than in the control strain G191/pGW635-1. The pga II related sequences present in the class A and D phages thus encode proteins with polygalacturonase activity. On the basis of the Western blots it is concluded that these gene products, polygalacturonaseA and polygalacturonaseD, respectively, migrate with a similar electrophoretic mobility as PGI.

A. nidulans G191/lambdaA-1 produces polygalacturonaseA also on the medium with glucose as the carbon source.

Example 14: The expression of hybrid interferon in transformed A. nidulans strains

For a cotransformation experiment approx. 20 µg of plasmid DNA pGII-IFN AM119 or pGIIss-IFN

AM119 is used. The cotransformation of A. nidulans G191 is performed as described in Example 9.2.

The relevant transformants are purified and analyzed as described for the transformants in Example 8.4. In this case the transformants are also grown on a medium containing 3% glucose as the carbon source (high phosphate, 0.1% yeast extract, 1% ammonium chloride).

At various times (every 20 hours) samples are taken, the cells are pelleted by centrifugation and broken by freezedrying and dry grindling. Supernatant and cell extracts are both tested for interferon activity as described (supra). The bulk of the interferon activity is found secreted into the medium in transformants carrying pGIIss-IFN AM119 while in transformants carrying pGII-IFN AM119 it is mainly in the cell extract which indicates that A. nidulans produces IFN from pGII-IFN AM119 or pGIIss-IFN AM119 when glucose is used as sole carbon source.

Example 15: Maceration properties of polygalacturonase II and polygalacturonase I.

Cell separation of cucumber tissue is used to assay the macerating activity of A. niger polygalacturonases. A cucumber fruit is purchased in a local grocery and the surface is subsequently decontaminated by wiping with a tissue soaked in ethanol or by floating for one hour in Javel water (grocery grade, diluted to 0.5% sodium hypochlorite). The cucumber is then sliced and the soft inner part of the slices is removed. Two of the resulting slices (approx. 12 g) are placed in a 100 ml Erlenmeyer flask containing 25 ml of a maceration buffer to which polygalacturonase had been previously added, or not. The subsequent incubation is for 24 hours with gentle unidirectional shaking in a water bath thermostated at 30° C. The flasks are then visually inspected. Complete maceration meets the following criteria: (i) the peel of the cucumber fruit is essentially free of adhering tissue, as indicated by macroscopic examination; ii) the maceration buffer has become highly turbid; iii) there is a positive correlation between cell separation and the high turbidity of the macerating buffer, as indicated by a high proportion of loose cells which appear to be intact as judged by microscopic visualization.

Polygalacturonase II and polygalacturonase I prepared as described in Example 1 are tested in different buffers. Maceration buffer A (MBA) is 50 mM sodium acetate pH 4.8. Maceration buffer B (MBB) is a phosphate-citrate buffer, (Ishii (1976), Phytopathology 66 , pp.281-289). MBB is prepared by mixing 0.1 M citric acid and 0.1 M $Na_2HPO_4$ solutions until the desired pH value, 4.5, is reached, and then an equal volume of distilled water is added and subsequently bovine serum albumin is added to a final concentration of 10 mg/25 ml, using a concentrated solution of 10 mg/ml.

Cucumber tissue is completely macerated within 24 hrs, using 1 ug of polygalacturonase II in MBA. In this buffer the cucumber tissue is not macerated in the absence of polygalacturonase or in the presence of 1 ug polygalacturonase I. Maceration of cucumber tissue in MBB is tested in the absence of polygalacturonase or in the presence of 10 ug polygalacturonase I or polygalacturonase II, respectively. Again, maceration is not observed in the absence of enzyme or in the presence of polygalacturonase I, whereas polygalacturonase II completely macerates the cucumber tissue. PGII prepared according to Example 10 has also macerating properties.

## REFERENCES

1. Rombouts, F.M., Geraeds, C.C.J.M., Visser, J. and Pilnik, W. Purification of various pectic enzymes on crosslinked polyuronides in T.C.J. Gribnau, J. Visser and R.F.J. Nivard (Eds) Affinity chromatography and Related Techniques pp. 255-260, 1982, Elsevier, Amsterdam.
2. Rozie, H., Somers, W., Bonte, A., Visser, J., van't Riet, K. and Rombouts, F.M. (1988) Biotechn. Appl. Biochem. 10 , 346-358.
3. Robyt, J.F., Ackerman, R.J. and Keng, J.G. (1973), Anal. Biochem. 45 , 517-524.
4. Matsudarai, P. (1987), J. Biol. Chem. 262 , 10035-10038.
5. Amons, R. (1987), Vapor-phase modification of sulfhydryl groups in proteins, FEBS Letters 212 , 68-72.
6. Maniatis, T., Fritsch, E.F. and Sambrook, J., Molecular cloning, a laboratory manual, Cold Spring Harbor Laboratory (1982).
7. Benton, W.D. and Davis, R.W. (1977), Science 196 , 180-182.
8. Caruthers, M.H. Chemical and Enzymatic Synthesis of Gene Fragments: a laboratory manual, Verlag Chemie (1982).
9. Frischauf, A.M., Lehrach, H., Poustra, A. and Murray, N. (1983), J. Mol. Biol. 170 , 827-842.

10. Dente, L. and Cortese, R. (1987), Methods in Enzymology, vol. 155 pp. 111-119.
11. BRL, M13 cloning/dideoxy sequencing instruction manual.
12. Holms, D.S. and Quigley, M. (1981), Anal. Biochem. 114 , 193.
13. Goosen, T., Bloemheuvel, G., Gysler, Ch., de Bie, D.A., van den Broek, H.W.J. and Swart, K. (1987), Current Genetics 11 , 499-503.
14. Boeke, J.D., Lacroute, F. and Fink. G.R. (1984), Mol. Gen. Genet. 197 , 345-346.
15. Rombouts, F.M. and Pilnik, W. (1980). Pectic Enzymes. In: A.H. Rose (ed.) Microbial Enzymes and Bioconversion. Economic Microbiology Vol.5. Academic Press. pp. 227-282.
16. Ohtsuka, E., Matsuki, S., Ikehara, M. Takahashi, Y. and Matsubara, K. (1985), J. Biol. Chem. 260 , 2605-2608.
17. Pharmacia T7 sequencing TM Kit Instruction Manual, pp 1-35.
18. Yelton, M.M., Hamer, J.E. and Timberlake, W.E. (1984) Proc. Natl. Acad. Sci. USA 81 , 1470-1474.
19. Karn, J., Brenner, S., Barneff, L. and Cesareni, G. (1980) Proc. Natl. Acad. Sci. USA 77 , 5172-5176.
20. Slater, R.J. (1984) in: Methods in Molecular Biology vol. 2 Ed. J.M. Walker, The Humana Press Inc.
21. Zissler, J. et al. (1971) in: The Bacteriophage Lambda, Cold Spring Harbor Labs, New York, A.D. Hersley editor.
22. Dente, L., Cesareni, G. and Cortese, R. (1983) Nucl. Acids Res. 11 , 1645-1655.
23. Dente, L., Sollazzo, M., Baldari, C., G. Cesareni and R. Cortese in: DNA Cloning vol. 1. a practical approach ed. D.M. Glover, IRL Press, Oxford 1985.
24. Norrander, J., Kempe, T. and Messing, J. (1983) Gene 26 , 101-106.
25. Boel E., Hansen M.T., Hjort I., Hoegh I., Fiil N.P. (1984), EMBO J. 3 , 1581-1585.
26. Mount S.M. (1982), Nucleic Acids Res. 10 , 459-472.
27. Ballance D.J. and Turner G. (1985), Gene 36 , 321-331.
28. Birnboim H.C. & Doly J. (1979), Nucleic Acids Res. 7 , 1513-1523.
29. Yanisch-Perron, C., Vieira, J. and Messing, J. (1985) Gene 33, pp. 103-119.
30. Messing, J., Crea, R. and Seeburg, P.H. Nucleic Acids Res. 9, 309-321 (1981).
31. Renard, C.M.G.C., Voragen, A.G.J., Schols, H.A., Searle-van Leeuwen, M.J.F., Thibault, J.F., Pilnik, W., (1989). Apple protopectin: preliminary study of enzymatic extraction. In: J.P. Roozen, F.M. Rombouts, A.G.J. Voragen (eds.): Food Science: Basic Research for Technological Progress. PUDOC, Wageningen, The Netherlands.
32. Voragen, A.G.J. (1989): Food enzymes: prospects and limitations. In: J.P. Roozen, F.M. Rombouts, A.G.J. Voragen (eds.): Food Science: Basic Research for Technological Progress. PUDOC, Wageningen, The Netherlands.
33. Beldman, G., Rombouts, F.M., Voragen, A.G.J., Pilnik, W. (1984) Enzyme Microb. Technol. 6 , 503-507.
34. Schmitt, J.J., and Cohen, B.N. (1983) Quantitative Isolation of restriction fragments from low-melting agarose by Elutip-a affinity chromatography. Analytical Biochemistry 133 , 462-4.
35. Horton, R.M., Hunt, H.D., Ho, S.N., Pullen, J.K., and Pease, L.R. (1989). Engineering hybrid genes without the use of restriction enzymes: gene splicing by overlap extension. Gene 77 , 61-68.
36. J.H.A.A. Uitzetter (1982). Studies on carbon metabolism in wild type and mutants of Aspergillus nidulans . PhD Thesis, Agricultural University Wageningen, The Netherlands.
37. Bowen, B., Steinberg J., Laemmli, U.K. and Weintraub H. (1980). Nucl. Acids Res 8 , 1-20.
38. Church, G.M. and Gilbert, W. (1984) Proc. Natl. Acad. Sci. USA 81 , 1991-1995.
39. von Heijne, G. (1986), Nucleic Acids Research 14 , 4683-4690.
40. Ausubel, F.M., Brent, R., Kingston, R.E., Moore, D.D., Smith, J.A., Seidman, J.G. and Struhl, K. (eds.) (1987), Current Protocols in Molecular Biology, John Wiley and Sons, New York, Chichester, Brisbane, Toronto, Singapore.
41. Rambosek, J. and Leach, J. (1987), Recombinant DNA in filamentous fungi: progress and prospects, CRC Critical Reviews in Biotechnology 6 , 357-393.
42. Goosen, T., Van Engelenburg, F., Debets, F., Swart, K., Bos, K., and Van den Broek, H.W.J. (1989) Mol.Gen.Genet. 219 , 282-288.
43. Shapiro, M.B., and P. Senapathy (1987) Nucleic Acids Res. 15 , 7155-7174.
44. K.S. Poutanen (1990). Communicated at the 199$^{th}$ ACS Meeting, Boston Mass., April 22-27, 1990; Abstract 90 of the Cellulose, Paper and Textile Division.

Deposited microorganisms:

Following microorganisms and phages are deposited with the Deutsche Sammlung von Mikroorganismen und Zellkulturen (DSM), Mascheroder Weg 16, D-3300 Braunschweig:

| Microorganisms: | DSM-No.: | Date of Deposition: |
|---|---|---|
| Escherichia coli JM109/pGW1800 | 5505 | August 30, 1989 |
| Escherichia coli DH5αF'/pGW1900 | 5866 | March 21, 1990 |
| Escherichia coli DH5aF'/pGW 1756 | 5942 | May 18, 1990 |
| Escherichia coli DH5αF'/pGW1910 | 5943 | May 18, 1990 |
| Escherichia coli DH5αF'/pGW**1911** | 5944 | May 18, 1990 |
| Escherichia coli LE 392 | 5941 | May 18, 1990 |
| Phages: | | all May 18, 1990 |
| λPG-A9 | 5945 | |
| λPG-A10 | 5951 | |
| λPG-A43 | 5964 | |
| λPG-B4 | 5949 | |
| λPG-B35 | 5960 | |
| λPG-B36 | 5961 | |
| λPG-C1 | 5948 | |
| λPG-C16 | 5954 | |
| λPG-C20 | 5956 | |
| λPG-C37 | 5962 | |
| λPG-D8 | 5947 | |
| λPG-D11 | 5952 | |
| λPG-D12 | 5953 | |
| λPG-E6 | 5946 | |
| λPG-E38 | 5963 | |
| λPG-F5 | 5950 | |
| λPG-G17 | 5955 | |
| λPG-G27 | 5957 | |
| λPG-X31 | 5958 | |
| λPG-Y33 | 5959 | |

Sequence Listing

SEQ ID NO. 1

SEQUENCE TYPE: Nucleotide with corresponding polypeptide
SEQUENCE LENGTH: 2495 base pairs
STRANDEDNESS: double
TOPOLOGY: linear
MOLECULE TYPE: genomic
ORIGINAL SOURCE ORGANISM: Aspergillus niger N400
IMMEDIATE EXPERIMENTAL SOURCE: Plasmid pGW1900 (DSM 5866)
FEATURES: Gene pgal encoding A. niger N400 prepro-polygalacturonase I
from 1 to 909: promoter region
from 901 to 963: coding region for PGI signal peptide
from 901 to 1002: coding region for PGI prepro-sequence
from 1003 to 2127: coding region for mature PGI
from 1138 to 1189: intron
from 1610 to 1671: intron
from 2138 to 2130: stop codon from 2131 to 2495: 3′ non coding region, part of transcriptional terminator region

```
GAATTCGGAA GAATGTCTGC GTTCGTGCGC ACAACGACGT    40
TCCTAAAATT TATCCGATGA TCAACCGAGG AACCAGGGTC    80
GAACCCCTGA AAGGAATCTC GCCGAAAGGA TCAATCATAT   120
TGATTCGCGG ACCCTGTAAA GTGCCCTAAA GGGTCTTTGC   160
CACTCGATAG TGGGATCGGC ACTGGCAGTT TCTAGTCTCG   200
TCAGTGCGGC CATTTCCGAC CGATCCGTAG TGCTGGTGGG   240
TGAGTCCCAG AGCAGTCTAT ATCGATATCA TCACCAAATT   280
CCAAGGACCG TGGGATGACG AGTATGCATT GGCACTGAGA   320
ATTGCCGAGA TCATGCCCCC GTCCTGGAGG TGCATCCATC   360
AAGCATGTTG CGACAGAGTG AAACAGCGTG GATAAGCCGG   400
ATGGGGCAGA TGGGGGAAGA GAGCGAGACA CGGCATCAGC   440
AGTGGAGATG CCGAGAAGTG CCGAGAGCCG CCGATGCTTC   480
```

```
GACCTTCAGC CCAAGCGTCA ATCCATGCCT TCTTGGGTCA        520
GGGTTGGCCG GACTGTAAGC CCGTTAGCGT CTGAGATACG        560
CCGGATGACA CGAACCTTGG TGCTTACCAA TGCTGTGATG        600
CATGCAGTGC CGGCGGTATC CCTGGCTGAA GCGCCCATCG        640
CCGTTAGAGA TTGATACCCG GGTGGATCGG AGGGTCCCCT        680
TGGTCTTTCC ACCAATAATG GAGGTATCTC ACTGCTTTGT        720
TCAGGAACAG AAGCTTAGCA TGGACCAGTC TTTCACGTAT        760
AAAACTCTCC AGGACTTCCG TCGTTGAGAT GCTCTATCCA        800
ACAACACCTC ACTCTTCCAC TCCTTGTTCT TTCTTTCTGT        840
TGACCAGACC CCATCCATTC TTTTGGCCGA AACCTGTTCT        880
GTTGACCAGA ACCTATACCA ACAATCACC ATG CAC TCT        918
                                Met His Ser
                                     1

TAC CAG CTT CTT GGC CTG GCC GCT GTC GGC TCC CTC    954
Tyr Gln Leu Leu Gly Leu Ala Ala Val Gly Ser Leu
      5                 10                  15

GTC TCT GCC GCC CCC GCT CCT TCT CGC GTC TCC GAG    990
Val Ser Ala Ala Pro Ala Pro Ser Arg Val Ser Glu
                 20                  25

TTC GCT AAG AAG GCC TCT ACC TGC ACC TTC ACC TCT   1026
Phe Ala Lys Lys Ala Ser Thr Cys Thr Phe Thr Ser
         30                  35

GCC TCT GAG GCC AGC GAG AGC ATC TCC AGC TGC TCC   1022
Ala Ser Glu Ala Ser Glu Ser Ile Ser Ser Cys Ser
 40                  45                  50

GAT GTT GTC CTG AGC AGC ATC GAG GTC CCC GCT GGC   1098
Asp Val Val Leu Ser Ser Ile Glu Val Pro Ala Gly
             55                  60
```

```
GAG ACC CTC GAC CTG TCC GAT GCT GCT GAT GGC TCC   1134
Glu Thr Leu Asp Leu Ser Asp Ala Ala Asp Gly Ser
    65                  70                  75

ACC GTATGTGCTC TCAGCCGTCT TCCATCCTGG CTATCACGCT   1177
Thr

AACACCATCT AG ATC ACC TTC GAG GGC ACC ACT TCC TTC 1216
              Ile Thr Phe Glu Gly Thr Thr Ser Phe
                           80                  85

GGA TAC AAG GAA TGG AAG GGC CCC CTG ATC CGC TTC   1252
Gly Tyr Lys Glu Trp Lys Gly Pro Leu Ile Arg Phe
                 90                  95

GGT GGT AAG GAT CTG ACT GTC ACC ATG GCC GAC GGC   1288
Gly Gly Lys Asp Leu Thr Val Thr Met Ala Asp Gly
        100                 105

GCT GTC ATC GAC GGT GAC GGT TCC CGC TGG TGG GAC   1324
Ala Val Ile Asp Gly Asp Gly Ser Arg Trp Trp Asp
110                 115                 120

AGC AAG GGT ACC AAC GGT GGC AAG ACC AAG CCC AAG   1360
Ser Lys Gly Thr Asn Gly Gly Lys Thr Lys Pro Lys
            125                 130

TTC ATG TAC ATC CAC GAT GTT GAG GAC TCG ACC TTC   1396
Phe Met Tyr Ile His Asp Val Glu Asp Ser Thr Phe
    135                 140                 145

AAG GGC ATC AAC ATC AAG AAC ACT CCC GTC CAG GCC   1432
Lys Gly Ile Asn Ile Lys Asn Thr Pro Val Gln Ala
                150                 155
```

```
ATC AGT GTC CAG GCT ACC AAC GTC CAC CTG AAC GAC      1468
Ile Ser Val Gln Ala Thr Asn Val His Leu Asn Asp
        160                     165

TTC ACC ATC GAC AAC TCC GAC GGT GAT GAC AAC GGT      1504
Phe Thr Ile Asp Asn Ser Asp Gly Asp Asp Asn Gly
170                 175                     180

GGC CAC AAC ACC GAC GGT TTC GAC ATC AGC GAG TCT      1540
Gly His Asn Thr Asp Gly Phe Asp Ile Ser Glu Ser
            185                     190

ACC GGT GTC TAC ATC AGC GGT GCT ACC GTC AAG AAC      1576
Thr Gly Val Tyr Ile Ser Gly Ala Thr Val Lys Asn
    195                     200                 205

CAG GAC GAC TGC ATT GCC ATC AAC TCT GGC GAG          1609
Gln Asp Asp Cys Ile Ala Ile Asn Ser Gly Glu
                210                     215

GCACGATATC CCTATTCCAC TATCATTCCT TCCATTCATA          1649

TCGCTAACAA TCAAACCCAC AG AGC ATC TCT TTC ACC         1686
                         Ser Ile Ser Phe Thr
                                     220

GGC GGT ACC TGC TCC GGT GGC CAC GGT CTC TCC ATC      1722
Gly Gly Thr Cys Ser Gly Gly His Gly Leu Ser Ile
            225                     230

GGC TCT GTC GGT GGC CGT GAT GAC AAC ACC GTC AAG      1758
Gly Ser Val Gly Gly Arg Asp Asp Asn Thr Val Lys
    235                     240                 245
```

AAC GTG ACC ATC TCC GAC TCC ACT GTC AGC AAC TCC 1794
Asn Val Thr Ile Ser Asp Ser Thr Val Ser Asn Ser
250 255

GCC AAC GGT GTC CGC ATC AAG ACC ATC TAC AAG GAG 1830
Ala Asn Gly Val Arg Ile Lys Thr Ile Tyr Lys Glu
260 265

ACC GGT GAT GTC AGC GAG ATC ACC TAC TCT AAC ATC 1866
Thr Gly Asp Val Ser Glu Ile Thr Tyr Ser Asn Ile
270 275 280

CAG CTC TCC GGA ATC ACC GAC TAC GGT ATC GTC ATC 1902
Gln Leu Ser Gly Ile Thr Asp Tyr Gly Ile Val Ile
285 290

GAG CAG GAC TAC GAG AAC GGC TCT CCC ACC GGC ACC 1938
Glu Gln Asp Tyr Glu Asn Gly Ser Pro Thr Gly Thr
295 300 305

CCC TCC ACC GGT ATC CCC ATC ACT GAT GTC ACC GTT 1974
Pro Ser Thr Gly Ile Pro Ile Thr Asp Val Thr Val
310 315

GAC GGT GTC ACC GGT ACT CTC GAG GAT GAC GCC ACC 2010
Asp Gly Val Thr Gly Thr Leu Glu Asp Asp Ala Thr
320 325

CAG GTC TAC ATT CTC TGC GGT GAC GGC TCT TGC TCT 2046
Gln Val Tyr Ile Leu Cys Gly Asp Gly Ser Cys Ser
330 335 340

GAC TGG ACC TGG TCC GGT GTT GAC CTC TCT GGT GGC 2082
Asp Trp Thr Trp Ser Gly Val Asp Leu Ser Gly Gly
345 350

```
AAG ACC AGC GAT AAA TGC GAG AAC GTT CCT TCC GGT    2118
Lys Thr Ser Asp Lys Cys Glu Asn Val Pro Ser Gly
    355                 360                 365

GCT TCT TGC TAA ATCGTTCCTC CGGATGCGAG GCAACGTCTG  2160
Ala Ser Cys
        368

TAGGACGTCT GGTTGTATAT ATCGATCCAC ACTTGACATG       2200
TACTAGTTAG GTGGTTTTAC TGCCATAGTG TTAGTGAATA       2240
ATAGGAGCTT TGCTCAATAT GTGAATTTGT AGCAGAAGTA       2280
AATGAGTAGA CTGATAGAGG TAATGATGAA AGATAGAATT       2320
TAATACCAGG TGATGAAGTT AATAACGGGG TGAATTAGGG       2360
CGCGTAGGCT TAGGTATATA AGTTGTCATC CCTCACACTC       2400
GAAATCTCTT CCTCTTTCTT ATATCTTCCA ACCTCTGAAC       2440
CACCAGTTGC CTCCACAGAC TAACAAGATT CTTCTATATC       2480
GATGCTTGAT CTCAA                                  2495
```

SEQ ID NO. 2

SEQUENCE TYPE: Nucleotide with corresponding polypeptide
SEQUENCE LENGTH: 3031 base pairs
STRANDEDNESS: double
TOPOLOGY: linear
MOLECULE TYPE: genomic
ORIGINAL SOURCE ORGANISM: Aspergillus niger N400
IMMEDIATE EXPERIMENTAL SOURCE: Plasmid pGW1800 (DSM 5505)
FEATURES: Gene pgaII encoding A. niger N400 prepro-polygalacturonase II
from 1 to 1356: promoter region
from 1357 to 1419: coding region for PGII signal peptide
from 1357 to 1437: coding region for PGII prepro-sequence
from 1438 to 2494: coding region for mature PGII
from 1987 to 2038: intron
from 2495 to 2497: stop codon
from 2498 to 3031: 3' non-coding region, part of transcriptional terminator region

```
TCTAGAAGCA CTTCCCGTGG TGGAGAACAT AACACGCTGG    40
TTAATCTGCT TGACGAACGC GTTGTCTCCA GGTCAGAGCA    80
TGACTCCCTC TCAATATAGC TGGCTTCTAC AACCTTGAAA   120
ATAGGGGTAA TGGAGCGAAT CCGGCTAGAA GAGGGTAATG   160
AACGGGACAA TATGGTTCAC CTGCCATGAT GGAATAGTGT   200
GCGCACTGGT GGCTTCCGAT GCACCAGACT CAGACATAAA   240
CCGTTTTGCG CCAGCACTAC TTTGTCCTCC TGCCCCGAGG   280
AATGAATCCA ATAGGAGGAA TGTCGATATT CGCGCTCGAT   320
AGCATCACTC GGATATCGAG AATACCGGGC TGAGGTTGTT   360
GCAGGTGGAA AGATTTGCTC AATCTGTATC GAAAAAACGT   400
AACTTGATGA ATGACCTTTC AACAACTAAC CGCCCCTATC   440
CACTGCTGTG ACCAGTATAG TCCACCGGAT TCGCTCACCG   480
TTAGCCAGTG GCTTCCTCCT TTTTCGTCAT CTTTTCCTCT   520
TCTTGACTGG CCCCACTCCA GGTTGCTTGT TGCCGGCAAG   560
```

```
CTTTCGACCC TGATTAGCGA GCTTTGCGCA CTATTCCTGA        600
TTTAGCTGTC GAAGGAACGG ATGGACGCTG CGCTAGTTTC        640
ATCTCGAATC TCTGATATTA ACATTGGCTA CAGACTAGCC        680
GATTACGCTC CGGAATCTGG CACACAGGGA GTTTATGGAC        720
CGTTCATTGG TGGAACTAGC AAAGCACCAT AATTGCCGCG        760
GACCCTGCAT TTCAGGCTGC AGCCTTACTA GGATTAGTAT        800
AGCTGTCTTC TCGTACTCGT ACACTGCAGT ACCGGCCTGG        840
AATCTCGGGA TCAACGATGG CATCCGCTTC CTCCAGGACT        880
TACGGCTGCT TGTGAAGGCC AATTATAGTG TTGCTTGTTT        920
ACGTGCCAAT TCAGCGGTAC ATACAAACGC TTCTCTATCT        960
TGCCCTTTTT GACAATAGGT TTACCCTCGG GAGCGGAGCT       1000
TTGCCTTCTT TCCACCGACA TGCGCATCCG TTCCATCACC       1040
CGCGGAACCC GTCGGCTGAT CAGCCACGCA CGGCTGGTAT       1080
AAATTAATCG GCCACTCATG TCGAACTGAG GTTCACGGGA       1120
AAACGCAATA TTTGAGACAA CACCTCAATA TGAAACGAGG       1160
ATCCAGGGTC CTACATTTCC TCCAGGGGCT GTCGGCAGTT       1200
ATGAACTTTT CGACCGGAAA AGATTCGCAA TAGTCGTGAG       1240
TATAAGAACC TCGTACCTGC TCACACTGAT GTCTACTTGC       1280
TCATCATTCC ACACTCATTC AAAATCTTAC CAACAACACT       1320
CCTTCTGTCA TTCTTTTCTA TTGTTAACAA TTAATC ATG CAC  1362
                                        Met His
                                         1

TCG TTT GCT TCT CTT CTC GCC TAC GGC CTG GTC GCC  1398
Ser Phe Ala Ser Leu Leu Ala Tyr Gly Leu Val Ala
         5                  0

GGC GCC ACC TTC GCT TCT GCC TCT CCT ATC GAA GCT  1434
Gly Ala Thr Phe Ala Ser Ala Ser Pro Ile Glu Ala
 15                  20                  25

CGA GAC AGC TGC ACG TTC ACC ACC GCT GCC GCT GCT  1470
Arg Asp Ser Cys Thr Phe Thr Thr Ala Ala Ala Ala
             30                  35
```

```
AAA GCG GGC AAG GCG AAA TGC TCT ACT ATC ACC CTT  1506
Lys Ala Gly Lys Ala Lys Cys Ser Thr Ile Thr Leu
     40                  45                  50

AAC AAC ATC GAA GTT CCA GCT GGA ACC ACC CTC GAC  1542
Asn Asn Ile Glu Val Pro Ala Gly Thr Thr Leu Asp
                 55                  60

CTG ACC GGT CTC ACC AGC GGT ACC AAG GTC ATC TTC  1578
Leu Thr Gly Leu Thr Ser Gly Thr Lys Val Ile Phe
         65                  70

GAG GGC ACC ACG ACC TTC CAG TAC GAA GAA TGG GCA  1614
Glu Gly Thr Thr Thr Phe Gln Tyr Glu Glu Trp Ala
 75                  80                  85

GGC CCC TTG ATC TCC ATG AGT GGC GAA CAT ATC ACC  1650
Gly Pro Leu Ile Ser Met Ser Gly Glu His Ile Thr
             90                  95

GTC ACT GGT GCC TCC GGC CAC CTC ATC AAT TGC GAT  1686
Val Thr Gly Ala Ser Gly His Leu Ile Asn Cys Asp
    100                 105                 110

GGT GCG CGC TGG TGG GAT GGC AAG GGA ACC AGC GGA  1722
Gly Ala Arg Trp Trp Asp Gly Lys Gly Thr Ser Gly
                115                 120

AAG AAG AAG CCC AAG TTC TTT TAC GCC CAT GGC CTT  1758
Lys Lys Lys Pro Lys Phe Phe Tyr Ala His Gly Leu
        125                 130

GAC TCC TCG TCT ATT ACT GGA TTA AAC ATC AAA AAC  1794
Asp Ser Ser Ser Ile Thr Gly Leu Asn Ile Lys Asn
135                 140                 145
```

```
ACC CCC CTT ATG GCG TTT AGT GTC CAG GCG AAT GAC  1830
Thr Pro Leu Met Ala Phe Ser Val Gln Ala Asn Asp
                150                 155

ATT ACG TTT ACC GAT GTT ACC ATC AAT AAT GCG GAT  1866
Ile Thr Phe Thr Asp Val Thr Ile Asn Asn Ala Asp
    160                 165                 170

GGC GAC ACC CAG GGT GGA CAC AAC ACT GAT GCG TTC  1902
Gly Asp Thr Gln Gly Gly His Asn Thr Asp Ala Phe
                175                 180

GAT GTT GGC AAC TCG GTC GGG GTG AAT ATC ATT AAG  1938
Asp Val Gly Asn Ser Val Gly Val Asn Ile Ile Lys
        185                 190

CCT TGG GTC CAT AAC CAG GAT GAC TGT CTT GCG GTT  1974
Pro Trp Val His Asn Gln Asp Asp Cys Leu Ala Val
195                 200                 205

AAC TCT GGC GAG GTAAGCAGCT CTGCATATAT GCTTGATTCG 2016
Asn Ser Gly Glu
            210

TAATTATATT GATATTCTAT AG AAC ATC TGG TTC ACC GGC 2056
                         Asn Ile Trp Phe Thr Gly
                                             215

GGC ACC TGC ATT GGC GGC CAC GGT CTC TCC ATC GGC  2092
Gly Thr Cys Ile Gly Gly His Gly Leu Ser Ile Gly
            220                 225

TCT GTC GGC GAC CGC TCC AAC AAC GTC GTC AAG AAC  2128
Ser Val Gly Asp Arg Ser Asn Asn Val Val Lys Asn
    230                 235                 240
```

```
GTC ACC ATC GAA CAC TCC ACC GTG AGC AAT TCC GAA  2164
Val Thr Ile Glu His Ser Thr Val Ser Asn Ser Glu
                    245                 250

AAC GCC GTC CGA ATT AAG ACC ATC TCT GGC GCC ACT  2200
Asn Ala Val Arg Ile Lys Thr Ile Ser Gly Ala Thr
        255                 260

GGC TCC GTG TCC GAG ATT ACG TAC TCC AAC ATC GTC  2236
Gly Ser Val Ser Glu Ile Thr Tyr Ser Asn Ile Val
265                 270                 275

ATG TCT GGC ATC TCC GAT TAC GGC GTG GTC ATT CAG  2272
Met Ser Gly Ile Ser Asp Tyr Gly Val Val Ile Gln
            280                 285

CAG GAT TAC GAA GAC GGC AAG CCT ACG GGT AAG CCG  2308
Gln Asp Tyr Glu Asp Gly Lys Pro Thr Gly Lys Pro
    290                 295                 300

ACG AAC GGT GTC ACT ATT CAG GAT GTT AAG CTG GAG  2344
Thr Asn Gly Val Thr Ile Gln Asp Val Lys Leu Glu
                    305                 310

AGC GTG ACT GGT AGC GTG GAT AGT GGG GCT ACT GAG  2380
Ser Val Thr Gly Ser Val Asp Ser Gly Ala Thr Glu
        315                 320

ATC TAT CTT CTT TGC GGG TCT GGT AGC TGC TCG GAC  2416
Ile Tyr Leu Leu Cys Gly Ser Gly Ser Cys Ser Asp
325                 330                 335

TGG ACC TGG GAC GAT GTG AAA GTT ACC GGG GGG AAG  2452
Trp Thr Trp Asp Asp Val Lys Val Thr Gly Gly Lys
            340                 345
```

```
AAG TCC ACC GCT TGC AAG AAC TTC CCT TCG GTG GCC   2488
Lys Ser Thr Ala Cys Lys Asn Phe Pro Ser Val Ala
    350                 355                 360

TCT TGT TAG GCTGCTAGGT TGGTGAGTTG TAGCCCTAGC      2527
Ser Cys
    362

TGAAATTCGT CTGCTTCGTC TGCTTCGTCT GCTTCGTCTG       2567
CTTCGTCTGC TTCTTCTGCT TCGTCTGCTT TGTCTGCTTT       2607
GTCTGCTTCG TCCACTTCGT CCACTTCGAC TGGTTAGATG       2647
GGCCTTGTAA TAGTTTTTAG AGAGAACAGA ATATGTACAG       2687
TAAGCCTTAG AGGTGGTACC GAGTTGTATA TTTATTTAAA       2727
ATGTTACCTA TCGCGTGTCT TTATATTTAT AGCCTTTTAC       2767
ATATATACGG AGCTACAGTG GATTATCTTA CAGCCCACAC       2807
TCATCGTGCT GGGAACTACG TGAATGAATG CTCGGTTAGA       2847
AGGCCTTGCT CACTGCCACA ACCAACCAGG AACCTTGGCA       2887
GGTACATGCT TGGGCATTTT TGTCTGGCCC TATCTCTTTC       2927
CAGATGGTGG TCTGGATGAG TCACGGCACG AGTAGATTGA       2967
CCGCTACTCC AACCCGCGCA TAAAGCATAC GCCAGAAGTG       3007
CAAGGGATAC AAGACAGCCA GCTG                        3031
```

SEQ ID NO. 3

SEQUENCE TYPE: Nucleotide with corresponding polypeptide
SEQUENCE LENGTH: 3047 base pairs
STRANDEDNESS: double
TOPOLOGY: linear
MOLECULE TYPE: genomic
ORIGINAL SOURCE ORGANISM: Aspergillus niger NW756
IMMEDIATE EXPERIMENTAL SOURCE: pGW1756 (DSM 5942)
FEATURES: Gene pgaII encoding A. niger NW756 prepro-polygalacturonase II
from 1 to 889: promoter region
from 890 to 952: coding region for PGII signal peptide
from 890 to 970: putative coding region for PGII prepro-sequence
from 971 to 2027: putative coding region for mature PGII
from 1520 to 1571: intron
from 2028 to 2030: stop codon
from 2031 to 3047: 3′ non coding region, part of transcriptional terminator region

```
CCGGATTTGC TCACCGTTGG CCAATGGCTT CCTCCTTTCC    40
TGTCTTCTCC TTCCTCTCCT TGACTGGCCC CACTCCAGGT    80
TGCTTATTGC CGGCAAGCTT TCAACCCTGA TTAGCGAGCT   120
TTGCACACTA TTCCCGATGT AGCTGTCGAA GGAACGGATG   160
GACGCTGCAC TAGTTTCATT TCGAATCTCT GATATTAATA   200
CTAACTACAG AGTGACTAGC CGATCACGCT CCGGATCTGG   240
CACAGAGTGA TTTTTTGAAT CATTCATTGG TGGAATTTGC   280
AAGGCACCAT AAATGCCGCA GACCCTGCAT ATCGAGCTAC   320
AGCCTTACTG GGATTAGTAT AGCTGCCATC TCGTACTCGT   360
ACACTGCAGA ACCGGCTTGG ATCCCAAGGC CTACGATGAC   400
ACCCGTTTCC TCCAGGACTG ATGGCTGTTT GTGAAGGCGA   440
ATTATAGTAC TGCTTGTCAA TGTCTTGAGT CAGCGGTATA   480
TATGAATCTG TCTTTGTCTT TCCACCTTGA CAATAATGTT   520
ACGCTCAAGA GGGCATTTTG CGTTCTTTTC ATCGACATGC   560
GAACCTGTTC GGTCACCCGC GGACCCCGTC GGCTGATCAG   600
```

```
CCACCACGGC TCATATATAT TAGTTGCCCA CCATGTCGAA      640
CTTAAGTTCA TTAAAAAAAA GGTAACATTT GAGACAATAT      680
CTTAATGTGA AACGTGAACC CTGGACTAGC ATCTCTCCAG      720
AGGCTGTCGG CAGTTATGAC TTTCCGATCA GAAGAGATGC      760
GCTGAAATTG TGACTATAAG AACCTCAAGC CTGCCGATGC      800
TGAGGTGAGT TTGCTCATCA TCCTACACTC ATTTGGCATC      840
AGACCGATTA CACTCTTTTT GTCCTTTTTT TCTATCGCTA      880

TCATTGACC ATG CAC TCC TTT GCT TCT CTT CTG GCC      916
          Met His Ser Phe Ala Ser Leu Leu Ala
            1               5

TAC GGC CTA GCC GCC AGC GCC ACC CTC GCT TCT GCC   952
Tyr Gly Leu Ala Ala Ser Ala Thr Leu Ala Ser Ala
 10                  15                  20

TCC CCC ATC GAA GCC CGG GGA AGC TGC ACC TTC AAA   988
Ser Pro Ile Glu Ala Arg Gly Ser Cys Thr Phe Lys
             25                  30

ACG GCT GCT GCT GCC AAA GCG GGC AAG GCA GGG TGC  1024
Thr Ala Ala Ala Ala Lys Ala Gly Lys Ala Gly Cys
     35                  40                  45

TCT ACC ATC ACC CTT GAC AAC ATC GAA GTC CCC GCT  1060
Ser Thr Ile Thr Leu Asp Asn Ile Glu Val Pro Ala
                 50                  55

GGA ACC ACC CTC GAC CTG ACC GGT CTC ACC AGC GGT  1096
Gly Thr Thr Leu Asp Leu Thr Gly Leu Thr Ser Gly
         60                  65

ACG AAG GTC ATC TTC GAG GGC ACC ACG ACC TTC GAT  1132
Thr Lys Val Ile Phe Glu Gly Thr Thr Thr Phe Asp
 70                  75                  80
```

```
TAT GAA GAA TGG GCA GGC CCC TTG ATC TCC ATG AGT  1168
Tyr Glu Glu Trp Ala Gly Pro Leu Ile Ser Met Ser
                 85                  90

GGC AAA GAT ATC ACC GTC ACT GGT GCC TCA GGC CAT  1204
Gly Lys Asp Ile Thr Val Thr Gly Ala Ser Gly His
     95                 100                 105

CTC ATC AAC TGC GAC GGT GCG CGG TGG TGG GAC GGC  1240
Leu Ile Asn Cys Asp Gly Ala Arg Trp Trp Asp Gly
                110                 115

AAG GGG ACC AGC GGA AAG AAG AAG CCC AAG TTC TTC  1276
Lys Gly Thr Ser Gly Lys Lys Lys Pro Lys Phe Phe
        120                 125

TAC GCT CAT GGC CTT GAC TCC TCG TCC ATT ACT GGA  1312
Tyr Ala His Gly Leu Asp Ser Ser Ser Ile Thr Gly
130                 135                 140

TTG AAT ATC AAG AAC ACT CCC CTT ATG GCG TTT AGT  1348
Leu Asn Ile Lys Asn Thr Pro Leu Met Ala Phe Ser
            145                 150

GTT CAG GCG GAT GAC ATC ACT CTG ACT GAC ATT ACC  1384
Val Gln Ala Asp Asp Ile Thr Leu Thr Asp Ile Thr
    155                 160                 165

ATC AAC AAC GCG GAC GGT GAT ACC CTG GGT GGA CAC  1420
Ile Asn Asn Ala Asp Gly Asp Thr Leu Gly Gly His
                170                 175

AAC ACT GAT GCG TTT GAT GTT GGT AAC TCT GTC GGT  1456
Asn Thr Asp Ala Phe Asp Val Gly Asn Ser Val Gly
        180                 185
```

```
GTG AAT ATC ATC AAA CCG TGG GTC CAT AAC CAG GAT   1492
Val Asn Ile Ile Lys Pro Trp Val His Asn Gln Asp
190                 195                 200

GAC TGT CTT GCG ATC AAC TCT GGC GAG GTAAGCAGCT    1529
Asp Cys Leu Ala Ile Asn Ser Gly Glu
            205                 210

TTGAACATAG ATTTGATTTG CATGTATGTT GATATTCTAT AG    1571

AAC ATC TGG TTT ACC AGC GGC ACC TGC ATT GGC GGC   1607
Asn Ile Trp Phe Thr Ser Gly Thr Cys Ile Gly Gly
                215                 220

CAC GGT CTC TCC ATC GGT TCT GTC GGC GGC CGC TCC   1643
His Gly Leu Ser Ile Gly Ser Val Gly Gly Arg Ser
        225                 230

AAC AAC GTT GTC AAG AAC GTC ACT ATC GAA CAC TCC   1679
Asn Asn Val Val Lys Asn Val Thr Ile Glu His Ser
235                 240                 245

ACC GTG AGC AAT TCC GAG AAC GCC GTC CGG ATT AAG   1715
Thr Val Ser Asn Ser Glu Asn Ala Val Arg Ile Lys
            250                 255

ACC GTC TCT GGT GCC ACT GGT TCC GTG TCT GAG ATC   1751
Thr Val Ser Gly Ala Thr Gly Ser Val Ser Glu Ile
    260                 265                 270

ACA TAC TCC AAC ATT GTC ATG TCC GGC ATC TCC GAT   1787
Thr Tyr Ser Asn Ile Val Met Ser Gly Ile Ser Asp
                275                 280
```

```
TAC GGC GTC GTT ATC CAG CAG GAT TAC GAG GAT GGC  1823
Tyr Gly Val Val Ile Gln Gln Asp Tyr Glu Asp Gly
        285                 290

AAG CCT ACG GGT AAG CCC ACG AAC GGT GTC ACT ATT  1859
Lys Pro Thr Gly Lys Pro Thr Asn Gly Val Thr Ile
295                 300                 305

ACG GAT GTC AAG CTG GAG AGC GTG ACT GGT ACT GTG  1895
Thr Asp Val Lys Leu Glu Ser Val Thr Gly Thr Val
            310                 315

GAT AGT AAG GCT ACT GAT ATC TAT CTC CTT TGC GGA  1931
Asp Ser Lys Ala Thr Asp Ile Tyr Leu Leu Cys Gly
    320                 325                 330

TCT GGT AGC TGC TCG GAC TGG ACT TGG GAC GAT GTG  1967
Ser Gly Ser Cys Ser Asp Trp Thr Trp Asp Asp Val
                335                 340

AAG GTC ACT GGA GGA AAG AAG TCT ACT GCT TGC AAA  2003
Lys Val Thr Gly Gly Lys Lys Ser Thr Ala Cys Lys
        345                 350

AAC TAC CCT TCG GTG GCT TCT TGC TAG GTTAGTAGGT   2040
Asn Tyr Pro Ser Val Ala Ser Cys
355                 360     362

TGTTCGGTTG TAGCACTTGC TAACATGCAT TTGCCTTGAG      2080
GGGTCAAATG GATTTGTGAA ATTATCGGTG TGAAAGAAGA      2120
GATGGTGTCC AGTAAGATTC AGTGGTGGCA GCGTGTATAG      2160
CTCTATACAA TGTTATTTAT CGCATAACTC TATATATCAA      2200
ATACTCGATT AAGAGAACCT GCCTTCAGCC ACAAATAACC      2240
AAGTTCCTCG GCAGGTATCA GATTCCGGGA ACCCCTACCT      2280
AGCCTTACTC CGTTGCAGAT AGTTCTGCTG GTAAATCAGG      2320
ATGGTATGCT GAATTACGAT GCCAGCAAAT TCACCGCTAC      2360
```

```
TCCAACCCGC GCATAAAACA TACGCCAGAA GTGCAAGGGA   2400
TAAAGACAGC CAGCTGTGTC TTCGCGCAAG TAACTCTGCA   2440
TAATCCAGTT TCTGACATAG TATAGTCATC TCTTCTACAC   2480
CTTGGCAAAC TCCCTCTCCA TAAACTCCCT CACAGTAATA   2520
AGCGTCTCCC TCCCCTTCCC GCCCTTGGCC TTAAACGCCG   2560
CCTTCTTCAA CGCCGCAGCC GTTTGAATCT CCGTAGGCCC   2600
ATTACCAAAG TACTCCCCAT CCCTAAACTG CGAATAAATA   2640
AAAACATCCC GCTCCTGCTC ACGCCAATAC TCCCCTTTCG   2680
TATTCATGTC GTTCGGGTCC GCTAGAACCT CATACCGGGC   2720
TTTCTTGCCA GTCACTATAC CCATCACCGT CAGCATCTAC   2760
TCATCACTCA TCACGTAAAA GATAATGATA AGTAAAACCT   2800
TACCAGAAAC AAACGTATTC ACCACCTCCG CCGATGAAAC   2840
AATATCACTC GACCCCTGAA TCAGCCGTCT ATTCCACCGC   2880
AACGGGTTAA CAAAAACCCC ATGAACCAGA TCCCCAAAGT   2920
CATCCTTCAT ACTGATCCAG GGGAAATCTT CTTTCCCGCC   2960
CCAAAATGGC GTCTTGTATG TTAGATAACC CTCAGAGTCA   3000
GGGAATGTGG GCCATCCGCC GAATAAGTCG GCGTAGTCCG   3040
GCTCGAG                                       3047
```

SEQ ID NO. 4

SEQUENCE TYPE: Nucleotide with corresponding polypeptide
SEQUENCE LENGTH: 2304
STRANDEDNESS: double
TOPOLOGY: linear
MOLECULE TYPE: genomic
ORIGINAL SOURCE ORGANISM: Aspergillus niger N400
IMMEDIATE EXPERIMENTAL SOURCE: pGW1910 (DSM 5943)
FEATURES: Gene pgaC encoding A. niger N400 prepro-polygalacturonaseC
from 1 to 662: promoter region
from 663 to 710: coding region for PGC signal peptide
from 663 to 782: putative coding region for PGC prepro-sequence
from 783 to 1995: putative coding region for mature PGC
from 927 to 1001: intron
from 1428 to 1483: intron
from 1614 to 1666: intron
from 1996 to 1998: stop codon
from 1999 to 2304: 3′ non-coding region, part of transcriptional terminator region

```
CCATGGCTGA AACTTTGCCC CTGACCTAGT CCATTTCATT     40
TTATATAATG CTGATGAGAT ATGGTTCTTG CACAACTATG     80
CTTTTCCTCG GTCGGTGCGT AATGTATAGT TCGTGGGTGG    120
TAAGGAAATT CACCGACAGC AACTCCCAGC ATAACATGGA    160
GAAGAACCGA TGAATGAGCA GCGACAAACA TGGTACCACA    200
ATTCTTGAAT AAGCATTTAG ACTCCGGCTT GGTTTTTTCC    240
GTTGCATTGG CGCTGGTGTT GTTCCTGCGC ACGGTGCAAC    280
CGTTATCTCC ACCAATGATT ACCTGCAGAA AGCCCATGGC    320
TGCAACCACC CAGCCGACTT GAGTCCACCT AACAGCATAC    360
TCAGCATACT CGATGCTGGG AATTACTTCA GGTATGGTCG    400
GCTGAATATG CAGTCGATTT CCGGCGCCAG GAACAGTCCG    440
GCCCCCTCAC CACGGACATA GACCCGCCAC CAAAACGTCG    480
ATAGCGGCCT CGCTTCGTCA ACGACCATTT TGCCCACCCC    520
```

```
TGACACCCAG GAAAGACGTC TTCCAATAAC AATATAAAAG        560
GGCGAGTCTT GTCCTCTCAC TTTCCGTCTT TCGAGTATGC        600
CTTGCCAGTT CCCAACTTGA CTTGAGACCT CCCATTTCGG        640

TCATTTGTCA CCCGCTATAA GA ATG GTC CGT CAG CTT       677
                         Met Val Arg Gln Leu
                          1               5

ATC CTG ATC AGC AGT CTG CTG GCA GCT GTT GCT GTG    713
Ile Leu Ile Ser Ser Leu Leu Ala Ala Val Ala Val
                 10                  15

CGC GCG CCT GCC GAT CCG GCT CAT CCC ATG GTT ACG    749
Arg Ala Pro Ala Asp Pro Ala His Pro Met Val Thr
         20                  25

GAA GCG CCT GAC GTC AAT TTG GTT GAA AAG AGG GCG    785
Glu Ala Pro Asp Val Asn Leu Val Glu Lys Arg Ala
 30                  35                  40

ACT ACT TGC ACC TTC TCG GGC TCC GAA GGT GCA TCC    821
Thr Thr Cys Thr Phe Ser Gly Ser Glu Gly Ala Ser
             45                  50

AAG GCC AGC AAG TCG AAA ACC TCT TGC TCC ACA ATC    857
Lys Ala Ser Lys Ser Lys Thr Ser Cys Ser Thr Ile
     55                  60                  65

TAC CTG TCC GAC GTG GCC GTC CCA TCT GGC ACA ACC    893
Tyr Leu Ser Asp Val Ala Val Pro Ser Gly Thr Thr
                 70                  75

CTT GAT CTC TCT GAC CTG AAT GAT GGA ACC CAC        926
Leu Asp Leu Ser Asp Leu Asn Asp Gly Thr His
         80                  85
```

```
GTACGTTCCC CGGGTGATTC ATGTCTTATT CTCACACCCA                966

ATCGCGTCAA TAGTACTGGC TGACAGATGT ACTAG GTG ATC TTC        1010
                                       Val Ile Phe
                                           90

CAG GGA GAA ACC ACT TTT GGA TAC GAG GAA TGG GAA           1046
Gln Gly Glu Thr Thr Phe Gly Tyr Glu Glu Trp Glu
                95                  100

GGG CCT CTT GTG CGT GTT TCT GGA ACT GAT ATC ACG           1082
Gly Pro Leu Val Arg Val Ser Gly Thr Asp Ile Thr
    105                 110                 115

GTC GAG GGG GAG AGC GAC GCG GTG CTC AAT GGC GAT           1118
Val Glu Gly Glu Ser Asp Ala Val Leu Asn Gly Asp
                    120                 125

GGC AGC CGC TGG TGG GAT GGA GAG GGT GGC AAT GGT           1154
Gly Ser Arg Trp Trp Asp Gly Glu Gly Gly Asn Gly
        130                 135

GGT AAA ACA AAG CCC AAG TTC TTC TAT GCC CAT GAC           1190
Gly Lys Thr Lys Pro Lys Phe Phe Tyr Ala His Asp
140                 145                 150

TTG ACC TCT TCC ACC ATC AAG AGC ATC TAC ATC GAG           1226
Leu Thr Ser Ser Thr Ile Lys Ser Ile Tyr Ile Glu
            155                 160

AAC TCT CCT GTG CAG GTG TTC AGC ATC GAT GGC TCC           1262
Asn Ser Pro Val Gln Val Phe Ser Ile Asp Gly Ser
    165                 170                 175
```

```
ACT GAT CTT ACC ATG ACT GAT ATC ACG GTG GAT AAC    1298
Thr Asp Leu Thr Met Thr Asp Ile Thr Val Asp Asn
                180                 185

ACG GAT GGT GAC ACG GAC GAC CTC GCC GCC AAT ACG    1334
Thr Asp Gly Asp Thr Asp Asp Leu Ala Ala Asn Thr
        190                 195

GAT GGC TTC GAC ATC GGG GAA AGC ACC TAT ATC ACG    1370
Asp Gly Phe Asp Ile Gly Glu Ser Thr Tyr Ile Thr
200                 205                 210

ATC ACA GGT GCC GAA ATC TAC AAC CAA GAT GAC TGC    1406
Ile Thr Gly Ala Glu Ile Tyr Asn Gln Asp Asp Cys
            215                 220

GTT GCC ATC AAT TCT GGA GAG GTATGCGTCC CCTGGCACTG 1447
Val Ala Ile Asn Ser Gly Glu
    225                 230

ATTGAGAGGA GAGGCGCCTT GCTGACGAT CTGGTAG AAC        1486
                                        Asn

ATT TAT TTC AGT GCC AGT GTG TGT TCT GGT GGT CAC    1522
Ile Tyr Phe Ser Ala Ser Val Cys Ser Gly Gly His
            235                 240

GGC CTG TCT ATT GGC TCT GTT GGT GGT CGG GAT GAT    1558
Gly Leu Ser Ile Gly Ser Val Gly Gly Arg Asp Asp
    245                 250                 255

AAC ACT GTC AAG AAC GTG ACG TTT TAT GAT GTC AAT    1594
Asn Thr Val Lys Asn Val Thr Phe Tyr Asp Val Asn
                260                 265
```

```
GTT CTC AAG TCC CAG CAA GGTAAGGGAA AGCATTTGAA     1632
Val Leu Lys Ser Gln Gln
        270

CCATCCGTTT GCCGTCCTCT AACGTATGCT TTA GCA ATC CGT  1674
                                     Ala Ile Arg
                                         275

ATC AAG ACC ATC TAC GGC GAC ACT GGC TCC GTC AGC   1710
Ile Lys Thr Ile Tyr Gly Asp Thr Gly Ser Val Ser
            280                 285

GAA GTC ACT TAC CAT GAG ATT GCC TTT TCG GAC GCT   1746
Glu Val Thr Tyr His Glu Ile Ala Phe Ser Asp Ala
    290                 295                 300

ACT GAT TAC GGC ATT GTC ATT GAG CAG AAC TAT GAT   1782
Thr Asp Tyr Gly Ile Val Ile Glu Gln Asn Tyr Asp
                305                 310

GAC ACC TCC AAG ACC CCT ACT ACC GGG GTA CCG ATC   1818
Asp Thr Ser Lys Thr Pro Thr Thr Gly Val Pro Ile
        315                 320

ACG GAT TTT GTG CTC GAG AAC ATC GTT GGA ACG TGT   1854
Thr Asp Phe Val Leu Glu Asn Ile Val Gly Thr Cys
325                 330                 335

GAA GAT GAT GAT TGT ACC GAA GTA TAC ATT GCC TGC   1890
Glu Asp Asp Asp Cys Thr Glu Val Tyr Ile Ala Cys
            340                 345

GGT GAT GGC AGC TGC TCG GAT TGG ACT TGG ACT GGC   1926
Gly Asp Gly Ser Cys Ser Asp Trp Thr Trp Thr Gly
    350                 355                 360
```

```
GTG AGC GTG ACT GGC GGC AGT GTC AGT GAC GAC TGC   1962
Val Ser Val Thr Gly Gly Ser Val Ser Asp Asp Cys
                365                 370

CTT AAT GTT CCC TCC GGG ATT AGC TGC GAT TTG TAG   1998
Leu Asn Val Pro Ser Gly Ile Ser Cys Asp Leu
        375                 380         383

GCCGTTGTGA TTGGGGGAGA TGTTCCCGGG TACTCTGGTC       2038
GTTCGTTTAG CCAGCCCTTT CTGTTGAGTG GCCTGTTCTT       2078
CTATACTGTC GATTGTGTGT GAGATTACTA CCTTGCCCGA       2118
GGAAAGAGCA GTGCATCCTA TCTTTTATTC TTTGCCGGTC       2158
GGGGTTGGCC TACTAGTACA TTGTACCCGA AGAGTCAACG       2198
GTGAAAGTAA AGGGCTTACA GAATTAGTCC AGGAGCAAAG       2238
ACAACTTTTA TCAGAGCGCA TCGGTCACGT CATGTTGAAA       2278
GTATTGATCA ATGAAGATAA CCTTGG                      2304
```

**Claims**

1. A recombinant DNA molecule comprising a DNA sequence selected from
   a) the Aspergillus niger DNA insert of pGW1800 or pGW1900,
   b) a DNA sequence which hybridizes to the coding region for the mature PGII or PGI comprised by a DNA insert of a) and which comprises a structural gene for a polypeptide with galacturonase or polygalacturonase activity, and optionally a promoter, a coding region for a signal or leader peptide and/or a transcriptional terminator,
   c) a derivative of a DNA sequence defined in a) or b), or
   d) a DNA sequence which codes for a mature PG or a signal peptide or a leader peptide thereof and which is degenerate within the meaning of the genetic code with respect to a DNA sequence of a), b) or c).
2. A recombinant DNA molecule according to claim 1 comprising the Aspergillus niger DNA insert of pGW1800 or pGW1900.
3. A recombinant DNA molecule according to claim 1 comprising a DNA sequence which hybridizes to the coding region for the mature PGII or PGI comprised by a DNA insert of pGW1800 or pGW1900 and which codes for a polypeptide with galacturonase or polygalacturonase activity, and/or a signal or leader peptide and/or has promoter and/or transcriptional terminator activity.
4. A recombinant DNA molecule according to claim 1 comprising a derivative of the Aspergillus niger DNA insert of pGW1800 or pGW1900, or of a DNA sequence which hybridizes to the coding region for the mature PGII or PGI comprised by a DNA insert of pGW1800 or pGW1900 and which codes for a polypeptide with galacturonase or polygalacturonase activity, and/or a signal or leader peptide and/or has promoter and/or transcriptional terminator activity.
5. A recombinant DNA molecule according to claim 4 comprising an insert or fragment thereof of a vector selected from the group of vectors consisting of λPG-A9, -A10, -A43, -B4, -B35, -B36, -C1, -C16, -C20, -C37, -D8, -D11, -D12, -E6, -E38, -F5, -G17, -G27, -X31 and -Y33, pGW1756, pGW1910, pGW1800, pGW1900 and pGW1756.
6. A recombinant DNA molecule according to claim 4 comprising a DNA fragment derived from an A. niger insert of pGW1800 or pGW1900, or a DNA sequence which hybridizes to the coding region for the mature PGII or PGI comprised by one of said DNA inserts and encodes a structural gene and/or a leader or signal peptide and/or comprises a sequence with promoter and/or transcriptional terminator activity.

7. A recombinant DNA molecule according to claim 6 comprising a DNA fragment with promoter activity derived from an insert of a vector selected from the group of vectors consisting of λPG-A9, -A10, -A43, -B4, -B35, -B36, -C1, -C16, -C20, -C37, -D8, -D11, -D12, -E6, -E38, -F5, -G17, -G27, -X31 and -Y33, pGW1910, pGW1800, pGW1900 and pGW1756.

8. A recombinant DNA molecule according to claim 6 comprising a DNA fragment encoding a leader peptide derived from an insert of a vector selected from the group of vectors consisting of λPG-A9, -A10, -A43, -B4, -B35, -B36, -C1, -C16, -C20, -C37, -D8, -D11, -D12, -E6, -E38, -F5, -G17, -G27, -X31 and -Y33, pGW1910, pGW1800, pGW1900 and pGW1756.

9. A recombinant DNA molecule according to claim 6 comprising a DNA fragment encoding a signal peptide derived from an insert of a vector selected from the group of vectors consisting of λPG-A9, -A10, -A43, -B4, -B35, -B36, -C1, -C16, -C20, -C37, -D8, -D11, -D12, -E6, -E38, -F5, -G17, -G27, -X31 and -Y33, pGW1910, pGW1800, pGW1900 and pGW1756.

10. A recombinant DNA molecule according to claim 6 comprising a DNA fragment with transcriptional terminator activity derived from an insert of a vector selected from the group of vectors consisting of λPG-A9, -A10, -A43, -B4, -B35, -B36, -C1, -C16, -C20, -C37, -D8, -D11, -D12, -E6, -E38, -F5, -G17, -G27, -X31 and -Y33, pGW1910, pGW1800, pGW1900 and pGW1756.

11. A recombinant DNA molecule according to claim 6 comprising a DNA fragment encoding a structural gene for a galacturonase or polygalacturonase or a fragment thereof with galacturonase or polygalacturonase activity derived from an insert of a vector selected from the group of vectors consisting of λPG-A9, -A10, -A43, -B4, -B35, -B36, -C1, -C16, -C20, -C37, -D8, -D11, -D12, -E6, -E38, -F5, -G17, -G27, -X31 and -Y33, pGW1910, pGW1800, pGW1900 and pGW1756.

12. A recombinant DNA molecule according to claim 1 which is an expression cassette.

13. A recombinant DNA molecule according to claim 1 which is a hybrid vector.

14. A recombinant DNA molecule according to claim 13 selected from the group of vectors consisting of pGW 1800, pGW 1900, pGW 1756, pGW 1910, λPG-A9, λPG-A10, λPG-A43, λPG-B4, λPG-B35, λPG-B36, λPG-C1, λPG-C16, λPG-C20, λPG-C37, λPG-D8, λPG-D11, λPG-D12, λPG-E6, λPG-E38, λPG-F5, λPG-G17, λPG-G27, λPG-X31 and λPG-Y33.

15. A DNA molecule comprising a gene for a PG or a functional fragment thereof which is isolated from a PG producing filamentous fungal strain.

16. A DNA molecule according to claim 15 which is isolated from Aspergillus spec .

17. A DNA molecule according to claim 15 which is an insert of a hybrid vector selected from the group of hybrid vectors consisting of pGW1800, pGW 1900, pGW 1756, pGW 1910, λPG-A9, λPG-A10, λPG-A43, λPG-B4, λPG-B35, λPG-B36, λPG-C1, λPG-C16, λPG-C20, λPG-C37, λPG-D8, λPG-D11, λPG-D12, λPG-E6, λPG-E38, λPG-F5, λPG-G17, λPG-G27, λPG-X31 and λPG-Y33 or a fragment thereof comprising a promoter region of a PG gene, or a coding region for a signal peptide, leader peptide, prepro-PG, PG or a fragment of a PG with galacturonase or polygalacturonase activity, or a transcriptional terminator region of a PG gene.

18. A process for the preparation of a recombinant DNA molecule according to claim 1 or a DNA molecule according to claim 15 comprising

a) isolating genomic DNA from suitable fungal cells, and selecting the desired DNA, e.g. using a DNA probe or using a suitable expression system and screening for expression of the desired polypeptide, or

b) isolating mRNA from suitable fungal cells, selecting the desired mRNA, e.g. by hybridization with a DNA probe or by expression in a suitable expression system and screening for expression of the desired polypeptide, preparing single-stranded cDNA complementary to that mRNA, then double-stranded cDNA therefrom, or

c) isolating cDNA from a cDNA library and selecting the desired cDNA, e.g. using a DNA probe or using a suitable expression system and screening for expression of the desired polypeptide, or/and

d) incorporating the double-stranded DNA of step a), b) or c) into an appropriate vector,

e) transforming appropriate host cells with the obtained hybrid vector,

f) selecting transformed host cells which contain the desired DNA from untransformed host cells or multiplicating the transformed host cell, and, when required,

g) isolating the desired DNA and/or converting the DNA into a mutant or fragment thereof.

19. A host transformed with a recombinant DNA molecule according to claim 1.

20. A process for the preparation of a transformed host according to claim 19 comprising treatment of a suitable host cell under transforming conditions with a recombinant DNA molecule of the present invention, especially a hybrid vector of the invention, optionally together with a selection marker gene and optionally selecting the transformants.

21. A process for the preparation of a polypeptide characterized in that a structural gene inserted in an expression cassette according to claim 12 is expressed in a suitable transformed host according to conventional methods and optionally the polypeptide is isolated in conventional manner.
22. A process according to claim 21 wherein an Aspergillus niger strain is used as host.
23. A process according to claim 21 wherein an Aspergillus nidulans strain is used as host.
24. A process according to claim 21 wherein the structural gene encodes the hybrid interferon BDBB.
25. A process according to claim 21 wherein the structural gene encodes a polypeptide with galacturonase or polygalacturonase activity and is derived from a DNA sequence according to claim 1.
26. A process for the preparation of a single PG wherein such PG is purified from a crude source according to conventional methods.
27. A process according to claim 26, wherein a PG is produced in purified form from a crude enzyme mixture obtained from Aspergillus niger .
28. A single galacturonase or polygalacturonase encoded by a DNA sequence according to claim 1 or a derivative thereof with galacturonase or polygalacturonase activity and biologically acceptable salts thereof.
29. An enzymatic composition comprising a polypeptide according to claim 28 or any mixture thereof optionally in a predetermined combination with one or more enzymes having other than PG activity.
30. The use of a polypeptide according to claim 28 or of an enzymatic composition according to claim 29 in plant material processing.

Claims for the following contracting states ES, GR:

1. A process for the preparation of a recombinant DNA molecule comprising a DNA sequence selected from
a) the Aspergillus niger DNA insert of pGW1800 or pGW1900,
b) a DNA sequence which hybridizes to the coding region for the mature PGII or PGI comprised by a DNA insert of a) and which comprises a structural gene for a polypeptide with galacturonase or polygalacturonase activity, and optionally a promoter, a coding region for a signal or leader peptide and/or a transcriptional terminator,
c) a derivative of a DNA sequence defined in a) or b), or
d) a DNA sequence which codes for a mature PG or a signal peptide or a leader peptide thereof and which is degenerate within the meaning of the genetic code with respect to a DNA sequence of a), b) or c),
which process comprises
- a) isolating genomic DNA from suitable fungal cells, and selecting the desired DNA, e.g. using a DNA probe or using a suitable expression system and screening for expression of the desired polypeptide, or
- b) isolating mRNA from suitable fungal cells, selecting the desired mRNA, e.g. by hybridization with a DNA probe or by expression in a suitable expression system and screening for expression of the desired polypeptide, preparing single-stranded cDNA complementary to that mRNA, then double-stranded cDNA therefrom, or
- c) isolating cDNA from a cDNA library and selecting the desired cDNA, e.g. using a DNA probe or using a suitable expression system and screening for expression of the desired polypeptide, or/and
- d) incorporating the double-stranded DNA of step a), b) or c) into an appropriate vector,
- e) transforming appropriate host cells with the obtained hybrid vector,
- f) selecting transformed host cells which contain the desired DNA from untransformed host cells or multiplicating the transformed host cell, and, when required,
- g) isolating the desired DNA and/or converting the DNA into a mutant or fragment thereof.

2. A process according to claim 1 for the preparation of a recombinant DNA molecule comprising the Aspergillus niger DNA insert of pGW1800 or pGW1900.
3. A process according to claim 1 for the preparation of a recombinant DNA molecule comprising a DNA sequence which hybridizes to the coding region for the mature PGII or PGI comprised by a DNA insert of pGW1800 or pGW1900 and which codes for a polypeptide with galacturonase or polygalacturonase activity, and/or signal or leader peptide and/or has promoter and/or transcriptional terminator activity.
4. A process according to claim 1 for the preparation of a recombinant DNA molecule comprising a derivative of the Aspergillus niger DNA insert of pGW1800 or pGW1900, or of a DNA sequence which hybridizes to the coding region for the mature PGII or PGI comprised by a DNA insert of pGW1800 or pGW1900 and which codes for a polypeptide with galacturonase or polygalacturonase activity, and/or signal or leader peptide and/or has promoter and/or transcriptional terminator activity.
5. A process according to claim 4 for the preparation of a recombinant DNA molecule comprising an insert or fragment thereof of a vector selected from the group of vectors consisting of λPG-A9, -A10, -A43, -B4, -B35, -B36, -C1, -C16, -C20, -C37, -D8, -D11, -D12, -E6, -E38, -F5, -G17, -G27, -X31 and -Y33, pGW1756, pGW1910, pGW1800, pGW1900 and pGW1756.

6. A process according to claim 4 for the preparation of a recombinant DNA molecule comprising a DNA fragment derived from the A. niger insert of pGW1800 or pGW1900, or a DNA sequence which hybridizes to the coding region for the mature PGII or PGI comprised by one of said DNA inserts and encodes a structural gene and/or a leader or signal peptide and/or comprises a sequence with promoter and/or transcriptional terminator activity.

7. A process according to claim 4 for the preparation of a recombinant DNA molecule comprising a DNA fragment with promoter activity derived from an insert of a vector selected from the group of vectors consisting of λPG-A9, -A10, -A43, -B4, -B35, -B36, -C1, -C16, -C20, -C37, -D8, -D11, -D12, -E6, -E38, -F5, -G17, -G27, -X31 and -Y33, pGW1910, pGW1800, pGW1900 and pGW1756.

8. A process according to claim 4 for the preparation of a recombinant DNA molecule comprising a DNA fragment encoding a leader peptide derived from an insert of a vector selected from the group of vectors consisting of λPG-A9, -A10, -A43, -B4, -B35, -B36, -C1, -C16, -C20, -C37, -D8, -D11, -D12, -E6, -E38, -F5, -G17, -G27, -X31 and -Y33, pGW1910, pGW1800, pGW1900 and pGW1756.

9. A process according to claim 4 for the preparation of a recombinant DNA molecule comprising a DNA fragment encoding a signal peptide derived from an insert of a vector selected from the group of vectors consisting of λPG-A9, -A10, -A43, -B4, -B35, -B36, -C1, -C16, -C20, -C37, -D8, -D11, -D12, -E6, -E38, -F5, -G17, -G27, -X31 and -Y33, pGW1910, pGW1800, pGW1900 and pGW1756.

10. A process according to claim 4 for the preparation of a recombinant DNA molecule comprising a DNA fragment with transcriptional terminator activity derived from an insert of a vector selected from the group of vectors consisting of λPG-A9, -A10, -A43, -B4, -B35, -B36, -C1, -C16, -C20, -C37, -D8, -D11, -D12, -E6, -E38, -F5, -G17, -G27, -X31 and -Y33, pGW1910, pGW1800, pGW1900 and pGW1756.

11. A process according to claim 4 for the preparation of a recombinant DNA molecule comprising a DNA fragment encoding a structural gene for a galacturonase or polygalacturonase or a fragment thereof with galacturonase or polygalacturonase activity derived from an insert of a vector selected from the group of vectors consisting of λPG-A9, -A10, -A43, -B4, -B35, -B36, -C1, -C16, -C20, -C37, -D8, -D11, -D12, -E6, -E38, -F5, -G17, -G27, -X31 and -Y33, pGW1910, pGW1800, pGW1900 and pGW1756.

12. A process according to claim 1 for the preparation of a recombinant DNA molecule according to claim 1 which is an expression cassette.

13. A process according to claim 1 for the preparation of a recombinant DNA molecule which is a hybrid vector.

14. A process according to claim 1 for the preparation of a recombinant DNA molecule selected from the group of vectors consisting of pGW1800, pGW 1900, pGW 1756, pGW 1910, λPG-A9, λPG-A10, λPG-A43, λPG-B4, λPG-B35, λPG-B36, λPG-C1, λPG-C16, λPG-C20, λPG-C37, λPG-D8, λPG-D11, λPG-D12, λPG-E6, λPG-E38, λPG-F5, λPG-G17, λPG-G27, λPG-X31 and λPG-Y33.

15. A process for the preparation of a DNA molecule comprising a gene for a PG or a functional fragment thereof which is isolated from a PG producing filamentous fungal strain which process comprises

a) isolating genomic DNA from suitable fungal cells, and selecting the desired DNA, e.g. using a DNA probe or using a suitable expression system and screening for expression of the desired polypeptide, or

b) isolating mRNA from suitable fungal cells, selecting the desired mRNA, e.g. by hybridization with a DNA probe or by expression in a suitable expression system and screening for expression of the desired polypeptide, preparing single-stranded cDNA complementary to that mRNA, then double-stranded cDNA therefrom, or

c) isolating cDNA from a cDNA library and selecting the desired cDNA, e.g. using a DNA probe or using a suitable expression system and screening for expression of the desired polypeptide, or/and

d) incorporating the double-stranded DNA of step a), b) or c) into an appropriate vector,

e) transforming appropriate host cells with the obtained hybrid vector,

f) selecting transformed host cells which contain the desired DNA from untransformed host cells or multiplicating the transformed host cell, and, when required, g) isolating the desired DNA and/or converting the DNA into a mutant or fragment thereof.

16. A process according to claim 15 for the preparation of a DNA molecule which is isolated from Aspergillus spec .

17. A process according to claim 15 for the preparation of a DNA molecule which is an insert of a hybrid vector selected from the group of hybrid vectors consisting of pGW1800, pGW 1900, pGW 1756, pGW 1910, λPG-A9, λPG-A10, λPG-A43, λPG-B4, λPG-B35, λPG-B36, λPG-C1, λPG-C16, λPG-C20, λPG-C37, λPG-D8, λPG-D11, λPG-D12, λPG-E6, λPG-E38, λPG-F5, λPG-G17, λPG-G27, λPG-X31 and λPG-Y33 or a fragment thereof comprising a promoter region of a PG gene, or a coding region for a signal peptide, leader peptide, prepro-PG, PG or a fragment of a PG with galacturonase or polygalacturonase activity, or a transcriptional terminator region of a PG gene.

18. A process for the preparation of a host transformed with a recombinant DNA molecule comprising a DNA sequence selected from

a) the Aspergillus niger DNA insert of pGW1800 or pGW1900,

b) a DNA sequence which hybridizes to the coding region for the mature PGII or PGI comprised by a DNA insert of a) and which comprises a structural gene for a polypeptide with galacturonase or polygalacturonase activity, and optionally a promoter, a coding region for a signal or leader peptide and/or a transcriptional terminator,

c) a derivative of a DNA sequence defined in a) or b), or

d) a DNA sequence which codes for a mature PG or a signal peptide or a leader peptide thereof and which is degenerate within the meaning of the genetic code with respect to a DNA sequence of a), b) or c), comprising treatment of a suitable host cell under transforming conditions with a recombinant DNA molecule of the present invention, especially a hybrid vector of the invention, optionally together with a selection marker gene and optionally selecting the transformants.

19. A process for the preparation of a polypeptide characterized in that a structural gene inserted in an expression cassette comprising a DNA sequence selected from

a) the Aspergillus niger DNA insert of pGW1800 or pGW1900,

b) a DNA sequence which hybridizes to the coding region for the mature PGII or PGI comprised by a DNA insert of a) and which comprises a structural gene for a polypeptide with galacturonase or polygalacturonase activity, and optionally a promoter, a coding region for a signal or leader peptide and/or a transcriptional terminator,

c) a derivative of a DNA sequence defined in a) or b), or

d) a DNA sequence which codes for a mature PG or a signal peptide or a leader peptide thereof and which is degenerate within the meaning of the genetic code with respect to a DNA sequence of a), b) or c), is expressed in a suitable host according to conventional methods and optionally the polypeptide is isolated in conventional manner.

20. A process according to claim 19 wherein an Aspergillus niger strain is used.

21. A process according to claim 19 wherein an Aspergillus nidulans strain is used.

22. A process according to claim 19 wherein the structural gene encodes the hybrid interferon BDBB.

23. A process according to claim 19 wherein the structural gene encodes a polypeptide with galacturonase or polygalacturonase activity and is derived from a DNA sequence selected from

a) the Aspergillus niger DNA insert of pGW1800 or pGW1900,

b) a DNA sequence which hybridizes to the coding region for the mature PGII or PGI comprised by a DNA insert of a) and which comprises a structural gene for a polypeptide with galacturonase or polygalacturonase activity, and optionally a promoter, a coding region for a signal or leader peptide and/or a transcriptional terminator,

c) a derivative of a DNA sequence defined in a) or b), or

d) a DNA sequence which codes for a mature PG or a signal peptide or a leader peptide thereof and which is degenerate within the meaning of the genetic code with respect to a DNA sequence of a), b) or c).

24. A process for the preparation of a single PG wherein such PG is purified from a crude source according to conventional methods.

25. A process according to claim 24, wherein a PG is produced in purified form from a crude enzyme mixture obtained from Aspergillus niger .

26. A process for the preparation of a single galacturonase or polygalacturonase encoded by a DNA sequence according to claim 1 or a derivative thereof with galacturonase or polygalacturonase activity and biologically acceptable salts thereof.

27. A process for the preparation of an enzymatic composition comprising a polypeptide according to claim 26 or any mixture thereof optionally in a predetermined combination with one or more enzymes having other than PG activity.

28. A process for plant material processing in which a polypeptide according to claim 26 or an enzymatic composition according to claim 27 is used.

FIGURE 1

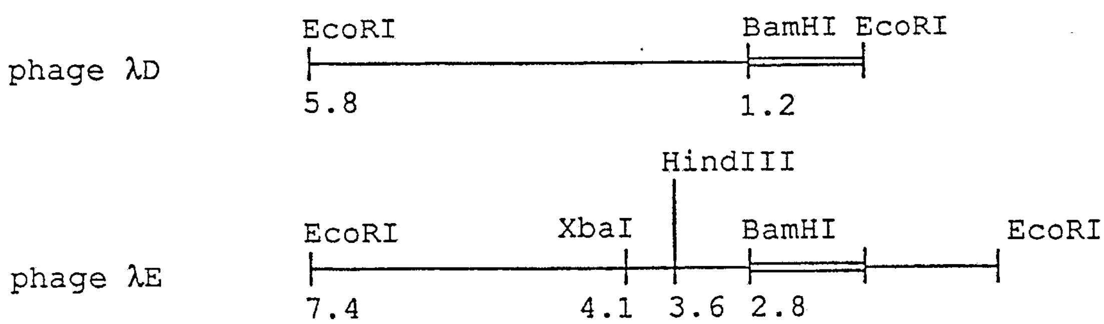


▭ · sequences hybridizing with the combined oligonucleotide mixtures HR6195 and HR6196.

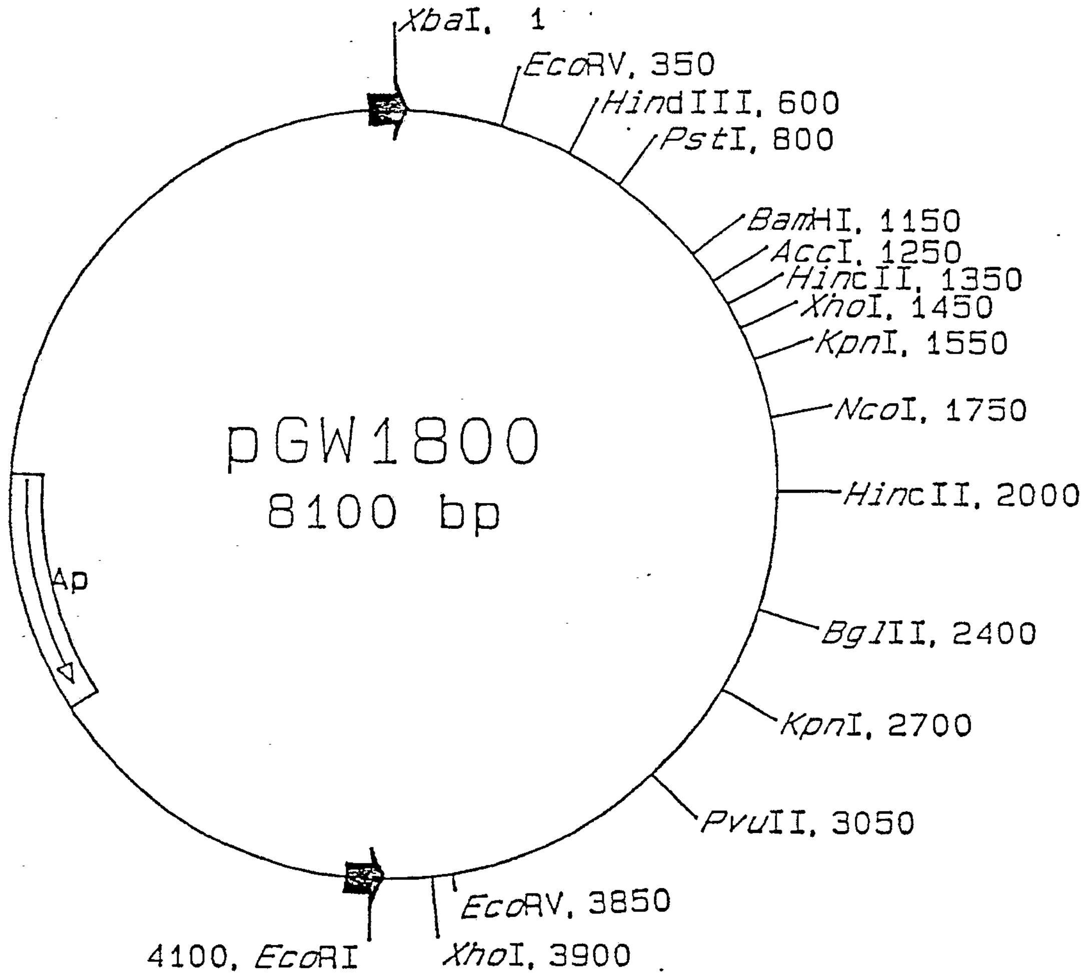

XbaI, 1
EcoRV, 350
HindIII, 600
PstI, 800
BamHI, 1150
AccI, 1250
HincII, 1350
XhoI, 1450
KpnI, 1550
NcoI, 1750
HincII, 2000
BglII, 2400
KpnI, 2700
PvuII, 3050
EcoRV, 3850
XhoI, 3900
4100, EcoRI
pGW1800
8100 bp
Ap

FIGURE 3

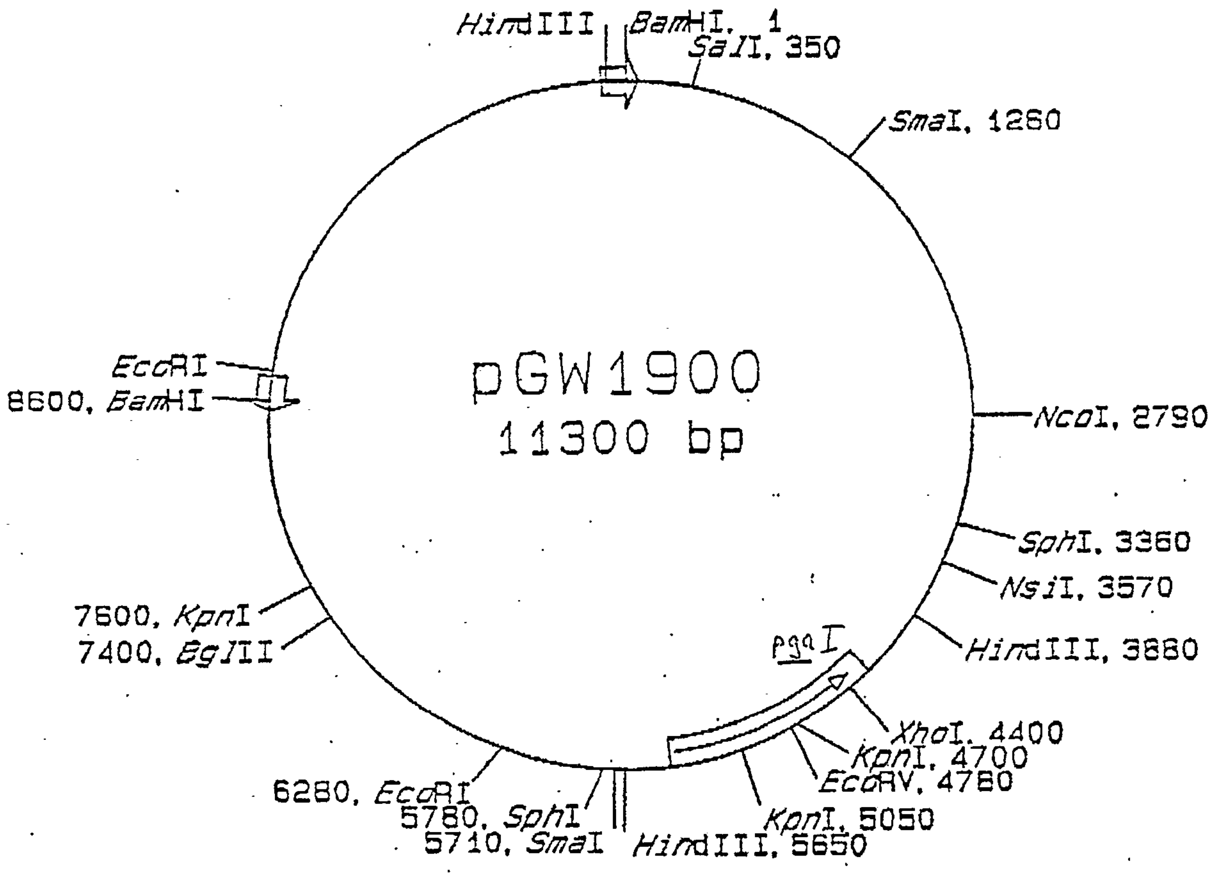
HindIII
BamHI, 1
SalI, 350
SmaI, 1260
NcoI, 2790
SphI, 3360
NsiI, 3570
HindIII, 3880
pgaI
XhoI, 4400
KpnI, 4700
EcoRV, 4780
KpnI, 5050
HindIII, 5650
5710, SmaI
5780, SphI
6280, EcoRI
7400, BglII
7600, KpnI
8600, BamHI
EcoRI
pGW1900
11300 bp

FIGURE 4

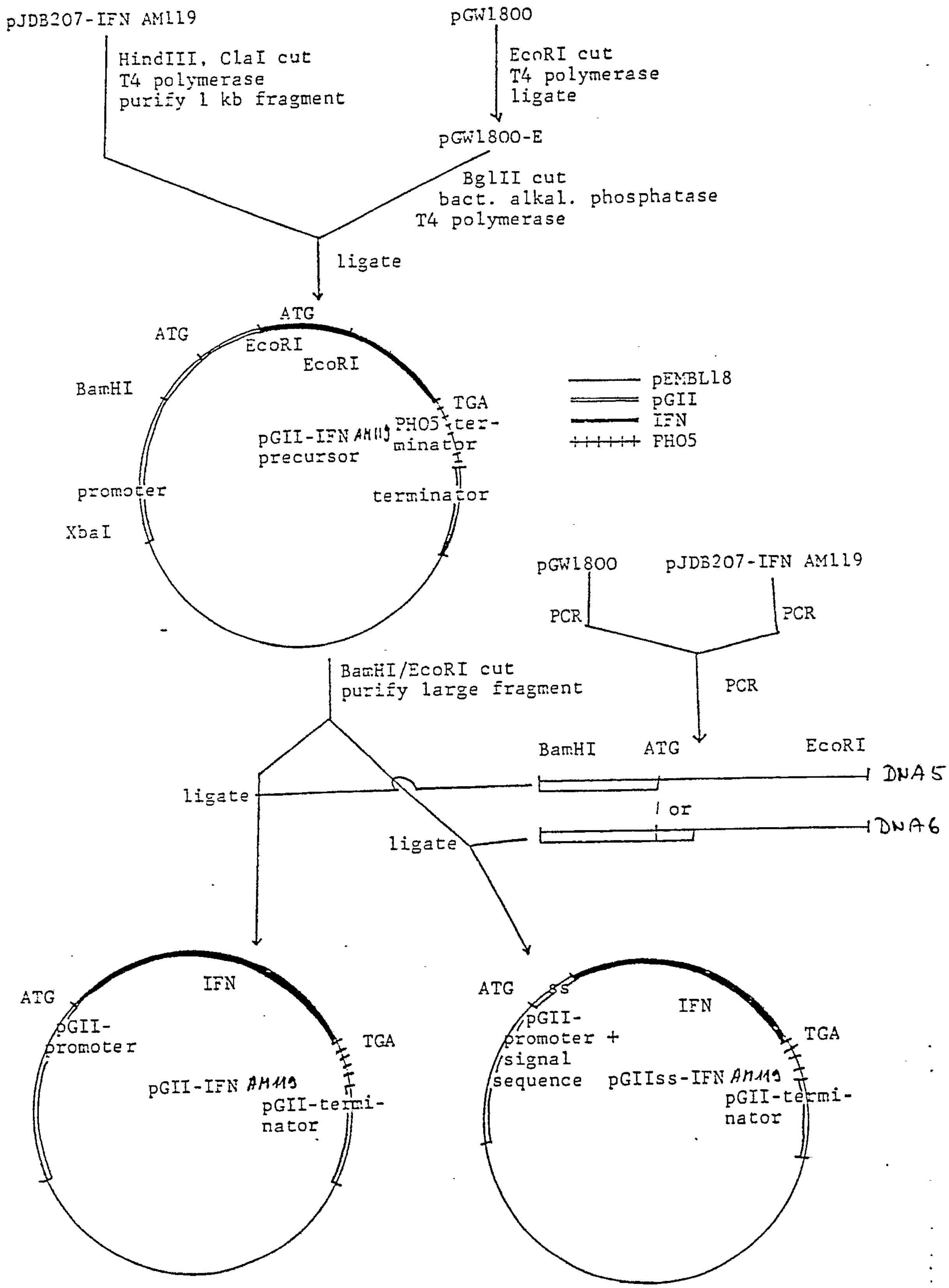
pJDB207-IFN AM119
HindIII, ClaI cut
T4 polymerase
purify 1 kb fragment
pGW1800
EcoRI cut
T4 polymerase
ligate
pGW1800-E
BglII cut
bact. alkal. phosphatase
T4 polymerase
ligate
ATG
ATG
EcoRI
EcoRI
BamHI
TGA
pGII-IFN AM119
precursor
PHO5 terminator
promoter
terminator
XbaI
pEMBL18
pGII
IFN
PHO5
pGW1800
pJDB207-IFN AM119
PCR
PCR
PCR
BamHI/EcoRI cut
purify large fragment
BamHI
ATG
EcoRI
DNA5
or
DNA6
ligate
ligate
ATG
IFN
pGII-promoter
TGA
pGII-IFN AM119
pGII-terminator
ATG
ss
pGII-promoter + signal sequence
IFN
TGA
pGIIss-IFN AM119
pGII-terminator

FIGURE 5

```
PCR with OLIGOs A and B :

      BamHI-
5' AACGAGGATCCAGGGTCCCTA  OLIGO A                                  OLIGO B  ataacaattgttaattagtacacactagacgga 5'
   ||||||||||||||||||||                                                      ||||||||||||||||||||
-- AACGAGGATCCAGGGTCCCTA ------------------ PGII ------------  TATTGTTAACAATTAATCATGCACTCGTTTGCT -->
                                                                                         MetHisSerPheAla -->
                                             |                                           |-- PRE PGII   -->
                                           GIVES
                                             |
                                             V
      BamHI-
   AACGAGGATCCAGGGTCCCTA ------------------ PGII ------------  TATTGTTAACAATTAATCATGTGTGATCTGCCT        ( DNA 1 )
                                                                                         MetCysAspLeuPro


PCR with OLIGOs A and C :

      BamHI-
5' AACGAGGATCCAGGGTCCCTA  OLIGO A                                  OLIGO C ccgcggtggaagcgaagacggacactagacgga 5'
   ||||||||||||||||||||                                                     ||||||||||||||||||||
-- AACGAGGATCCAGGGTCCCTA ------------------ PGII ------------  GGCGCCACCTTCGCTTCTGCCTCTCCTATCGAA -->
                                                               GlyAlaThrPheAlaSerAlaSerProIleGlu
                                             |           -- signal sequence of PGII -->|-- PRO PGII -->
                                           GIVES
                                             |
                                             V
      BamHI-
   AACGAGGATCCAGGGTCCCTA ------------------ PGII ------------  GGCGCCACCTTCGCTTGTGATTGTGATCTGCCT       ( DNA 2 )
                                                               GlyAlaThrPheAlaSerAlaCysAspLeuPro


PCR with OLIGOs D and F :

5' TTCGCTTGTGATTGTGATCTGCCTCAGACTCA OLIGO D                           OLIGO F actgaaacttaaggggtcc 5'
            ||||||||||||||||||||                                                 ||||||||||||||||||||
-- AATTCGACAATGTGTGATCTGCCTCAGACTCA --------- IFN-AM119 ------------------ TGACTTTGAATTCCCCCAGG -->
           MetCysAspLeuProGlnThrHis -->                                             EcoRI-
              |------- Mature IFN ------>       |
                                              GIVES
                                                |
                                                V

   TTCGCTTGTGATTGTGATCTGCCTCAGACTCA --------- IFN-AM119 ------------------ TGACTTTGAATTCCCCCAGG        ( DNA 3 )
   PheAlaSerAlaCysAspLeuProGlnThrHis -->                                             EcoRI-


PCR with OLIGOs E and F :


5' CAATTAATCATGTGTGATCTGCCTCAGAC OLIGO E                              OLIGO F actgaaacttaaggggtcc 5'
          ||||||||||||||||||||                                                   ||||||||||||||||||||
-- AATTCGACAATGTGTGATCTGCCTCAGAC----------- IFN-AM119 ------------------ TGACTTTGAATTCCCCCAGG -->
          MetCysAspLeuProGln -->                                                 EcoRI-
             |--- Mature IFN -->              |
                                            GIVES
                                              |
                                              V

   CAATTAATCATGTGTGATCTGCCTCAGAC ------------ IFN-AM119 ------------------ TGACTTTGAATTCCCCCAGG        ( DNA 4 )
          MetCysAspLeuProGln -->                                                 EcoRI-
```

( continued )

FIGURE 5 continued

PCR with DNAs 1 and 4 and OLIGOs A and F :

```
5' AACGAGGATCCAGGGTCCCTA  OLIGO A
   |||||||||||||||||||||
   AACGAGGATCCAGGGTCCCTA —— PGII of DNA 1 —— CAATTAATCATGTGTGATCTGCCT
                                             ||||||||||||||||||||||||
                                             CAATTAATCATGTGTGATCTGCCT —— IFN-AM119 of DNA 4 —— TGACTTTGAATTCCCCCAGG
                                                                                               ||||||||||||||||||||
                                                                                       OLIGO F actgaaacttaagggggtcc 5'

                                                  |
                                                GIVES
                                                  |
                                                  V

   AACGAGGATCCAGGGTCCCTA ——— PGII ———— CAATTAATCATGTGTGATCTGCCT ———— IFN ———— TGACTTTGAATTCCCCCAGG
        BamHI-                                  MetCysAspLeuPro — IFN —>                 EcoRI-
                                                                                          ( DNA5 )
```

This is a perfect in frame fusion of the 5' end of the mature IFN-AM119 coding region to what was the first methionine of PGII

PCR with DNAs 2 and 3 and OLIGOs A and F :

```
5' AACGAGGATCCAGGGTCCCTA  OLIGO A
   |||||||||||||||||||||
   AACGAGGATCCAGGGTCCCTA —— PGII of DNA 2 —— TTCGCTTGTGATTGTGATCTGCCT
                                             ||||||||||||||||||||||||
                                             TTCGCTTGTGATTGTGATCTGCCT —— IFN-AM119 of DNA 3 —— TGACTTTGAATTCCCCCAGG
                                                                                               ||||||||||||||||||||
                                                                                       OLIGO F actgaaacttaagggggtcc 5'

                                                  |
                                                GIVES
                                                  |
                                                  V

   AACGAGGATCCAGGGTCCCTA ——— PGII ———— TTCGCTTGTGAT TGTGATCTGCCT ———— IFN ———— TGACTTTGAATTCCCCCAGG
        BamHI-                        PheAlaSerAla CysAspLeuPro — IFN —>                 EcoRI-
                                                                                          ( DNA6 )
```

This is a perfect in frame fusion of the 5' end of the mature IFN-AM119 coding region to the end of the PGII signal sequence.

FIGURE 6

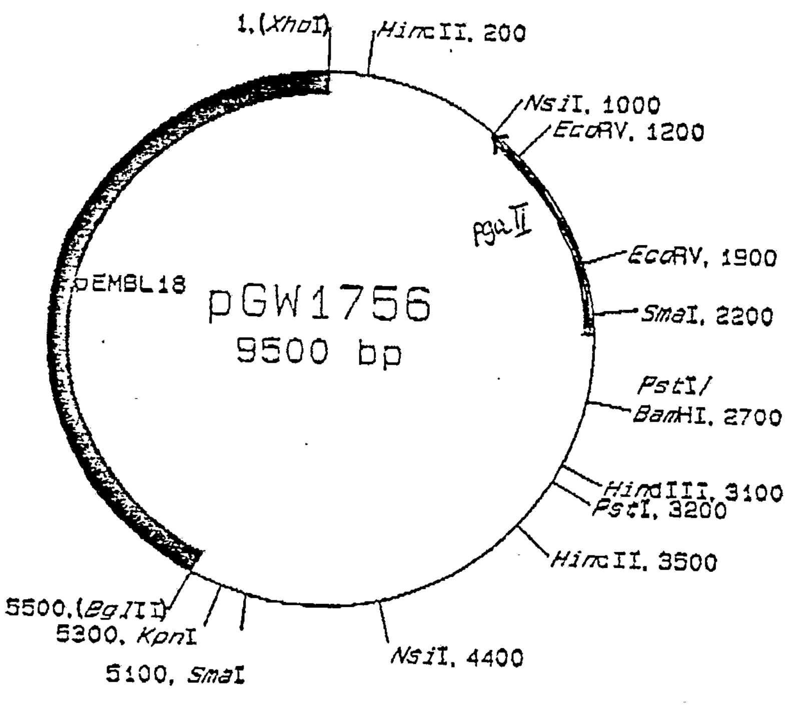
1, (XhoI)
HincII, 200
NsiI, 1000
EcoRV, 1200
pgaII
EcoRV, 1900
SmaI, 2200
PstI/
BamHI, 2700
HindIII, 3100
PstI, 3200
HincII, 3500
NsiI, 4400
5100, SmaI
5300, KpnI
5500, (BglII)
pEMBL18
pGW1756
9500 bp

FIGURE 7

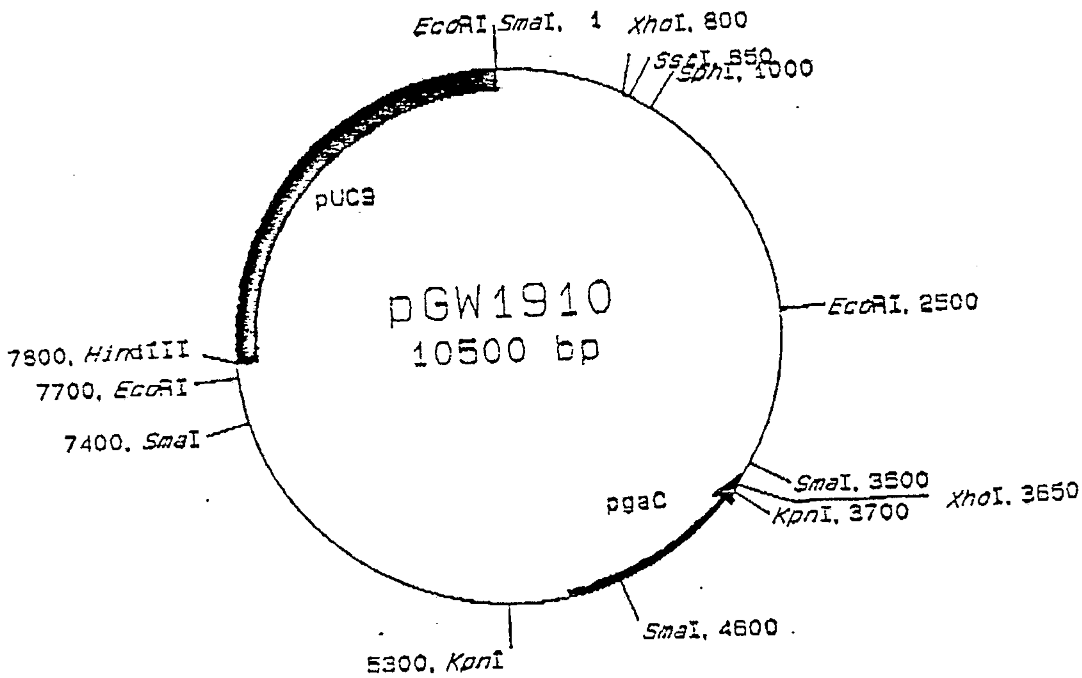
EcoRI
SmaI, 1
XhoI, 800
SstI, 850
SphI, 1000
pUC8
pGW1910
10500 bp
EcoRI, 2500
7800, HindIII
7700, EcoRI
7400, SmaI
SmaI, 3500
XhoI, 3650
KpnI, 3700
pgaC
SmaI, 4600
5300, KpnI